# EUROPEAN PATENT SPECIFICATION

(11) **EP 0 635 051 B1**
(45) Date of publication and mention of the grant of the patent: **23.06.2004**
(21) Application number: 93904049.9
(22) Date of filing: 15.02.1993
(51) Int. Cl.: C12N 1/00

(54) **INCREASING THE TREHALOSE CONTENT OF ORGANISMS BY TRANSFORMING THEM WITH COMBINATIONS OF THE STRUCTURAL GENES FOR TREHALOSE SYNTHASE**
ERHÖHUNG DES TREHALOSEGEHALTES IN ORGANISMEN DURCH TRANSFORMATION MIT KOMBINIERTEN STRUKTURGENEN DER TREHALOSESYNTHASE
AUGMENTATION DE LA TENEUR EN TREHALOSE DES ORGANISMES PAR TRANSFORMATION A L'AIDE DE COMBINAISONS DE GENES DE STRUCTURE POUR LA TREHALOSE-SYNTHASE

(30) Priority: 14.02.1992 US 836021; 28.02.1992 US 841997
(43) Date of publication of application: 25.01.1995
(73) Proprietor: BTG INTERNATIONAL LIMITED, London EC4M 7SB (GB)
(72) Inventor: LONDESBOROUGH, John, SF-00100 Helsinki (FI); VUORIO, Outi, SF-00410 Helsinki (FI)
(74) Representative: Dolan, Anthony Patrick
(86) International application number: PCT/FI1993/000049
(87) International publication number: WO 1993/017093

(56) References cited:
- EP-A- 0 451 896
- Dialog Information Services, File 155, Medline, Dialog Accession No. 7683102, Medline Accession No. 91202102, LONDESBOROUGH J. et al.: "Trehalose-6-Phosphate Synthase/Phosphatasecomplex from Baker's Yeast: Purification of a Proteolytically Activated Form", J Gen Microbiol Feb 1991, 137 (Pt 2) p 323-30.

## Description

### FIELD OF INVENTION

This invention relates to the genetic engineering of the trehalose synthetic pathway of yeasts, such as baker's and distiller's yeasts, and to the transfer of this pathway by genetic engineering to other organisms. In particular, the present invention concerns trehalose synthase, novel genes encoding the trehalose synthase, novel vectors containing the novel genes, and host cells and organisms transformed with the novel vectors. The invention also relates to the production of trehalose and ethanol and to the improvement of the stress resistance of organisms, in particular yeasts and crop plants.

### BACKGROUND OF THE INVENTION:

It is well known that sugars and other polyhydric compounds stabilize isolated proteins and phospholipid membranes during dehydration, probably by replacing the water molecules that are hydrogen-bonded to these macromolecules [reviewed by Crowe, J.H. et al. (1987) Biochemical Journal 242, 1-10]. Trehalose (α-glucopyranosyl-α-D-glucopyranose) is a dimer of two glucose molecules linked through their reducing groups. Because it has no reducing groups, it does not take part in the Maillard reactions that cause many sugars to damage proteins, and it is one of the most effective known protectants of proteins and biological membranes in vitro.

In nature, trehalose is found at high concentrations in yeasts and other fungi, some bacteria, insects, and some litoral animals, such as the brine shrimp. It is notable that all these organisms are frequently exposed to osmotic and dehydration stress. Accumulation of trehalose in higher plants is rare, but high levels occur in the so-called resurrection plants, such as the pteridophyte, Selaginella lepidophylla, which can survive extended drought [Quillet, M. and Soulet, M. (1964) Comptes Rendus de l'Academie des Sciences, Paris 259, pp. 635-637; reviewed by Avigad, G. (1982) in Encyclopedia of Plant Research (New Series) 13A, pp. 217-347].
A decreased availability of intracellular water to proteins and membranes is a common feature not only of dehydration and osmotic stress, but also of freezing, in which ice formation withdraws water from inside the cells, and heat stress, which weakens the hydrogen bonds between water and biological macromolecules. In recent years several publications have shown a close connection between the trehalose content of yeast cells, especially of the species Saccharomyces cerevisiae, and their resistance to dehydration and osmotic, freezing and heat stresses. This work has lead to the concept [summarized by Wiemkem, A. (1990) Antonie van Leeuwenhoek 58, 209-217] that, whereas the main storage or reserve carbohydrate in yeast is glycogen, the prime physiological function of trehalose is as a protectant against these and other stresses, including starvation and even poisoning by copper, ethanol and hydrogen peroxide, which all stimulate trehalose accumulation [Attfield, P.V. (1987) Federation of European Biochemical Societies Letters 225, 259-263].

Thus, during growth of Saccharomyces cerevisiae on glucose, glycogen begins to accumulate about one generation before the glucose is exhausted, and begins to be steadily consumed as soon as all external carbon supplies are exhausted. In contrast, accumulation of trehalose (partly at the expense of glycogen) only begins after all the glucose has been consumed, and the trehalose level is then maintained until nearly all the glycogen has been consumed [Lillie, S.A. & Pringle, J.R. (1980) Journal of Bacteriology 143, 1384-1394]. The eventual consumption of trehalose is accompanied by a rapid decrease in the number of viable cells.

When trehalose levels in S. cerevisiae have been manipulated by varying the growth conditions or administering heat shocks, high positive correlations have been found between the trehalose content of the cells and their resistance to dehydration [Gadd, G. et al. (1987) Federation of European Microbiological Societies Microbiological Letters 48, 249-254], heat stress [Hottiger, T. et al., (1987) Federation of European Biochemical Societies Letters 220, 113-115] and freezing [Gélinas, P. et al. Applied and Environmental Microbiology 55, 2453-2459]. Also, strains of S. cerevisiae and other yeasts selected for resistance to osmotic stress [D'Amore, T. et al. (1991) Journal of Industrial Microbiology 7, 191-196] or high performance in frozen dough fermentation [Oda, Y. (1986) Applied and Environmental Microbiology 52, 941-943] were found to have unusually high trehalose contents. Furthermore, a mutation in the cyclic AMP signaling system of S. cerevisiae that causes constitutive high trehalose levels also causes constitutive thermotolerance, whereas another mutation in this system that prevents the usual rise in trehalose during heat shock also prevents the acquisition of thermotolerance [Hottiger, T. et al., (1989) Federation of European Biochemical Societies Letters 255, 431-434]. Thus, there is much evidence pointing to a connection between trehalose content and stress resistance in yeasts, especially S. cerevisiae. Similar findings have been made for several other fungi [e.g., Neves, M.J., Jorge, J.A., Francois, J.M. & Terenzi, H.F. (1991) Federation of European Biochemical Societies Letters 283, 19-22]. However, a causative relationship has not yet been demonstrated. Further, nearly all conditions that cause increases in the trehalose content of yeast also cause increases in the levels of the so-called heat shock proteins. The 1989 publication of Hottiger and colleagues, cited above, claims that canavanine does not cause an increase in either trehalose levels or thermotolerance, whereas this compound is reported to induce heat shock proteins.

Whether or not there is a causal relation between trehalose content and stress resistance, it has become general practice in the manufacture of baker's yeast to maximise the trehalose content of the yeast. Various maturation processes have been developed to achieve this aim, and they are of crucial importance in the manufacture of active dried yeast. The details of these processes are often secret, but they are generally empirical regimes in which carbon and nitrogen feeds, aeration and temperature are carefully controlled and selected strains of yeast are used. They demand time and energy inputs during which little increase in cell number occurs. A more rational and controlled process would be of economic benefit.

According to Cabib, E. & Leloir, L.F. [(1957) Journal of Biological Chemistry 231, 259-275], trehalose is synthesized in yeast from uridine diphosphoglucose (UDPG) and glucose--6-phosphate (G6P) by the sequential action of two enzyme activities, trehalose-6-phosphate synthase and trehalose--6-phosphate phosphatase. Londesborough, J.& Vuorio, O. [(1991) Journal of Microbiology 137, 323-330, expressly incorporated herein by reference] have purified from baker's yeast a proteolytically modified protein complex that exhibited both these activities and appeared to contain a short polypeptide chain (57 kDa) and two truncated versions (86 kDa and 93 kDa) of a long polypeptide chain. The intact long chain was estimated to have a mass of at least 115 kDa. It was not disclosed which enzyme activity or activities was associated with which polypeptide, nor indeed whether both polypeptide were essential for either or both enzymatic activities.
Anti-sera against both polypeptides were reported, but no amino acid sequences were disclosed.

An earlier patent application [EP 451 896; see Claim 1] has claimed for a transformed yeast "comprising.... one gene encoding....trehalose-6-phosphate synthase". However, no information about the either the gene or the protein it encodes was provided.

Several authors have reported increases in TPS activity in conditions that lead to accumulation of trehalose by S. cerevisiae, and Schizosaccharomyces pombe both during the approach to stationary phase [Winkler, K., et al. (1991) Federation of European Biochemical Societies Letters 291, 269-272; Francois, J., et al. (1991) Yeast 7, 575-587] and after temperature shift-ups to about 40 °C [De Virgilio, C, et al. (1990) Federation of European Biochemical, Societies Letters 273, 107-110]. Panek and her colleagues [Panek, A.C., et al. (1987) Current Genetics 11, 459-465] have claimed that TPS activity is increased by dephosphorylation of pre-existing enzyme molecules, i.e., that it is the result of post-translational regulation. This claim has been challenged [Vandercammen, A., et al., (1989) European Journal of Biochemistry 182, 613-620] but continues to be made [Panek, A.D. & Panek, A.C. (1990) Journal of Biotechnology 14, 229-238]. Evidence for or against an increase in the amount of enzyme during trehalose accumulation is conflicting. Inhibitors of mRNA synthesis inhibited trehalose accumulation by S. cerevisiae shifted from 30 to 45 °C [Attfield (1987) loc.cit.], whereas under very similar conditions Winkler et al [(1991) loc.cit.] found that cycloheximide (an inhibitor of protein synthesis) did not prevent the accumulation of trehalose, which, however, occurred without an observable increase in TPS activity. In a lower temperature range (a shift from 23 to 36 °C), trehalose accumulation was accompanied by a three-fold increase in TPS activity, and cycloheximide prevented the increase in TPS [Panek, A.C., et al. (1990) Biochemie 72, 77-79]. In Schizosaccharomyces pombe, [De Virgilio, C., et al. (1991) loc. cit.] temperature shiftup caused a large accumulation of trehalose and increase of TPS which were not prevented by cycloheximide, leading the authors to suggest that in this yeast a post-translational activation occurs. We now disclose that in S. cerevisiae the co-ordinate increases in TPS and TPP activities during exhaustion of glucose are accompanied by an increase in antigenic material recognized by anti-sera to the short and long chains of a purified trehalose synthase. Hence, a method to increase the trehalose content of cells, and so, their stress tolerance, would be to isolate, clone, and modify the structural genes (hereinafter referred to as TSS1, TSL1, and TSL2) of these polypeptides and cause their expression in yeast or other host cells under the control of suitable promoters. If the expression of these genes could be controlled, then so could the trehalose content of the host cells.

The well known metabolic theory of Kacser & Burns [(1973) Symposium of the Society of Experimental Biology 27, 65-107] teaches that in principle the concentration of any intermediate, such as trehalose, can be increased by increasing the amount of any enzyme synthesizing it or decreasing the amount of any enzyme degrading it, but that the size of the increase may not be significant. The novelty of the present invention lies in the identification and characterization of the particular yeast genes that must be modified to increase the amounts of trehalose synthase and the recognition of the advantages of modifying the synthetic pathway rather than the degradative pathway. These advantages include (i) leaving the highly regulated [see, e.g., Thevelein, J.M. (1988) Experimental Mycology 12, 1-12] degradative pathway intact to avoid the physiological problems likely in yeast that cannot activate this pathway, (ii) the possibility of causing yeast to synthesize trehalose under physiological conditions where wild type yeasts do not (so that blocking the degradative pathway cannot increase the amount of trehalose) and (iii) the important possibility of introducing by these genes a trehalose-synthetic capacity to organisms, such as most higher plants, that naturally lack this capacity.

Expression of the genes for trehalose synthesis in yeast under conditions where trehalase is active will increase the operation of a so-called "futile" cycle, in which glucose is continuously phosphorylated, converted to trehalose and regenerated by hydrolysis of the trehalose, resulting in increased consumption of ATP. This ATP must be regenerated, and under fermentative conditions this will occur by conversion of sugars into ethanol. Therefore, introduction of TSS1, TSL1 and TSL2 into yeast under the control of promoters active under fermentative conditions is expected to decrease the yield of cell mass on carbon source and increase that of ethanol. The many attempts [e.g., Schaaf et al. (1989) Yeast 5, 285-290] to increase fermentation rates in yeast by increasing the levels of glycolytic enzymes have been unsuccessful. The probable reason is that availability of ADP limits the rate of glycolysis in yeast. Introduction of a futile cycle-ATPase is thus expected to increase this rate. The feasibility of this invention is demonstrated by the finding [Gancedo, J.-M. & Navas, M.A. (1992) Yeast 8 S574] that expression of the gluconeogenic enzymes, fructose bisphosphatase and phosphoenolpyruvate carboxykinase under fermentative conditions (so causing two futile cycles) caused a 50 % increase in the fermentation rate of resting yeast. Use of the trehalose futile cycle has the added advantage that the cells must then contain a steady state level of trehalose, which increases their tolerance to osmotic and temperature stress.

The present invention includes transformed strains of distiller's yeast, in which the presence of modified forms of any or all of TSS1, TSL1 and TSL2 results in an increased yield of ethanol from carbohydrate sources.

As well as being used to improve the properties of yeast, especially active dried yeast and yeast for frozen doughs, this invention has other obvious applications. First, by increasing the proportion of trehalose in yeast, the industrial scale production of trehalose from yeast is made more economic. It is particularly advantageous to obtain trehalose from yeast because, since yeast is a traditional and safe food stuff, a minimal purification of the trehalose will often be adequate: preparations of trehalose containing yeast residues could be safely added to food stuffs for human or animal consumption. Trehalose also has medical applications, both as a stabilizer of diagnostic kits, viruses and other protein material [WO 87/00196] and, potentially, as a source of anti-tumour agents [Ohtsuro et al. (1991) Immunology 74, 497-503]. Trehalose for internal applications in humans would be much more safely obtained from yeast than from a genetically engineered bacterium.

Second, by transferring these genes to higher plants after making suitable modifications obvious to anyone skilled in the art (in general, replacements of adenine/thymine base pairs by guanine/cytosine base pairs as suggested by Perlak et al. [(1991) Proceedings of the National Academy of Sciences of the U.S.A. 88, 3324-3328] and the introduction of suitable promoters, some of which may be tissue-specific, to direct the synthesis of trehalose to frost and drought-sensitive tissues), the resistance of the plants to various stresses, especially frost and dehydration, should be improved. The economic importance of such improvements is potentially enormous, because even small increases in cold-tolerance will lead to large increases in growing season, whereas dehydration resistance can save entire crops in time of drought. Frost and drought resistance in higher plants is usually accompanied by increases in compounds such as proline rather than trehalose [reviewed by Stewart (1989) in "Plants under Stress", pp 115-130], but, as mentioned above, resurrection plants accumulate large amounts of trehalose and there seems, a priori, to be no reason why this strategy should not be successful. Therefore, the present invention includes a process to transform crop plants by introducing recombinant forms of the structural genes of yeast trehalose synthase (TSS1, TSL1 and TSL2) so as to increase the trehalose content of some of their tissues compared to those of the parent plant. Such transformed plants can also be economic and safe sources of trehalose. Third, the shelf-life of food products can be increased by adding trehalose to them [WO 89/00012]. A further aspect of the present invention is a novel process for producing trehalose-enriched food products from plants by causing them to express the structural genes for yeast trehalose synthase in their edible tissues.

### SUMMARY OF INVENTION:

The present invention provides two isolated genes encoding, respectively a short and a long chain of yeast trehalose synthase and a third gene encoding a 99 kDa polypeptide that occurs in some trehalose synthase preparations and has trehalose-6-phosphatase activity. These genes can be used to transform an organism (such as a yeast, other fungus or higher eukaryote), whereby the transformed organism produces more trehalose synthase resulting in a trehalose content higher than that of the parent organism. The higher trehalose content confers improved stress resistance and storage properties on the transformed organism as compared to the parent organism, and the transformed organism can be used to provide large quantities of trehalose. Thus, a process for producing a crop plant which has increased resistance to water deprivation, heat and cold, comprises transforming the plant by introducing at least one of the novel genes into the plant's tissue.

The invention also provides a trehalose synthase which exhibits trehalose-6-phosphate synthase activity activatable by fructose-6-phosphate and also trehalose-6-phosphatase activity.

Finally, the invention provides processes for producing trehalose by cultivating a host or an organism which has been transformed with at least one of the novel genes, and processes for producing trehalose enriched food products from plants by introducing at least one of the novel genes and allowing said genes to express the trehalose synthase in the edible tissues of the plant.

### BRIEF DESCRIPTION OF FIGURES

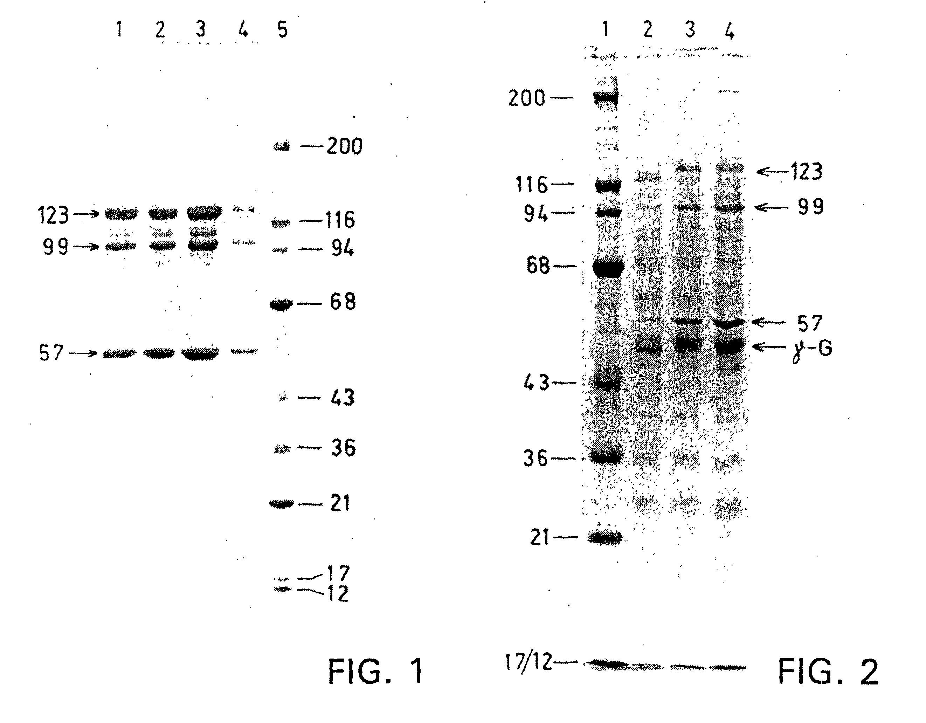

Fig. 1. SDS-PAGE of intact trehalose synthase
   A 6-13 %T gradient gel was used. Lane 1 contains 8.3 µg of intact trehalose synthase eluted from the UDPG-Glucuronate-Agarose column with 0.2 M NaCl (#11 of Table 1). Lanes 2, 3 and 4 contain, respectively, 7.7, 12 and 1.0 µg of enzyme eluted from the column with 0.4 M NaCl containing 10 mM UDPG (#13, #14 and #15 from Table 1). Lane 5 contains about 1 µg each of molecular mass markers (myosin, β-galactosidase, α-phosphorylase, BSA, ovalbumin, lactate dehydrogenase, triosephosphate-isomerase, myoglobin and cytochrome c). The major polypeptides of intact trehalose synthase are named on the left and the molecular mass calibration, in kDa, is shown on the right.
Fig. 2. SDS-PAGE of immunoprecipitates of wild-type yeast grown on YP/2 % glucose
   A 9 %T gel was used. Lane 1 contains about 1 µg each of the molecular mass markers used in Fig 1. Lanes 2, 3 and 4 contain immunoprecipitates from 3.8 mg fresh yeast harvested after 16.1 h (1.2 % residual glucose), 18.1 h (no residual glucose) and 39 h. The molecular mass calibration is shown on the left and the major polypeptides of trehalose synthase and the heavy chain of γ-globulin are shown on the right.

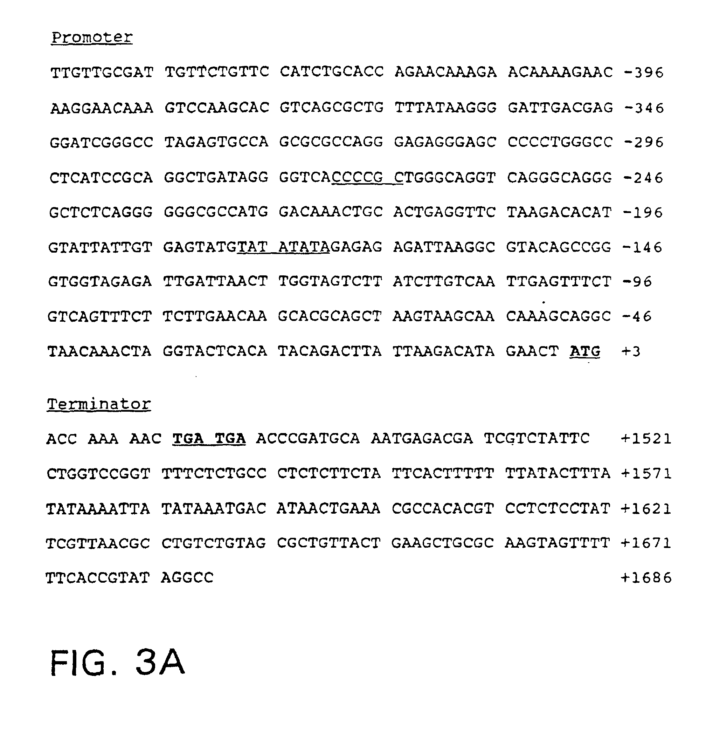

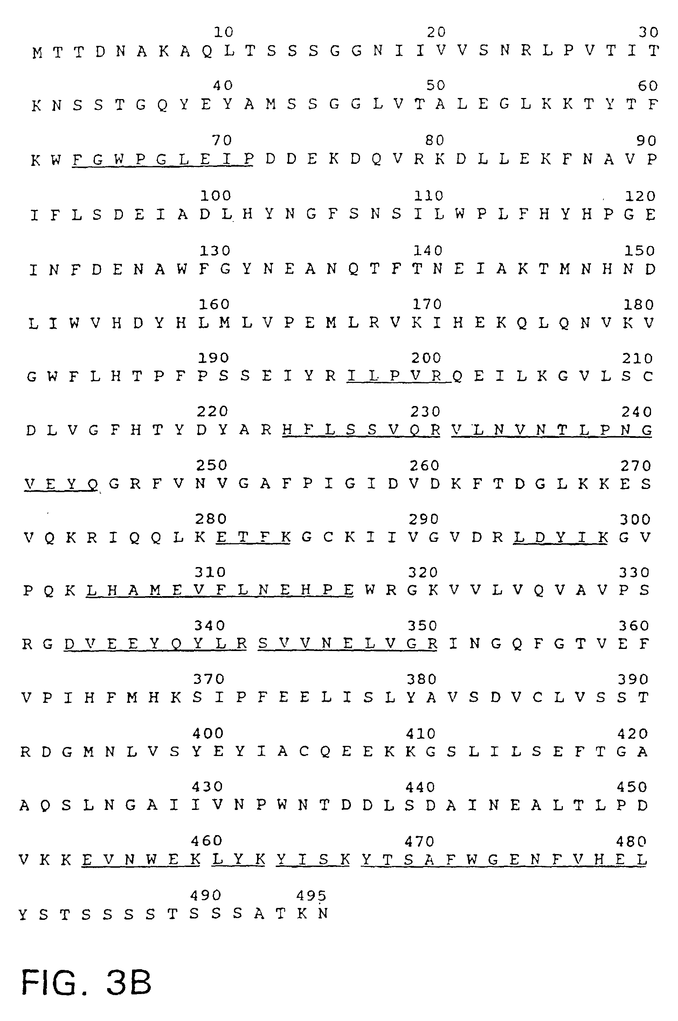

Fig. 3. The promoter and terminator of TSS1 and the amino acid sequence deduced from its ORF.
   (a) In the promoter and terminator regions, the start ATG and tandem TGA stop codons are bold and underlined and a TATA box and putative catabolite repression element are underlined. (b) In the amino acid sequence (SEQ ID NO:2), the sequences found in peptides isolated from the short chain of trehalose synthase are underlined.

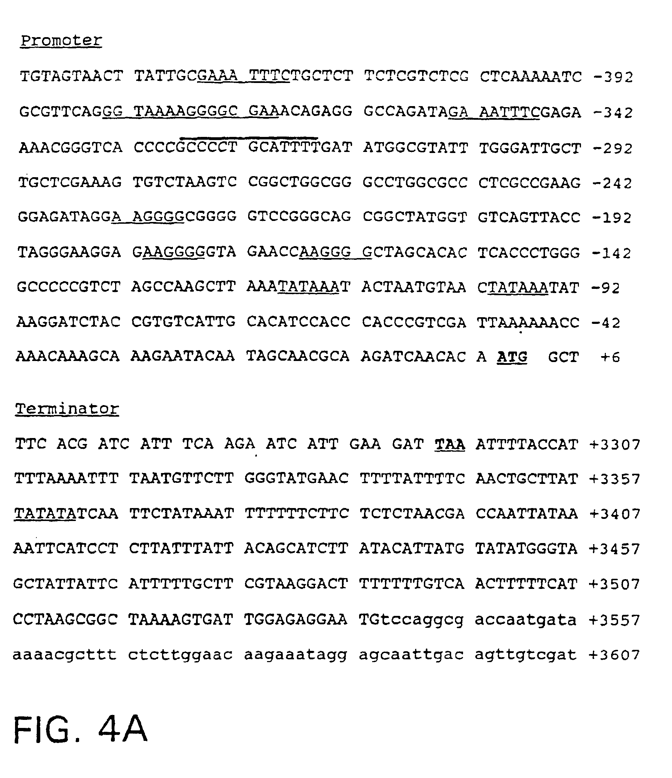

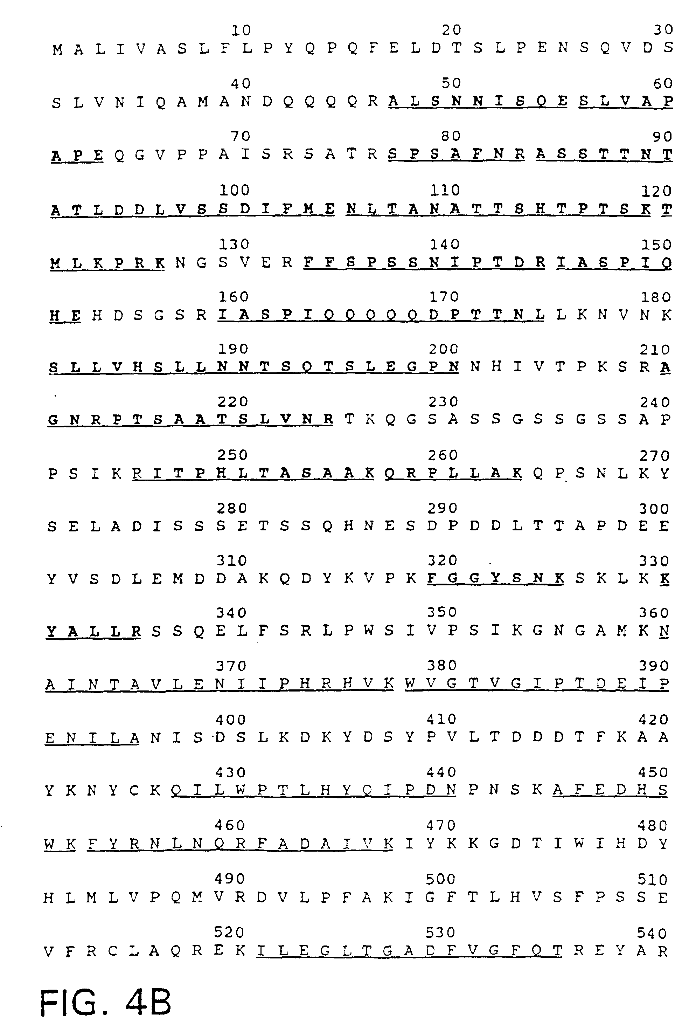

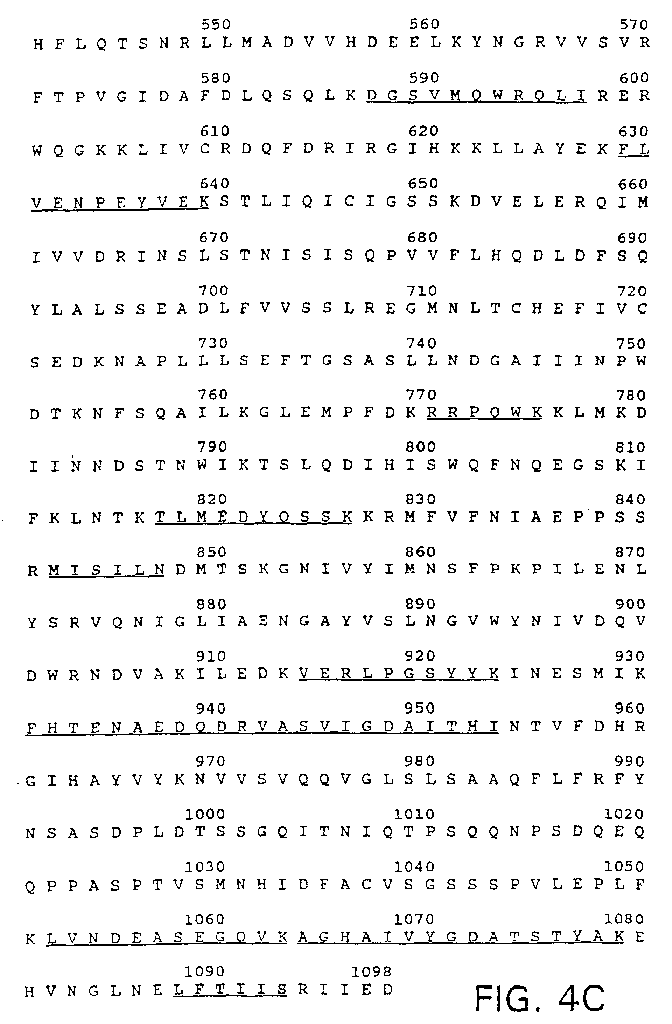

Fig. 4. The promoter and terminator regions of TSL1 and the amino acid sequence deduced from its ORF.
   (a) In the promoter region, the start ATG codon is bold and underlined and two TATA boxes and six putative heat shock elements are underlined. A putative MIG1 binding site is overlined. In the terminator region, the TAA stop codon is bold and underlined and a putative transcription termination element is underlined. Lower case letters show the end of the terminator region of the ARGRII gene, which has opposite polarity. (b) In the amino acid sequence (SEQ ID NO:82), sequences found in peptides isolated from (fragments of) the 123 kDa long chain are underlined, and those from peptides liberated from intact trehalose synthase by limited digestion with trypsin are underlined and bold.

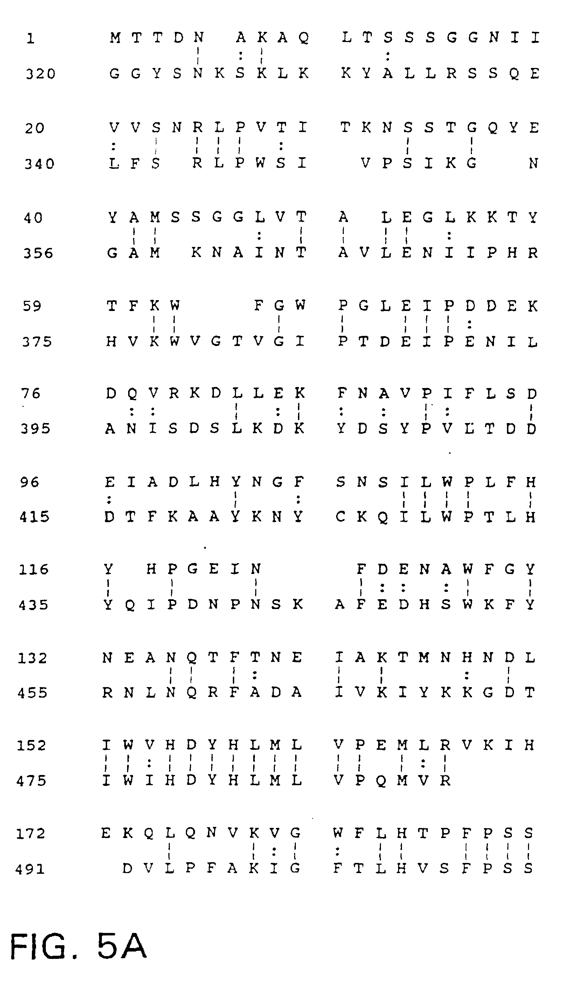

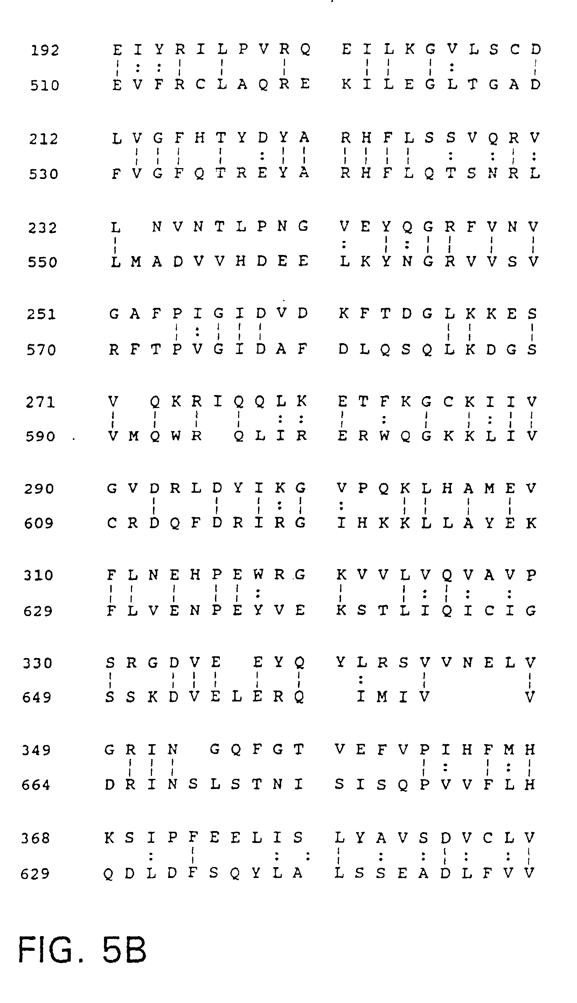

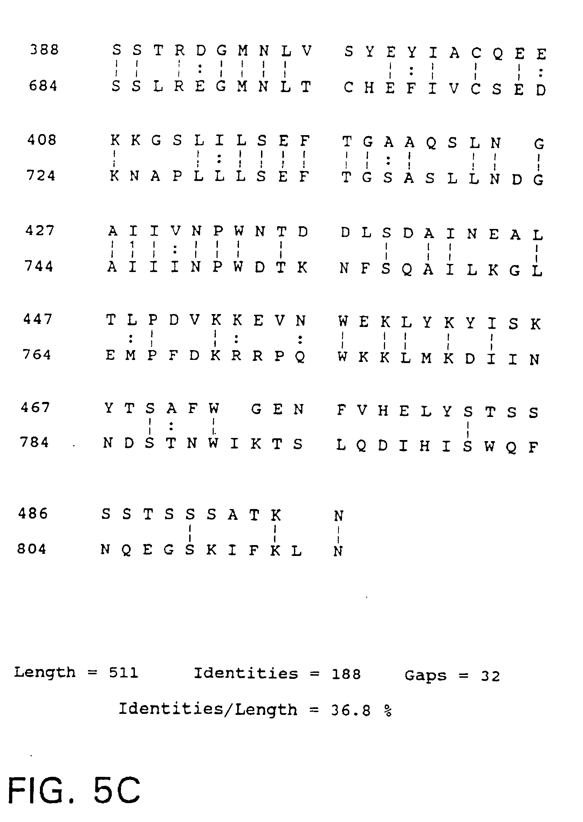

Fig. 5. Alignment of the amino acid sequences of the short and long chains of trehalose synthase
   The complete short chain sequence (SEQ ID NO:2; the upper sequence) is aligned against residues 320 to 814 of the 123 kDa long chain (SEQ ID NO:4; the lower sequence). 32 gaps are introduced to optimize the alignment. Vertical dashes indicate identical residues. Colons indicate conservative substitutions.
Fig. 6. Important restriction sites in TSS1 and TSL1
   The heavy lines indicate open reading frames. The scale bar shows one kb.
Fig. 7. Synthesis of [¹⁴C]-trehalose from [U-¹⁴C]-glucose 6-phosphate by an extract of wild-type yeast
   Reaction mixtures (100 µl) contained 40 mM HEPES/KOH pH 6.8, 1 mg BSA/ml, 10 mM MgCl₂ 10 mM [U-¹⁴C]-G6P (736 c.p.m./nmol) and (a) no phosphate or (b) 5 mM K phosphate pH 6,8 and (0) 5 mM UDPG, (●) 2.5 mM ADPG or (□) neither UDPG nor ADPG. Reactions were started by adding 10 µl (equivalent to 94 µg fresh yeast) of a 28,000 g supernatant of stationary phase X2180. Reactions were stopped by transfer to boiling water for 2 min and addition of 1.0 ml of a slurry of AG1-X8 (formate) anion exchange resin [Londesborough & Vuorio (1991) loc. cit.]. The radioactivity in the resin supernatant was measured.

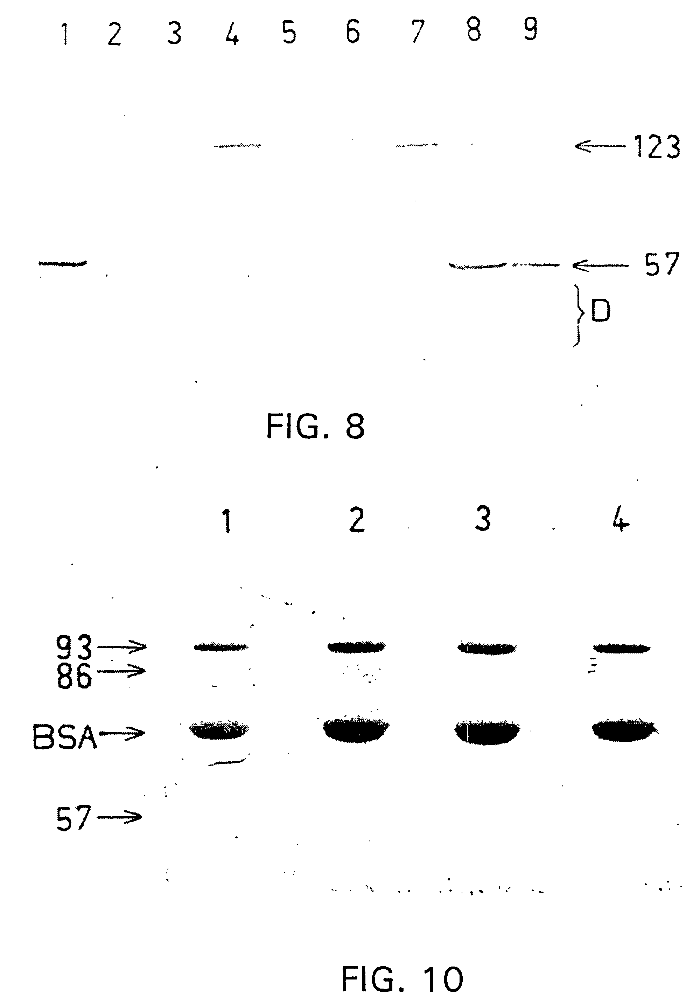

Fig. 8. Western analysis of Klg 102 and X2180 yeasts
   Growth of the yeasts is described in Example 7. The loads of fresh yeast per lane were: lane 1, 200 µg X2180/2; lanes 2 and 5, 330 µg 2669/1: lanes 3 and 6, 610 µg 2669/2; lanes 4 and 7, 810 µg 2670/1+2; lane 8, 560 µg X2180/1 and lane 9, 280 µg X2180/1. The blot was probed with anti-TPS/P serum at a dilution of 1/30,000. Major bands of trehalose synthase are identified on the right.

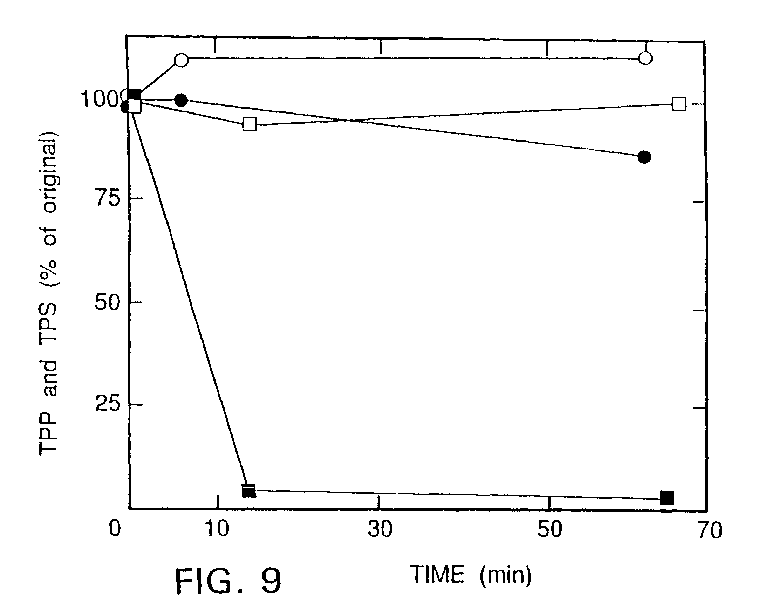

Fig. 9. Treatment of truncated trehalose synthase with 1.9 mM NEM
   Truncated enzyme (0.13 TPS units/ml ≈ 43 µg/ml) in 2 mg BSA/ml 50 mM HEPES pH 7.0 containing 67 mM NaCl, 0.2 mM EDTA, 0.17 mM dithiothreitol, 0.17 mM benzamidine and 1.7 mM UDPG was incubated at 24 °C with (closed symbols) or without (open symbols) 1.9 mM NEM. TPS (●,○] and TPP (■,□) activities were measured.

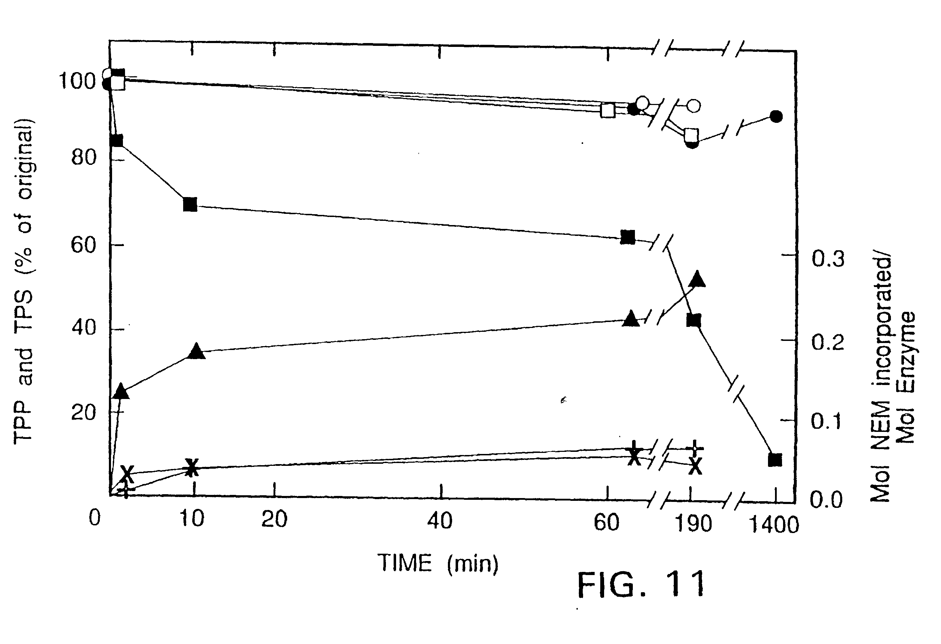

Fig. 10. Autoradiogram of truncated trehalose synthase labelled with [¹⁴C]-NEM and separated by SDS-PAGE
   Labelling was performed as described in Example 8 for 1.5, 10.5, 63 and 190 min in lanes 1, 2, 3 and 4, respectively. The positions of the (57 kDa) short chain, 93 and 86 kDa long chain fragments and the carrier BSA are indicated.
Fig. 11. Treatment of truncated trehalose synthase with ethyl-labelled NEM.
   Truncated enzyme (7.2 TPS units/ml ≈ 0.24 mg/ml) in 1 mg BSA/ml 25 mM HEPES pH 7.0 containing 2 mM MgCl2, 1 mM EDTA and 0.2 M NaCl was incubated at 23 °C with (solid symbols) or without (open symbols) 32 µM ethyl-labelled NEM. TPS (●,O] and TPP (■,□) activities and the amounts of [¹⁴C]-NEM incorporated into the 93 (▲), 86 (+) and 57 (X) kDa polypeptides were measured. 0.1 mol NEM incorporated per mol (150 Kg) of enzyme corresponds to an excess radioactivity of 75 c.p.m. in bands cut from the gel.

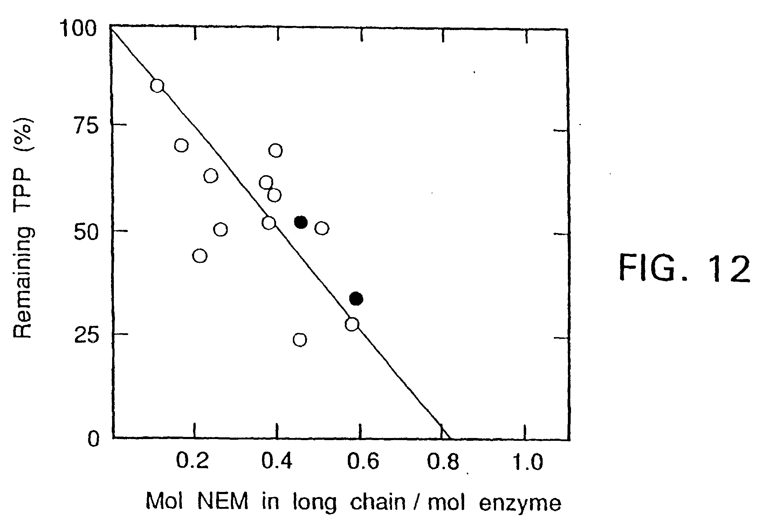

Fig. 12. Stoichiometry of NEM labelling
   Residual TPP activity is plotted against the amount of NEM incorporated to the 93 and 86 kDa fragments of the long chain. Ring-labelled (●) and ethyl-labelled (0) NEM were used.

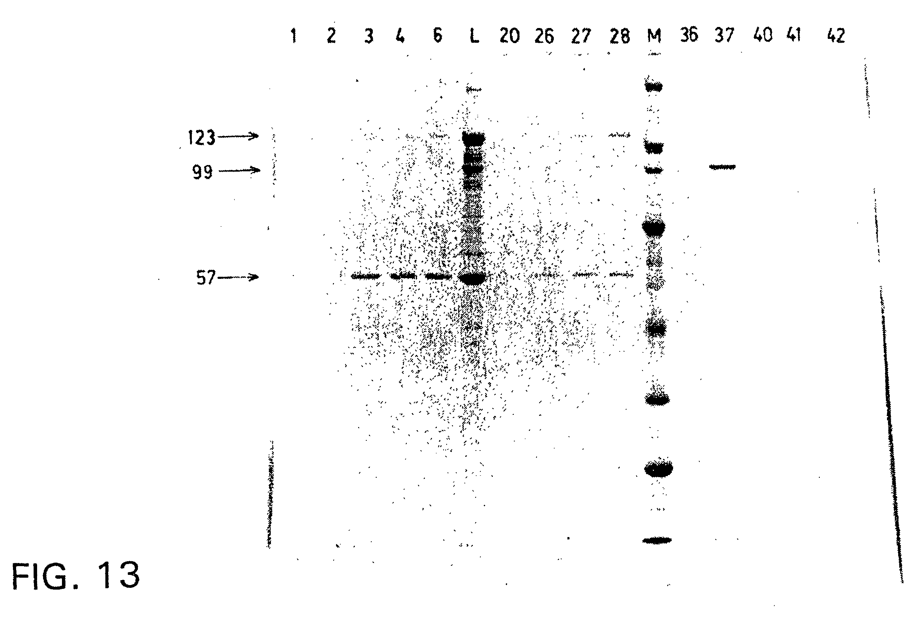

Fig. 13. SDS-PAGE analysis of fractions eluted from the cellulose-phosphate with buffer containing 0.3 % Triton
   Lane L contains 47 µl of the intact trehalose synthase applied to the column. Lane M contains about 1 µg each of the molecular mass markers used in Fig 1. The numbered lanes contain 33 µl of selected 1.5 ml fractions eluted from the column. The NaCl gradient began to appear in fraction 6 and reached 300 mM at fraction 27. A step to 600 mM NaCl emerged between fractions 36 and 37. Fractions 40 to 42 were eluted with 200 mM K phosphate. The major bands in the trehalose synthase preparation are identified on the left. Details are given in Example 9.

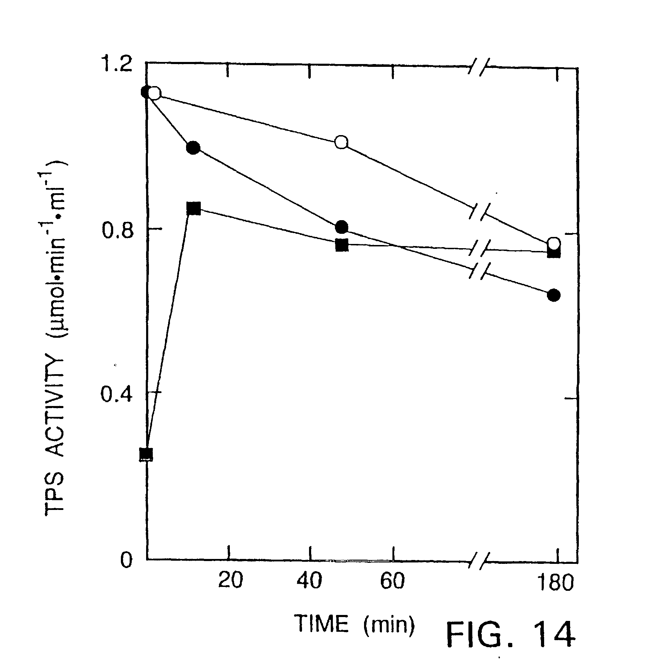

Fig. 14. In vitro activation of trehalose synthase by limited tryptic digestion
   Intact trehalose synthase was incubated with (solid symbols) and without (open symbols) trypsin and its TPS activity measured in the presence of 5 mM F6P in reaction mixtures containing (0, ) no phosphate or (■) 5 mM K phosphate pH 6,8. Details are given in Example 10.

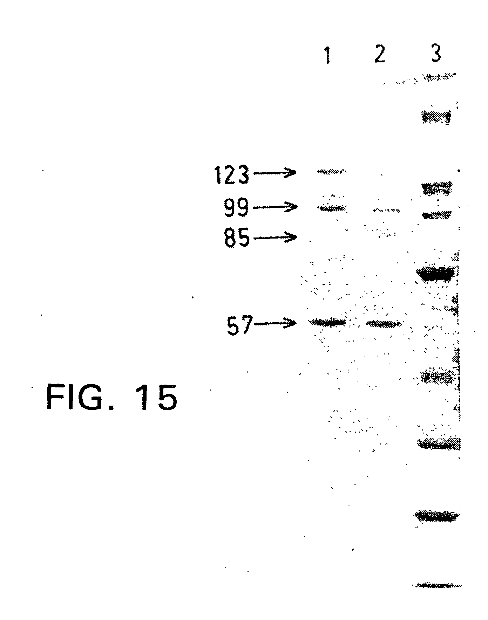

Fig. 15. Limited tryptic digestion of intact trehalose synthase
   Lane 1 contains the untreated trehalose synthase used in Fig. 15 and lane 2 the same amount of enzyme after 48 min treatment with trypsin. Lane 3 contains molecular mass standards. The major polypeptides of trehalose synthase are identified on the left.

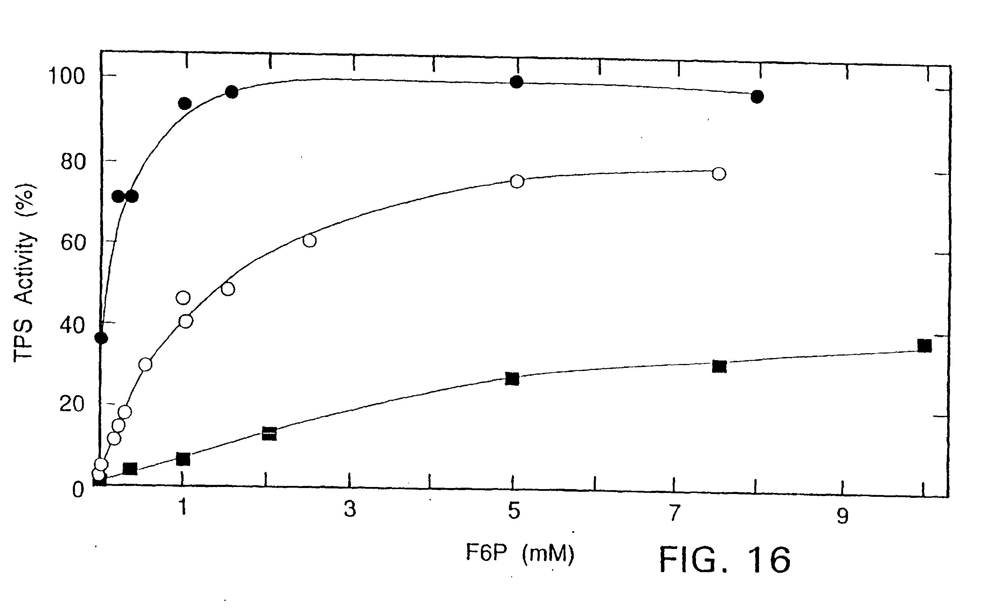

Fig. 16. The effect of fructose 6-phosphate on the TPS activity of intact trehalose synthase at different phosphate concentrations
   The TPS activity of native trehalose synthase was measured between zero and 10 mM F6P. Other conditions were as in the standard TPS assay with (●) no changes, (○) 1.3 mM K phosphate pH 6.8 added or (■) 4 mM K phosphate pH 6.8 and 0.1 M KCl added and the MgCl₂ concentration decreased to 2.5 mM. Activities are shown as percentages of that in the standard assay (i.e., at 5 mM F6P and no phosphate).

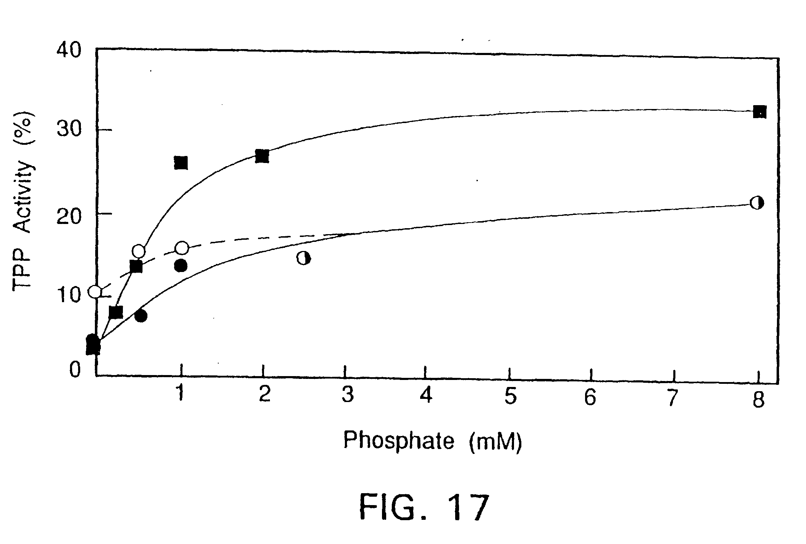

Fig. 17. Activation of the TPP activities of intact and truncated trehalose synthase by phosphate
   TPP activities were measured at 0.5 mM [¹⁴C]-trehalose-6-phosphate in assay mixtures containing 50 mM Hepes pH 6.8, 1 mg bovine albumin/ml and the indicated concentrations of K phosphate pH 6.8 and are shown as percentages of the standard TPS activity. Initial rates are shown for the (■) intact and (●) truncated enzyme. Rates during the second five minutes of the accelerating reaction obtained with truncated enzyme are also shown (○).

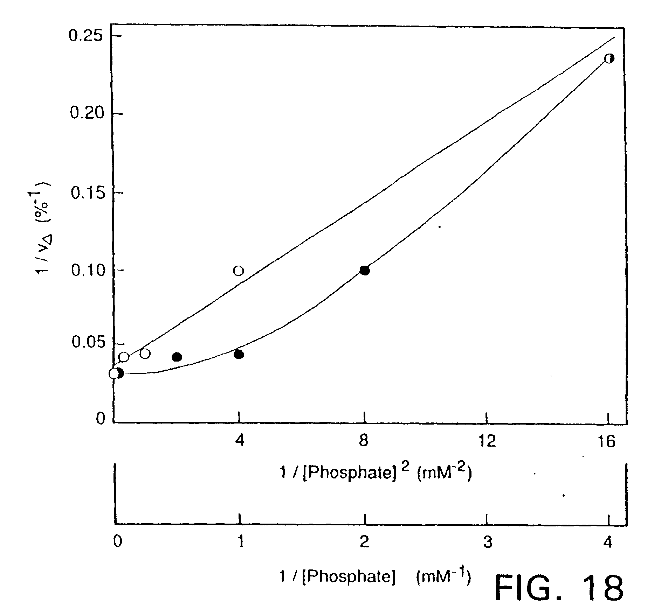

Fig. 18. Phosphate-dependence of the TPP activity of intact trehalose synthase
   The reciprocal of the increase in rate (V_{Δ}) caused by the phosphate is plotted against (○) [phosphate]⁻² or (●) [phosphate]⁻¹. V_{Δ} is shown as a percentage of the standard TPS activity.

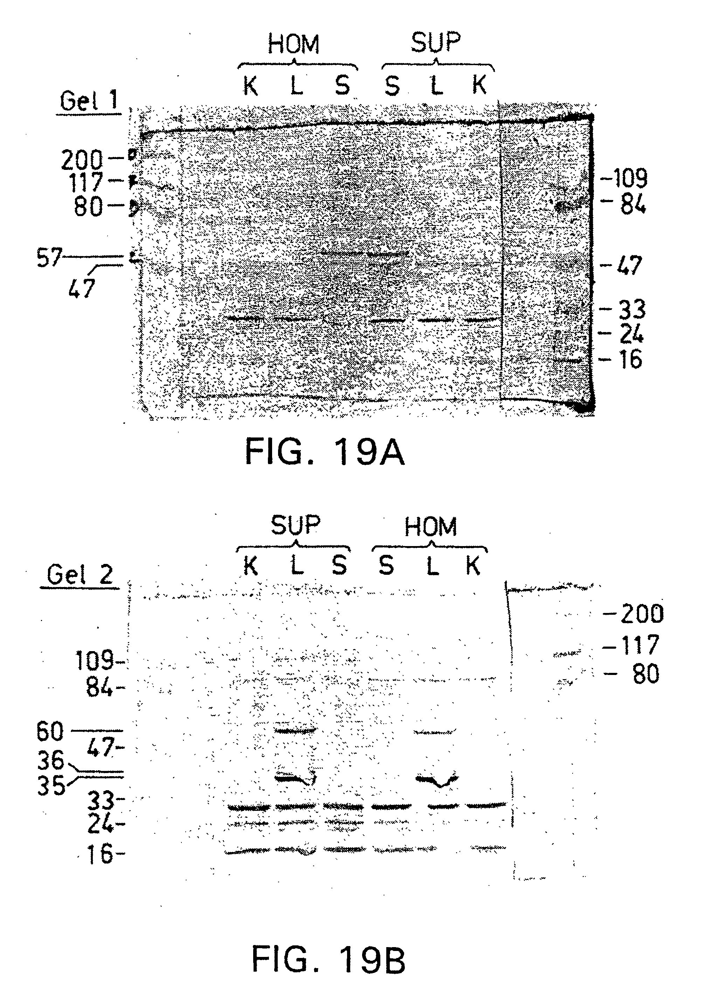

Fig. 19. Western analyses of E. coli transformed with TSS1 and TSL1
   The gels were loaded with samples of whole homogenates (HOM) equivalent to 300 ± 12 µg fresh cells or 28 000 g supernatants (SUP) equivalent to 340 ± 25 µg fresh cells. The letters above the lanes indicate the cell types: K, control (HB101) cells; L, ALKO3568 (HB101 transformed with TSL1); S, ALKO3566 (HB101 transformed with TSS1). Gel 1 was probed with anti-57K serum (1/20 000) and gel 2 with anti-93K serum (1/20 000). The positions of the 57 kDa short chain and about 60, 36 and 35 kDa fragments of the 123 kDa long chain are shown. Molecular mass standards are labelled in kDas.

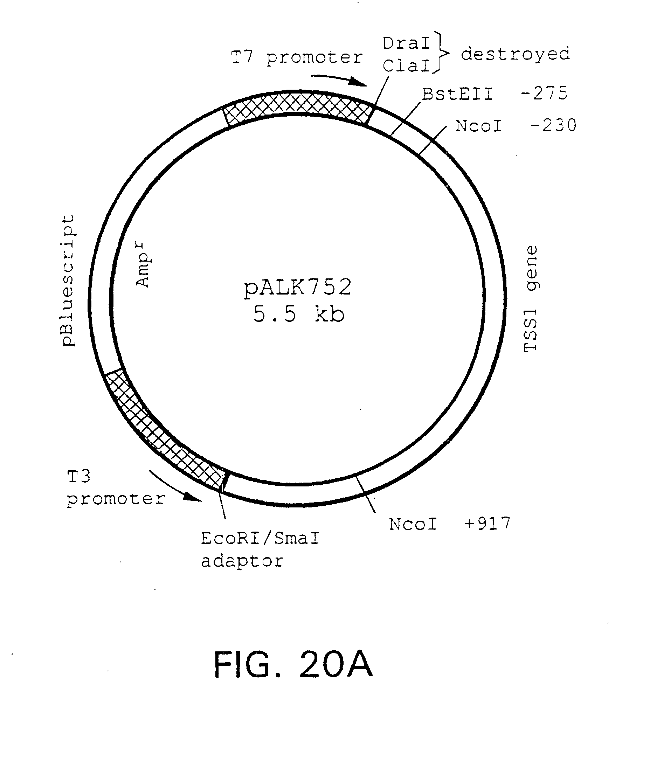

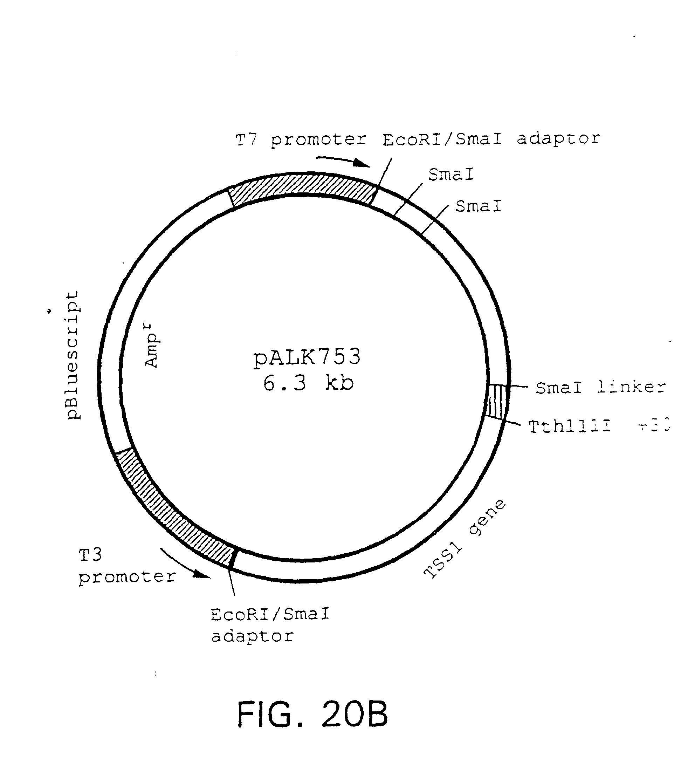

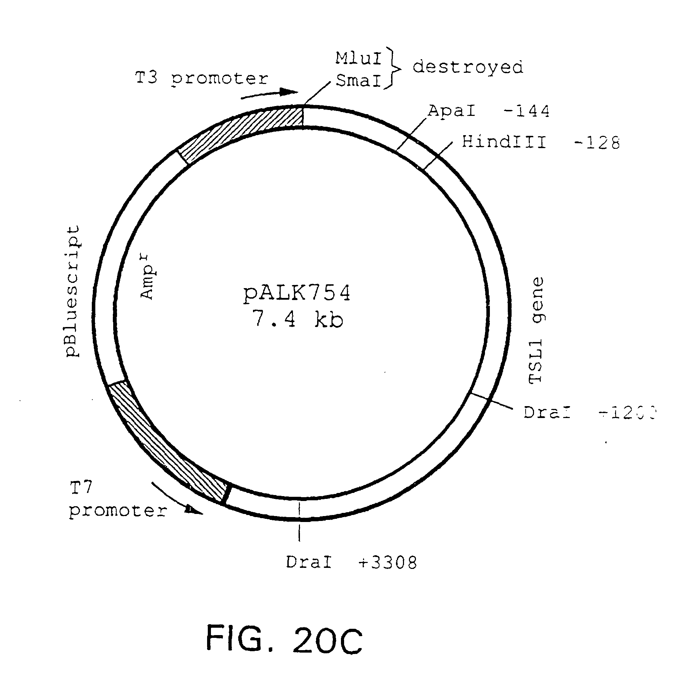

Fig. 20. Plasmids containing TSS1 and TSL1
   pBluescript containing (a) TSS1 with its own promoter, (b) TSS1 without its promoter and (c) TSL1 with its own promoter are shown.

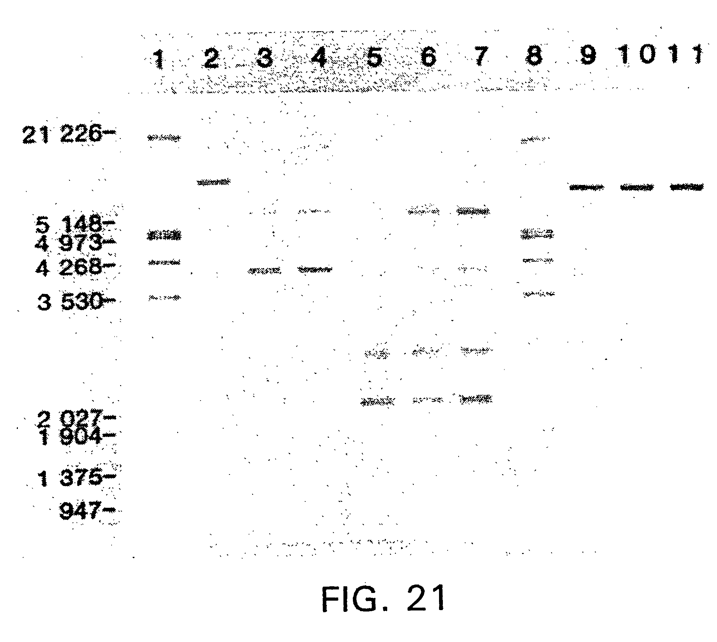

Fig. 21. Southern analysis of two tss1 disruptants of S.cerevisiae.
   ClaI digests of DNA from control yeast (S150-2B; lanes 2,5 and 9), and two tss1 disruptants, ALKO 3569 (lanes 3, 6 and 10) and ALKO 3570 (lanes 4,7 and 11) were probed with TSS1 (lanes 2 to 4), LEU2 (lanes 5 to 7) and TSL1 (lanes 9 to 11). Lanes 1 and 8 contain DNA standards

### DETAILED DESCRIPTION OF THE INVENTION:

In the following description, trehalose-6-phosphate synthase (TPS) and trehalose-6-phosphate phosphatase (TPP) refer to catalytic activities, not to proteins, unless specifically stated otherwise, whereas trehalose synthase refers to a protein that can convert uridine diphosphoglucose (UDPG) and glucose-6-phosphate (G6P) into trehalose, and also exhibits as partial reactions TPS and TPP activities. TSS1, TSL1, and TSL2 are structural genes that encode, respectively, the short (57 kDa) and the about 130 and 99 kDa long chains of trehalose synthase. It is well known that mutations occur in genes and can cause changes in the amino acid sequence of the encoded polypeptide. Changes can also be introduced by genetic engineering techniques. As used herein, the term TSS1 (or TSL1 or TSL2) gene includes all DNA sequences homologous with the sequences herein disclosed for TSS1 (or TSL1 or TSL2) and encoding polypeptides with the functional or structural properties of the 57 kDa (or about 130 kDa or 99 kDa, respectively) polypeptide. Sequences articially derived from these genes but still encoding polypeptides with the desired functional or structural properties are also included.

The present inventors previously reported the isolation of a partially degraded protein preparation that contained a short (57 kDa) polypeptide chain and two fragments (86 and 93 kDa) of a long polypeptide chain and possessed both TPS and TPP catalytic activities [Londesborough, J & Vuorio, O. (1991) Journal of General Microbiology 137, 323-330]. The size of the full-length intact long chain, from which both the 86 and 93 kDa fragments were then believed to be derived, and whether one or other polypeptide possessed one or other of the catalytic activities were not known at that time.

The inventors have now isolated an undegraded trehalose synthase that contains the 57 kDa short chain, and two long chains of about 130 kDa and 99 kDa as its major polypeptides. Traces of other polypeptides are also present that appear to be degradation products of the about 130 and 99 kDa chains. Two genes, TSS1 and TSL1, that encode, respectively, the short and about 130 kDa long chains have been cloned and sequenced. Because the size of this long chain is now known from its gene to be 123 kDa, it is hereafter called the 123 kDa long chain. TSS1 encodes a polypeptide with a theoretical molecular weight of 56.2 kDa; however, this short chain and the 99 kDa long chain are still called after their apparent molecular weights by SDS-PAGE analysis, the error in such analyses being at least ± 10 kDa at 130 kDa.

The sequences of TSS1 and the polypeptide it encodes are disclosed as SEQ ID NOS:1 and 2, respectively. The sequences of TSL1 and the polypeptide it encodes are disclosed as SEQ ID NOS:83 and 82, respectively (earlier versions of these sequences, lacking the 5'- and N-terminal regions, are listed as SEQ ID NOS:3 and 4). Genetic evidence is disclosed that shows that a functional TSS1 gene is involved in the expression of both TPS and TPP catalytic activities in S. cerevisiae: (1) both activities are absent from a mutant strain (Klg 102) that lacks a properly functional TSS1 gene and does not express the short chain in a form recognizable in Western blots although it does express immunologically recognisable long chain; (2) disruption of TSS1 eliminates TPS and TPP activities, abolishes the short chain signal from Western blots and prevents the accumulation of trehalose, and these defects are simultaneously reversed by transformation with TSS1, which also increased the resistance of the cells to freezing stress; and (3) transformation of Escherichia coli with TSS1 causes a large increase in the TPS activity of the transformants (but no detected increase in their TPP activity).

We disclose biochemical evidence that the TPP catalytic activity of a truncated trehalose synthase requires a functional long chain: incorporation of about 1 mole of ¹⁴C-N-ethylmaleimide into the 93 kDa long chain fragment per mole of truncated trehalose synthase results in complete loss of TPP activity but only a slight loss of TPS activity. Furthermore, we have been able to isolate the 99 kDa polypeptide and show that it possesses residual TPP activity but no TPS activity. Also, intact trehalose synthase is partially resolved into a 99 kDa-enriched form with a relatively high TPP/TPS ratio and a 123 kDa-enriched form with a lower TPP/TPS ratio. However, truncation of the 123 kDa long chain has dramatic and important effects on the TPS activity of trehalose synthase: removal of the N-terminal 330 or so amino acids decreases the sensitivity of the TPS catalytic activity to inhibition by phosphate and almost eliminates its activation by fructose-6-phosphate. Further, transformation of E. coli with TSL1 causes an increase in the TPS activity of the transformants (but no detected increase in their TPP activity).

Thus, both the short and the long chains make essential contributions to both the TPS and the TPP catalytic activities of trehalose synthase. The situation is therefore that there are at least two different structural genes for a trehalose synthase, neither of which can be completely described as the structural gene of either a trehalose-6-phosphate synthase protein or a trehalose-6-phosphate phosphatase protein.

We disclose that the amino acid sequences of peptides isolated from both the 86 and 93 kDa long chain fragments found in the truncated enzyme described by Londesborough & Vuorio [(1991) loc. cit] are encoded by TSL1. Surprisingly, however, none of the peptides isolated from the 99 kDa polypeptide in the intact enzyme is encoded by TSL1. Therefore, the structural genes encoding polypeptides of yeast trehalose synthase include a third member, TSL2. The 99 kDa polypeptide encoded by TSL2 was not visible in SDS-PAGE analyses of truncated enzyme. However, one of the 6 peptides isolated from the 93 kDa fragment was not encoded by TSL1 and had an amino acid sequence also found in a peptide isolated from the 99 kDa polypeptide. Thus, traces of a degradation product of the 99 kDa polypeptide are present in truncated enzyme, and migrate with the 93 kDa fragment during SDS-PAGE.

The inventors have not yet sequenced this third structural gene, TSL2, but disclose information that provides obvious methods for its isolation and cloning by a person ordinarily skilled in the art. Also a clone (pALK7756) comprising at least part of this gene has been deposited (Accession number, DSM 7425; Deutsche Sammlung von Mikroorganismen und Zellkulturen, Mascheroder Weg 1 B, D-3300 Brauschweig)

We disclose that the genes TSS1 and TSL1 contain extensive similarities such that the amino acid sequence of the entire short chain is 37 % identical to residues 495 to 814 of the long chain.

A novel feature of the present invention, therefore, is that in order to increase the capacity of a yeast or some other host organism for trehalose synthesis it can be necessary to increase the expression of both the TSS1 and the TSL1 and TSL2 genes or modify these genes in some other way, not because either TPS or TPP activity is "rate-limiting", but because more than one gene affects each activity. Thus, the results summarised above disclose that both TSS1 and TSL1 affect TPS activity and both TSS1 and TSL2 affect TPP activity. However, these results also disclose that the TSL2 gene product (the 99 kDa polypeptide) isolated by chromatography is itself a trehalose-6-phosphatase whereas the TSS1 gene product expressed in E. coli is a trehalose-6-phosphate synthase, although the catalytic efficiency of these separate polypeptides can be less than when they are correctly assembled in a trehalose synthase complex.

A surprising finding was that the TSS1 gene is identical with a gene variously called FDP1 or CIF1. This gene has pleiotropic effects on the utilization of sugars by S. cerevisiae. In particular, haploid yeast bearing certain alleles of this gene (the so-called fdp1 and cif1 mutants) are unable to grow on mannose, or on mannose or sucrose, or on mannose, sucrose or fructose, or on mannose, sucrose, fructose or glucose, depending upon the severity of the defect [Van de Poll & Schamhart, (1977) Molecular and general Genetics 154, 61-66; Ba 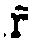 uelos, M. & Fraenkel, D.G. (1982) Molecular and Cellular Biology 2, 921-929]. Such mutants grow normally on galactose. Therefore, during the selection of strains in which the TSS1 gene has been deleted or modified it is sometimes essential and always advisable to grow the transformants on galactose, because in many cases the desired transformant will be unable to grow on any other common sugar, including the routinely used glucose. This is an unexpected methodological consideration that would not be obvious even to a person skilled in the art: special knowledge about the sequence and chromosomal location of the TSS1 gene is required, which we now disclose.

Since our disclosure of the identity of TSS1 with FDP1 and CIF1 in USPA 841,997, a confirmation has been published by Bell, W., et al. [(1992) European Journal of Biochemistry 209, 951-959)]

The inventors' previous work [Londesborough & Vuorio (1991) loc. cit.] showed that the TPS catalytic activity of what is now known to be trehalose synthase requires a so-called TPS-Activator protein, which is a dimer of 58 kDa subunits. We have identified this protein by the amino acid sequences of peptides it contains and by its catalytic activity and disclose that it is yeast phosphoglucoisomerase. We disclose that fructose-6-phosphate (F6P), which could be made by phosphoglucoisomerase from the glucose-6-phosphate (G6P) in the assay mixtures used to measure TPS activity, is a powerful activator of the TPS activity of intact trehalose synthase. Also, when the assay mixture contains an equilibrium mixture of G6P and F6P the TPS-Activator has no further effect, so that its phosphoglucoisomerase activity is a complete explanation of the activation it causes. Furthermore, the TPS activity of truncated trehalose synthase does not require F6P, and is not so strongly inhibited by phosphate as is that of the native enzyme. Thus, a trehalose synthetic pathway can in principle be transferred to any organism by transforming the organism with the structural genes for yeast trehalose synthase: it is not necessary to simultaneously introduce the TPS-activator, because F6P is a ubiquitous component of cells. Furthermore, if the amounts of F6P in an organism are inadequate, or phosphate concentrations are too high, the organism can be transformed with a truncated version of TSL1 encoding the truncated long chain that confers insensitivity to phosphate and F6P. This aspect of the present invention is particularly significant, because it both allows the introduction of a trehalose synthetic pathway to organisms in which the cytosolic phosphate and F6P concentrations would prevent the efficient function of yeast trehalose synthase, and also may permit trehalose synthase to function efficiently at stages of yeast growth when native trehalose synthase would be inhibited by cytosolic phosphate. We disclose that intact trehalose synthase can be liberated from phosphate inhibition by treatment with trypsin in vitro.

From the knowledge gained from the present invention, it is possible to produce trehalose recombinantly by transforming a host cell with the appropriately modified TSS1, TSL1 and TSL2 genes. Methods of transformation and appropriate expression vectors are well-known in the art.

Expression vectors are known in the art for both eukaryotic and prokaryotic systems, and the present invention contemplates use of both systems. For transformation of yeast at least two classes of promoters are contemplated. Yeast that accumulates more trehalose but at the usual time (i.e., after consumption of fermentable carbon sources) can be made by inserting extra copies of the genes under their own promoters, or stronger promoters with similar control. Such yeast can have improved storage properties and stress resistance and be more economic sources of trehalose. Yeast that synthesizes trehalose during fermentation can be made by replacing the genes' own promoters with promoters (such as ADH1) that are active during fermentation. As explained above, such yeast can have increased fermentation rates, ethanol yields and resistance to osmotic and temperature stress during fermentation.

Also contemplated are modifications of the DNA sequence which would provide "preferred" codons for particular expression systems (e.g., bacteria and higher plants). In addition, the TSS1, TSL1 and TSL2 DNA sequences may be modified by certain deletions or insertions, provided the translated polypeptides are enzymatically functional. Expression of functional polypeptides from TSS1, TSL1 and TSL2 may be confirmed by assaying for TPS and/or TPP activity in the expression system by the methods described in Londesborough and Vuorio [(1991) loc. cit.]. Deletion of the first 330 amino acids or so from the 123 kDa long chain to give an enzyme active at higher phosphate and lower F6P concentrations has already been mentioned.

The genes of the present invention may be transferred and expressed in plants by using the Ti plasmid system which is well known in the art. The internal transforming genes of a cloned T-DNA can be removed by recombinant DNA techniques and replaced by the genes of the present invention and expressed in plant tissues. Commonly, the coding sequence of the foreign gene (for instance, TSL1) is substituted for the coding region of the opine synthetase gene. In this way, the natural promoter and polyadenylation signals of the opine synthetase gene confer high-level expression of the foreign protein. Any method known in the art, however, may be used to transform higher plants with the genes of the present invention.

The following examples are for illustration of the present invention and should not be construed as limiting the present invention in any manner.

### EXAMPLES

### General Methods and Materials.

Materials. Fructose 6-phosphate (F6P) and adenosine 5'-diphosphoglucose (ADPG) were from Sigma Chemicals. Glucose 6-phosphate (G6P), phenylmethylsulphonyl fluoride (PMSF), uridine 5'-diphosphoglucose (UDPG) and other commercial reagents were from the sources stated in Londesborough Vuorio [(1991) loc. cit.]. Truncated trehalose synthase (proteolytically activated "TPS/P") and TPS activator were prepared as described in Londesborough & Vuorio [(1991) loc. cit.]. The antisera, anti-TPS/P, anti-57K and anti-93K were made in rabbits using as antigen, respectively, truncated trehalose synthase, the short (57 kDa) chain and the 93 kDa fragment of the long chain of trehalose synthase as described in Londesborough & Vuorio [(1993) loc. cit.].

Buffers for enzyme extraction and purification. Two standard cocktails, HBMED (25 mM Hepes/KOH pH 7.0/1 mM benzamidine/1 mM MgCl₂/0.1 mM EDTA/1 mM dithiothreitol) and HB2M1ED (HBMED but with final concentrations of 2 mM MgCl₂ and 1 mM EDTA) were used as basal buffers during preparation of cell extracts and purification of enzyme. Where indicated, the Hepes and benzamidine concentrations were increased to 50 mM and 5 mM, respectively.
Yeasts. Commercial baker's yeast was from Alko's Rajamäki factory. The standard laboratory strains of S. cerevisiae used were X2180 (ATCC 26109) and S288C (ATCC 26108). Mutant strains are described in the Examples and Table 1 lists important strains of microorganisms and plasmids. Laboratory yeast were routinely grown on 1 % yeast extract/2% peptone (YP) containing the indicated carbon source in aerobic shake flasks at 30 °C and 200 r.p.m. Cells were harvested by centrifugation for 5 minutes at 3000 g, resuspended in distilled water and again centrifuged 5 minutes at 3000 g. The pellets were suspended in about 20 volumes of HB2M1ED and centrifuged in tared tubes for 10 minutes at 15,000 g. Tubes and pellets were weighed to give the mass of fresh yeast. For trehalose determinations, portions of the pellets were treated as described by Lillie, S.H. & Pringle, J.R. [(1980) Journal of Bacteriology 143, 1384-1394]. The washed cells were broken by suspending them at 0 °C in 1 to 4 volumes of HB2M1ED, adding fresh stock PMSF/pepstatin (1 mg pepstatin A/ml 0.1 M PMSF in methanol) to give final concentrations of 10 µg pepstatin/ml and 1 mM PMSF, and shaking with glass beads for three 1 minute periods in a Braun MK II homogenizer or (for amounts less than 0.3 g fresh yeast) by vortexing in an Eppendorf tube. The glass beads were removed and the volume of homogenate was measured. Samples for SDS-PAGE were made at once by dilution with Laemmli sample buffer [Laemmli, U.K. (1970) Nature, London 227, 680-685]. The homogenates were then centrifuged as indicated (usually 5 min at 5,000 g or 20 minutes at 28,000 g). Enzyme assays were made on the homogenates and supernatants and protein determined in the supernatants from A280 and A260 measurements.

**Table 1.**

| List of important strains and plasmids | | |
|---|---|---|
| Name | Description | Source |
| Saccharomyces cerevisiae | | |
| X2180 (ATCC 26109) | Standard laboratory yeast (diploid) | - |
| | | |
| S288C (ATCC 26108) | Standard laboratory yeast (haploid) | - |
| | | |
| Klg 102 | cif1-102, leu1, ura1, trp5, MATα | 1 |
| | | |
| MV6807 | fdp1, leu2, ura3, his3, lys2, ade8, trp1, MATα | 2 |
| | | |
| S150-2B | leu2, his3, trp1, ura3, Mata | - |
| | | |
| ALKO3569 | tss1::LEU2 (from S150-2B) | This work |
| | | |
| ALKO3570 | tss1::LEU2 (from S150-2B) | This work |
| | | |
| WDC-3A | cif1::HIS3, his3, ura3, ade2, MATα | 3 |

| Escherichia coli | | |
|---|---|---|
| HB101 (ALKO 683) | | |
| ALKO3566 | HB101 containing pALK752 | This work |
| | | |
| ALKO3568 | HB101 containing pALK754 | This work |

| Plasmids | | |
|---|---|---|
| pALK751 (DSM 6928) | pBluescript containing an 8.2 kb insert comprising TSL1 | This work |
| | | |
| pALK752 | pBluescript containing a 2.5kb insert comprising TSS1 | This work |
| | | |
| pALK753 | pBluescript containing a 3.3 kb insert comprising the ORF of TSS1 | This work |
| | | |
| pALK754 | pBluescript containing a 4.4 kb insert comprising TSL1 | This work |
| | | |
| pALK756 (DSM 7425) | pBluescript containing a 3.5 kb insert comprising at least part of TSL2 | This work |
| | | |
| pALK757 | pBluescript containing an insert comprising the ORF of TSL1 | This work |
| | | |
| pMB14 | YEp352 containing CIF1 | 3 |
| Sources: 1. Dr. D. Fraenkel, Harvard Medical School, U.S.A. 2. Dr. J. Thevelein, Lab voor Plantenbioch., Heverlee Belgium. 3. Dr. C. Gancedo, CSIC, Madrid, Spain. | | |

Enzyme Assays. TPP and TPS standard assays and other kinetic measurements were made as described by Londesborough & Vuorio [(1991) loc. cit.] except that the standard TPS assay mixture contained 5 mM F6P unless stated otherwise. Where appropriate, TPS assays were corrected by measuring UDP production from UDPG in the absence of G6P and F6P.

DNA manipulations. Stratagene's (La Jolla, California) Escherichia coli strain XL-1 Blue {recA1, endA1, gyrA96, thi, hsdR17, supE44, relA1, lac, [F' proAB, lacIq ADM15, Tn10 (tetR)]} were used as host bacteria. When needed, XL-1 Blue cells were made competent by the method of Mandel & Higa (1970) Journal of Molecular Biology 53, 159-162]. The cloning vector was Stratagene's Lambda Zap II, predigested with EcoRI, where the cloning site is near the N-terminus of the gene for β-galactosidase, thus enabling the color selection of recombinant clones. The sequencing vectors M13mp18 and M13mp19 from Pharmacia LKB Biotechnology were also used.

High molecular mass DNA from the haploid S288C strain was prepared as described Johnston, J.R. [(1988) in Yeast, A Practical Approach, IRL Press, Oxford) and partially digested with either HaeIII or EcoRI restriction enzyme. For the large scale HaeIII digestion, e.g., a reaction mixture of 330 µl containing 30 µg of DNA and 4.8 U of enzyme was incubated at 37 °C for 60 minutes. The reaction was stopped with 10 µl of 0.5 M EDTA and transferred to ice. The methods for such digestions and their agarose gel electrophoretic analysis are well known in the art and are described, e.g., in Sambrook et al., Molecular Cloning, A Laboratory Manual [Cold Spring Harbor Laboratory Press, 2nd ed., (1989)].

Plasmid DNA was isolated using standard methods for small scale purification Sambrook et al. [(1989) Molecular Cloning, A Laboratory Manual, 2nd ed., Cold Spring Harbor Laboratory Press, hereby expressly incorporated by reference]. Large scale purifications of plasmid DNA were done with Qiagen tip-100 columns from Diagen following their instructions.

DNA sequences were determined either manually by the dideoxy-chain termination method [Sanger et al. (1977) Proceedings of the National Academy of Sciences U.S.A. 74, 5463-5467], sequencing directly from pBluescript plasmids, or automatically with the Applied Biosystems Model 373A automatic DNA sequencer, sequencing either directly from these plasmids or from M13 subclones.

Southern and Western hybridizations and other standard manipulations were carried out by well known procedures [see, e.g., Sambrook et al. (1989) loc. cit.].

### Example 1. Purification of intact trehalose synthase

Intact trehalose synthase was purified from commercial baker's yeast. The method described by Londesborough & Vuorio [(1991) loc. cit.] for purification of "proteolytically activated TPS/P" was modified as follows:
1. All buffers contained 2 mM MgCl₂ and 1 mM EDTA. This increased yields in the early steps and probably helped to decrease proteolysis in the later steps.
2. In the first ammonium sulphate fractionation, the EDTA concentration was increased to 2.5 mM before addition of ammonium sulphate.
3. All buffers were adjusted to between 0.4 and 1 mM PMSF and between 4 and 10 µg pepstatin A/ml by addition, immediately before use, of the appropriate amount of a freshly prepared stock solution containing 1 mg pepstatin A/ml 0.1 M PMSF in methanol (called, stock PMSF/pepstatin). When, as in chromatography, buffers were used for several hours, more stock PMSF/pepstatin was added at intervals, but so as not to exceed 1.5 % methanol in the buffer, or a fresh lot of buffer was taken into use, because of the short half-life of PMSF in aqueous solution. All columns were equilibrated with at least one bed volume of buffer containing PMSF and pepstatin A immediately before application of enzyme.
4. Experience permitted the enzyme-containing fractions (a total of 17.8 ml in the preparation of Table 2) from Heparin-Sepharose to be identified as soon as they were eluted. Stock PMSF/pepstatin (150 µl) and 0.1 M EDTA (200 µl) were immediately added to them. Then 7.2 g of powdered ammonium sulphate was slowly added (over 20 min). After 30 min equilibration, the mixture was centrifuged 15 min at 28,000 g. The pellets were packed for 5 min at 28,000 g and expressed buffer was removed with a pasteur pipette. The pellets were dissolved to 2.0 ml in HB2M1ED containing 0.8 mM PMSF and 8 µg pepstatin A/ml, centrifuged 5 min at 28,000 g and applied to a 2.6 x 34 cm column of Sepharose 6B freshly equilibrated with HB2M1ED containing 50 mM NaCl, 0.4 mM PMSF and 4 µg pepstatin A/ml. The interval between elution from Heparin-Sepharose and application to Sepharose 6B was 5 h. In the Londesborough & Vuorio [(1991) loc. cit.] procedure, the Heparin-Sepharose eluates were stored at about 3 °C, without addition of PMSF or pepstatin A, for 5 days before the second ammonium sulphate fractionation and application to Sepharose 6B.
5. Fractions (3.7 ml) from the Sepharose 6B column were immediately mixed with 20 µl of stock PMSF/pepstatin and then assayed. Again, experience permitted the correct fractions to be pooled, based on activity and A280 measurements without SDS-PAGE analysis, and immediately applied to a 0.7 x 7 cm column of UDP-Glucuronate-Agarose equilibrated with HB2M1ED containing 50 mM NaCl, 0.4 mM PMSF and 4 µg pepstatin A/ml. The enzyme was eluted as described by Londesborough & Vuorio [(1991) loc. cit.] and 10 µl of stock PMSF/pepstatin added to each 1.7 ml fraction. Each fraction was divided into three. Two portions were stored at -70 °C and one at 0 °C.

Table 2 summarizes a purification and Fig. 1 shows the SDS-PAGE analysis of fractions eluted from UDP-Glucuronate-Agarose. No obvious differences were apparent between enzyme eluted by 0.2 M NaCl and that eluted by 10 mM UDPG/0.4 M NaCl. The major bands present had molecular masses of 57, 99 and 123 kDa. Several weaker bands were present between the 123 kDa band and about 90 kDa. In Western analyses the 123 kDa, 99 kDa and most, if not all, of the fainter bands in this region were recognized by the anti-TPS/P and anti-93K sera. This suggests that the fainter bands are partially degraded long chains. The weak bands at 68 kDa also reacted with the anti-93K serum, but could be removed by chromatography on DEAE-cellulose (see Example 9). When the antibodies from anti-93K serum that bound to the 99 kDa band were eluted from a nitrocellulose blot [as described by Pringle, J.R. (1991) Methods in Enzymolgy 194, 565-590] and used to probe another blot, they bound also to the 123 kDa band, showing that the two long chains of trehalose synthase have epitopes in common.

Intact enzyme binds less tightly to the UDP-Glucuronate-Agarose than the truncated enzyme purified by Londesborough & Vuorio [(1991) loc. cit.] and the proportion of enzyme remaining bound at 0.2 M NaCl varied from preparation to preparation. When #9 of Table 2 was re-run on the same column, 76 % of the TPS activity was again recovered at 0.2 M NaCl (and 25 % by 0.4 M NaCl/10 mM UDPG), so that overloading of the column is not the reason why this enzyme eluted at 0.2 M NaCl. However, when enzyme eluted at 0.2 M NaCl was truncated with trypsin as described in Example 10, it then bound to the column at 0.2 M NaCl and was only recovered at 0.4 M NaCl/10 mM UDPG. Thus, as well as altering the kinetic properties of the enzyme (see Examples 10 & 12), this truncation also increases the affinity for UDP-Glucuronate-Agarose. Presumably there are subtle differences in factors such as the amount of adventitious proteolysis and state of aggregation between enzyme eluted at 0.2 M NaCl and that remaining bound. For the preparation summarised in Table 2, the ratio of standard TPP and standard TPS activities increased from 22 % in #9 to 39 % in #14, showing that there are differences, even though they could not be clearly detected by SDS-PAGE.

These findings disclose that a highly purified trehalose synthase containing a 57 kDa short chain, a 123 kDa long chain and a 99 kDa polypeptide that is recognised by the anti-93K serum possesses both TPS activity activatable by TPSActivator protein (or F6P) and TPP activity. The rate of hydrolysis of 1 mM G6P in either phosphate or Hepes buffer was less than 1 % of that of 0.5 mM trehalose-6-phosphate, so that the TPP activity is highly specific. An unexpected finding is that this highly purified preparation contains the 99 kDa polypeptide, which is not present in the purified truncated trehalose synthase. It is disclosed later that this polypeptide is not a degradation product of the long (123 kDa) chain, whereas both the 86 and 93 kDa polypeptides of truncated enzyme contain amino acid sequences that identify them as fragments of the long (123 kDa) chain. This novel preparation possesses some unexpected catalytic properties, which are described in more detail in Example 11.

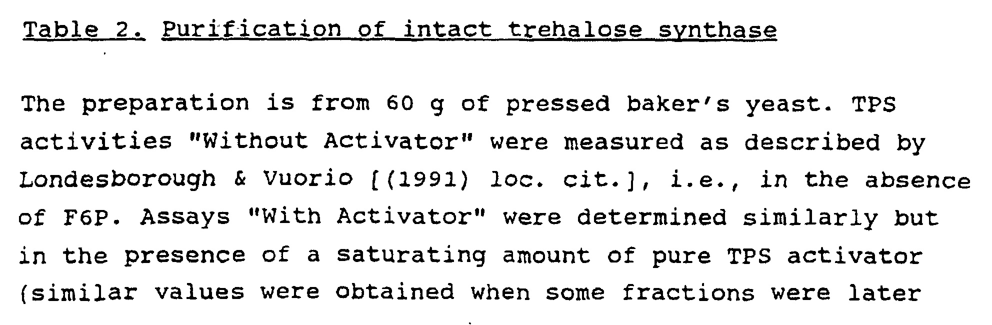

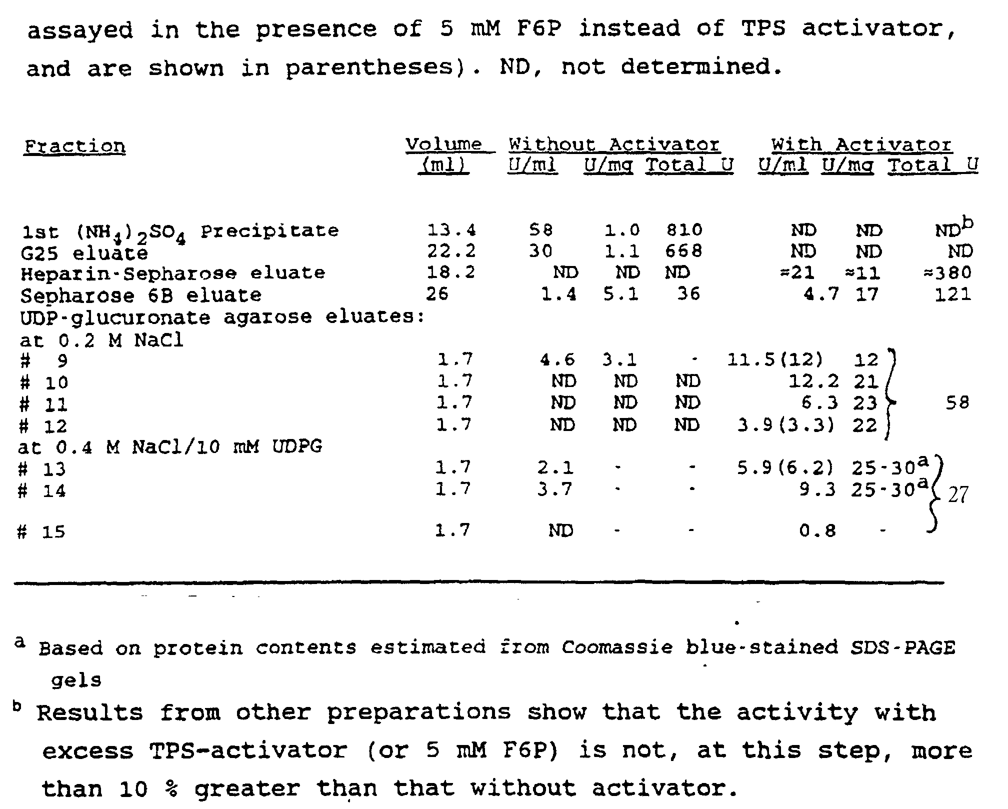

### Example 2 Increased expression by S. cerevisiae of the long and short chains of trehalose synthase after consumption of glucose

Three 500 ml lots of YP/2 % glucose in 1 l shake flasks were each inoculated with 1 ml of a suspension of X2180 cells of A600 1.0 and shaken at 200 r.p.m. at 30 °C. At the times shown in Table 3, the cells were harvested, broken and analyzed as described in General Materials and Methods. The 28,000 g supernatants were stored for a week at -18 °C, thawed and re-centrifuged for 20 min at 28,000 g. Portions of 150 µl (each equivalent to 53 mg of fresh yeast) were mixed with 30 µl of anti-TPS/P serum, equilibrated for 30 min at 0 °C and centrifuged for 10 min at 10,000 g. The pellets were washed with 250 µl of HBMED and then dissolved in Laemmli sample buffer and subjected to SDS-PAGE (Fig 2). Bands at 57, 99 and 123 kDa were strong in the sample (C) from stationary phase yeast and in the sample (B) harvested immediately after disappearance of glucose from the medium, but were absent or very weak in the sample (A) from yeast growing in the presence of 1.2 % glucose.

**Table 3.**

| Appearance of TPS and TPP activities in X2180 yeast grown on YP/2 % qlucose. | | | |
|---|---|---|---|
| Enzymes were assayed in the 28,000 g supernatants. | | | |

| | A | B | C |
|---|---|---|---|
| Age (h) | 16.1 | 18.1 | 39.0 |
| Residual glucose (g/100 ml medium) | 1.2 | ≤0.001 | ≤0.001 |
| Fresh yeast mass (mg/ml medium) | 7.6 | 14.8 | 29.5 |
| Trehalose (mg/g dry yeast) | 0.73 | 3.1 | 94 |
| TPS (U/g fresh yeast) | 1.2 | 7.4 | 10.5 |
| TPP (U/g fresh yeast) | 0.29 | 2.2 | 3.0 |
| TPP/TPS (%) | 24 | 30 | .29 |

Control experiments (not shown) indicated that pre-immune serum did not precipitate the 57, 99 and 123 kDa bands, and that using 50 µl of serum instead of 30 µl did not precipitate more of these three bands from the C sample.

These results disclose that the co-ordinate, 7-fold increase in TPS and TPP activities that occurs during less than 2 h when glucose disappears from the medium is accompanied by increases in the amounts in yeast of three polypeptides, of mass 57, 99 and 123 kDa, that are immunoprecipitated by anti-TPS/P serum. These polypeptides are those found in the intact trehalose synthase purified in Example 1. Thus, increase in the amount of enzyme protein is a major mechanism by which the capacity of yeast to synthesize trehalose is increased.

### Example 3 Determination of the N-terminal amino acid sequences of peptides isolated from the various polypeptides of trehalose synthase

The 57, 86 and 93 kDa polypeptides of the truncated trehalose synthase were separated by SDS-PAGE, digested on nitrocellulose blots and fractionated by HPLC as described by Londesborough & Vuorio [(1991) loc. cit.]. Also, these polypeptides and polypeptides of molecular mass 57, 99 and 123 kDa immunoprecipitated from yeast extracts as described in Example 2 were separated by SDS-PAGE and digested in the gel with lysylendopeptidase C as described by Kawasaki, H., Emori, Y. and Suzuki, K. (in press). The derived peptides were separated by HPLC using a DEAE pre-column before the reverse-phase column essentially as described by Kawasaki et al [(1990) Analytical Biochemistry 186, 264-268]. The 99 kDa polypeptide isolated by chromatography on phosphocellulose in the absence of triton (see Example 9) was digested with lysylendopeptidase C and the peptides separated by HPLC. In all cases, isolated peptides were sequenced in a gas-pulsed liquid phase sequencer as described by Kalkinen, N. & Tilgman, C [(1988) Journal of Protein Chemistry 7, 242-243], the released PTH-amino acids being analysed by on-line, narrow-bore, reverse-phase HPLC. The sequences are shown in Table 4.

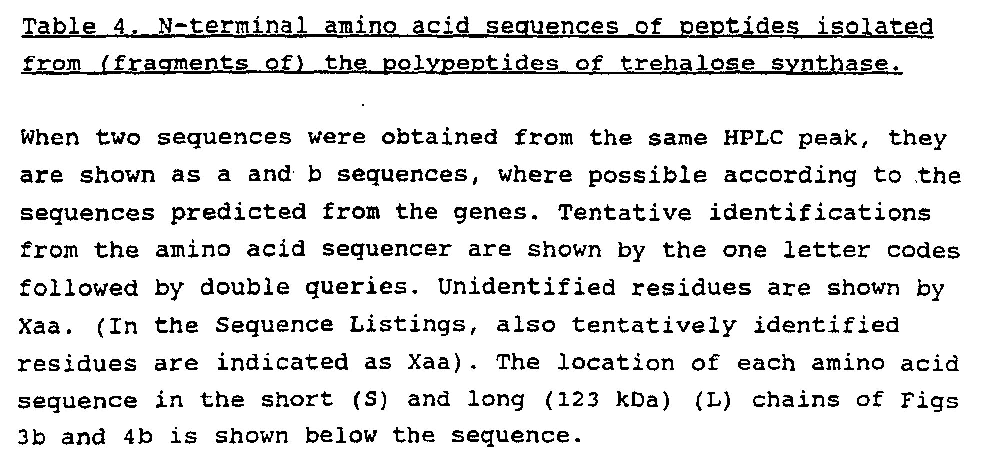

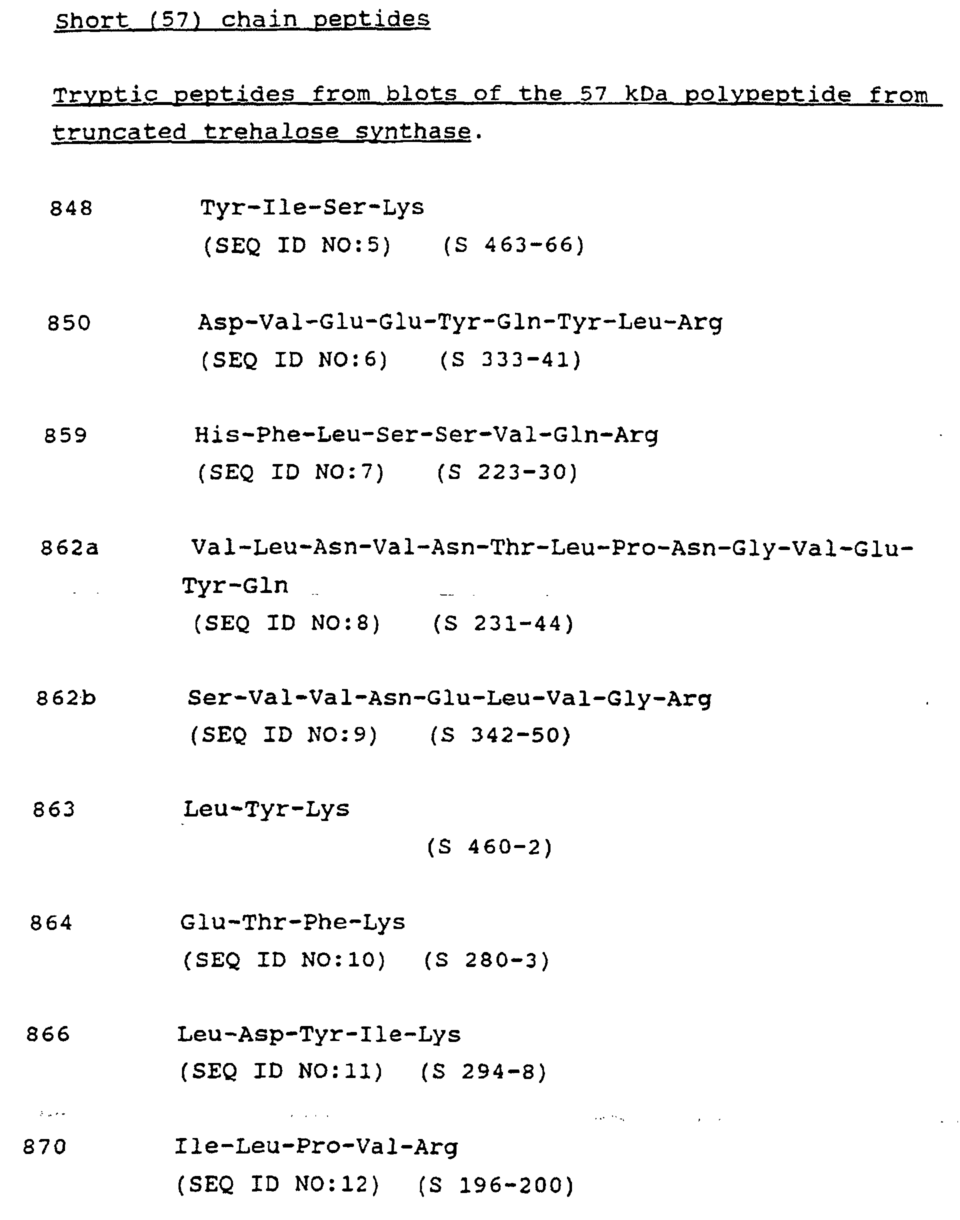

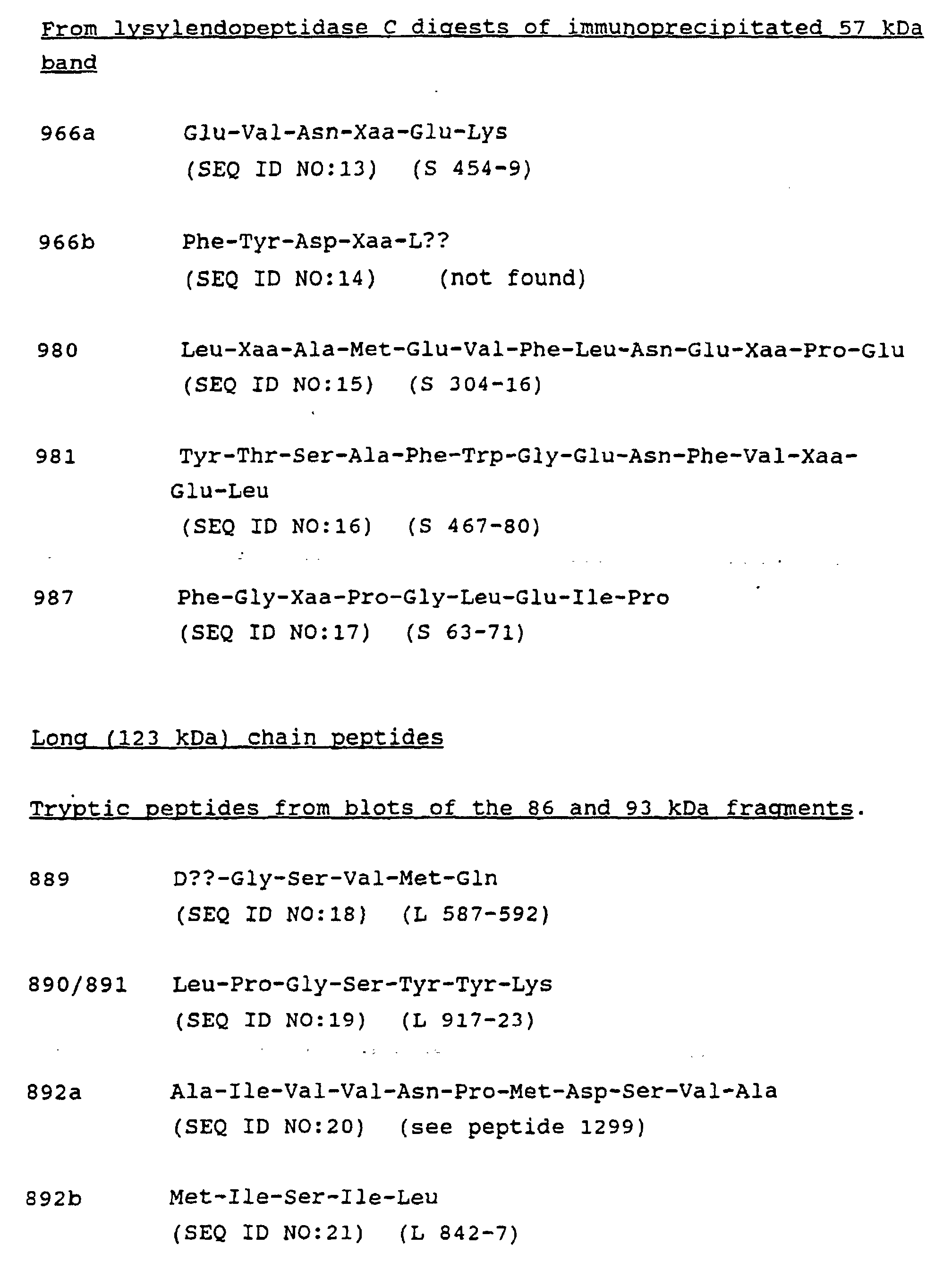

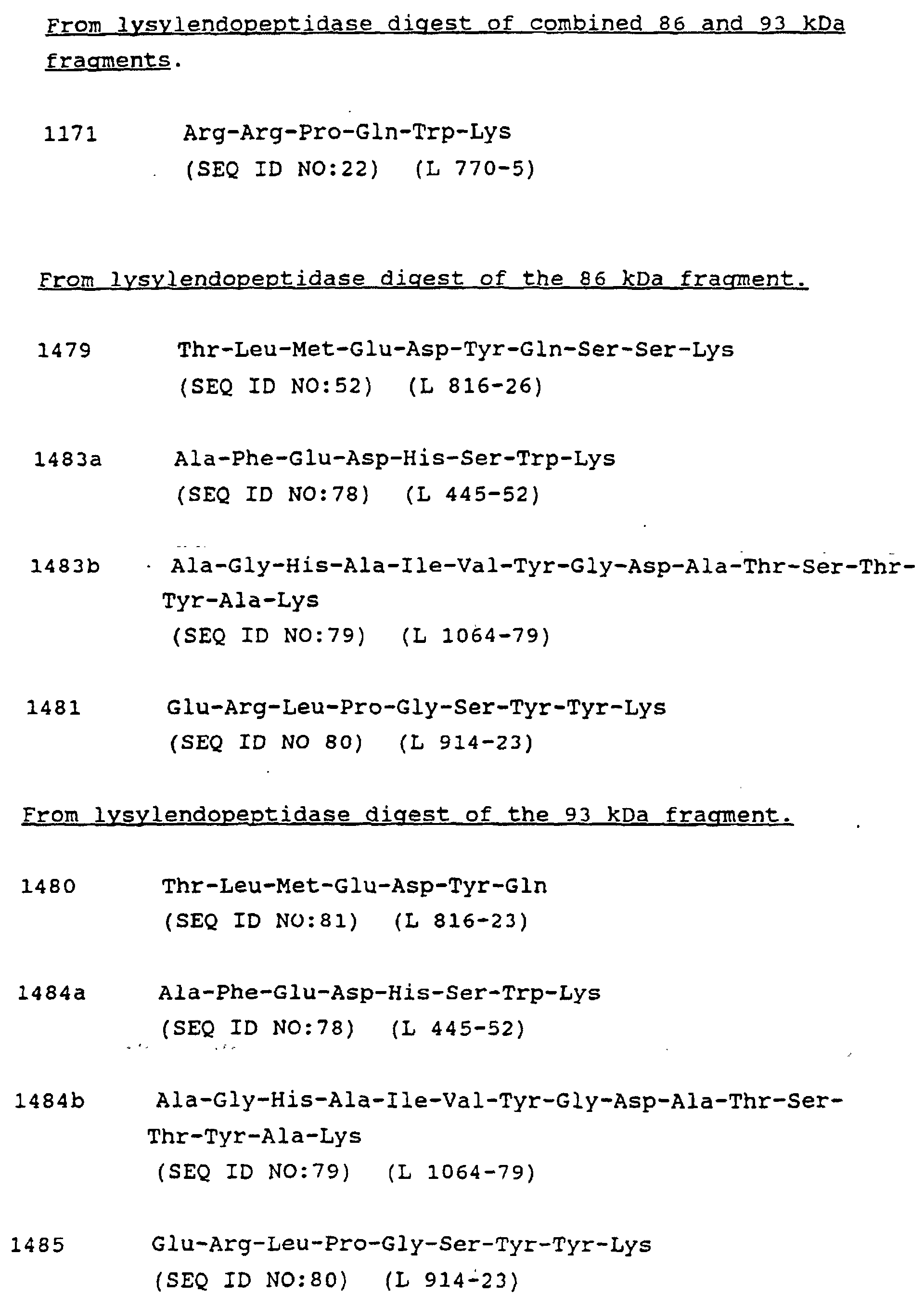

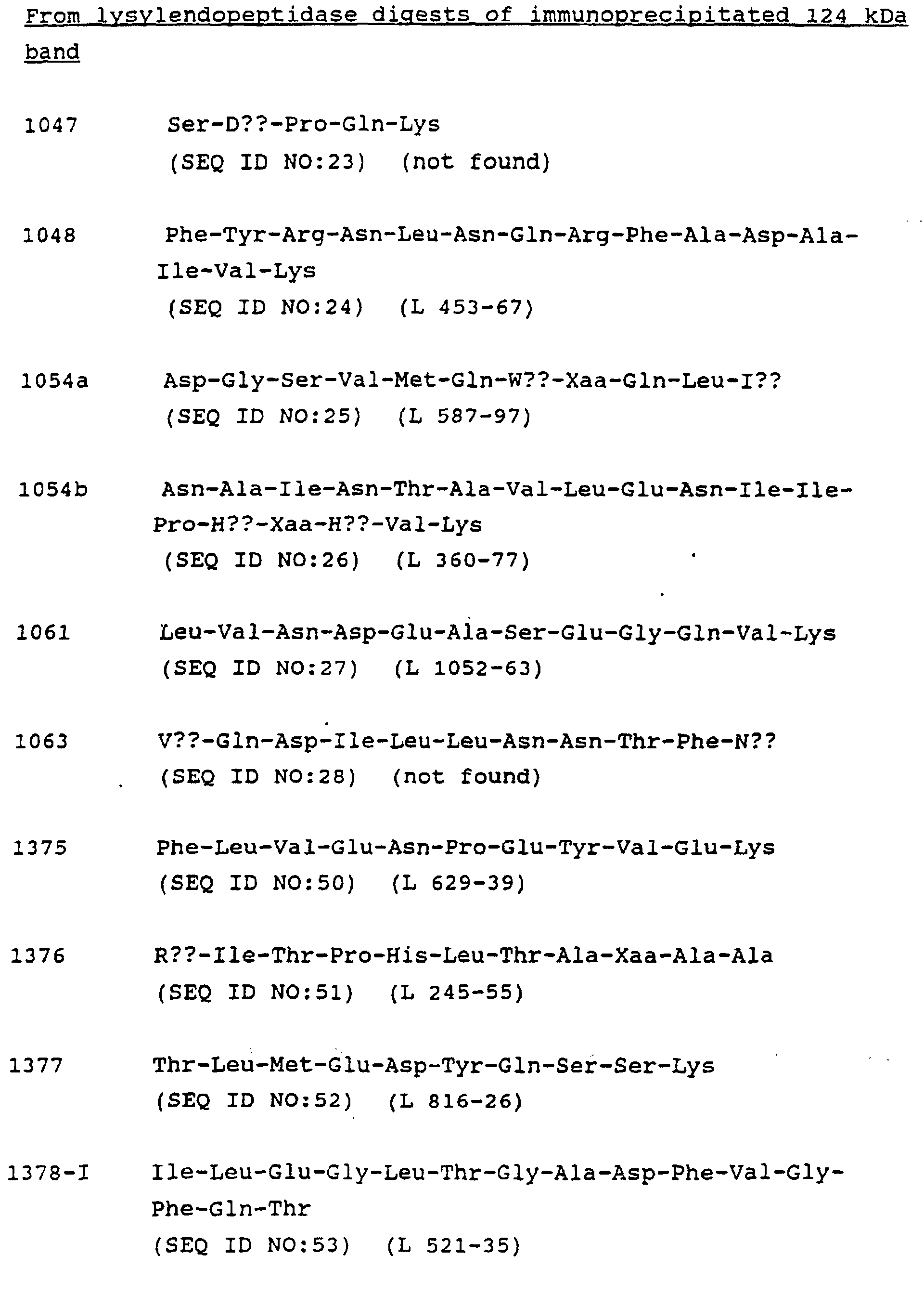

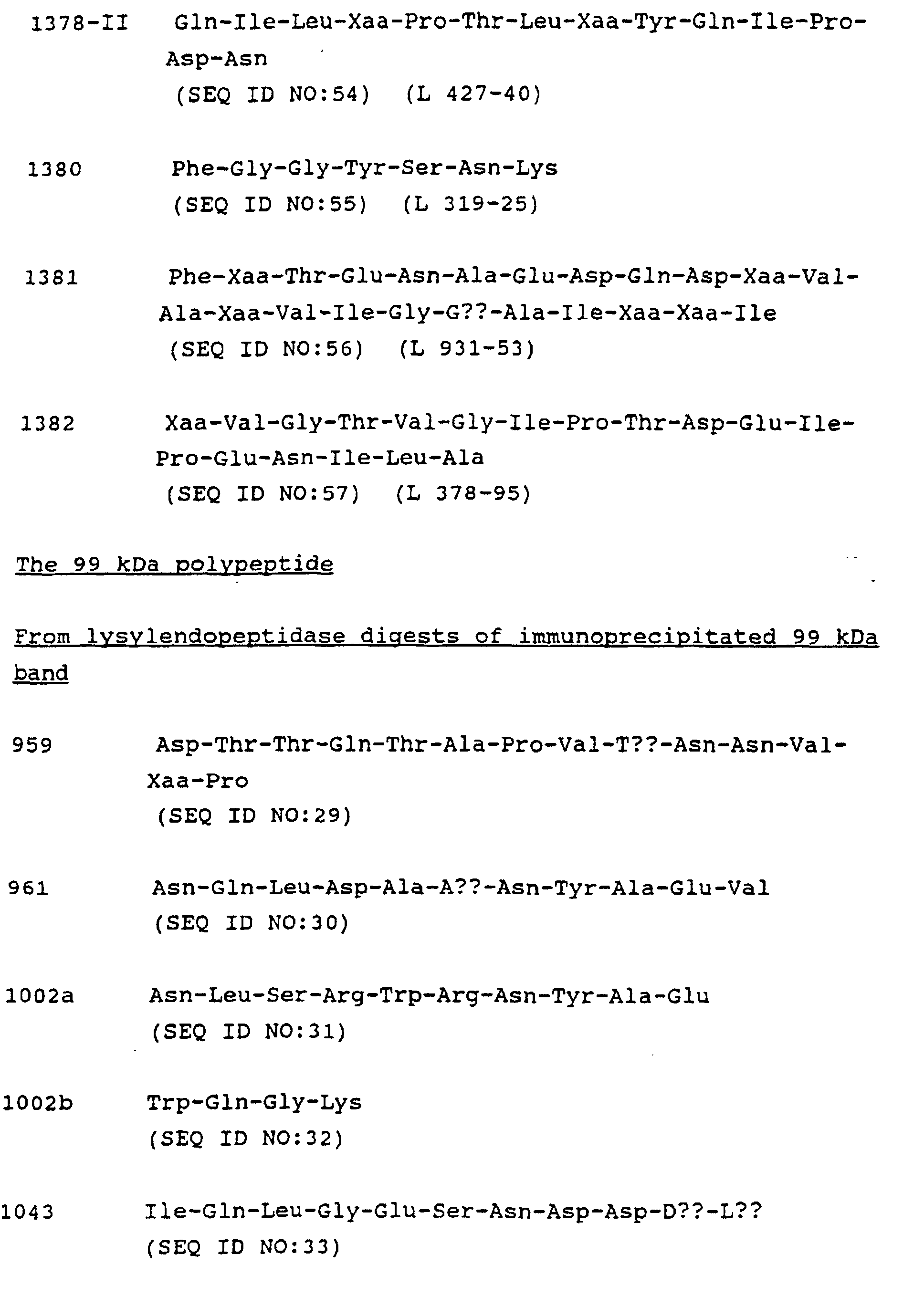

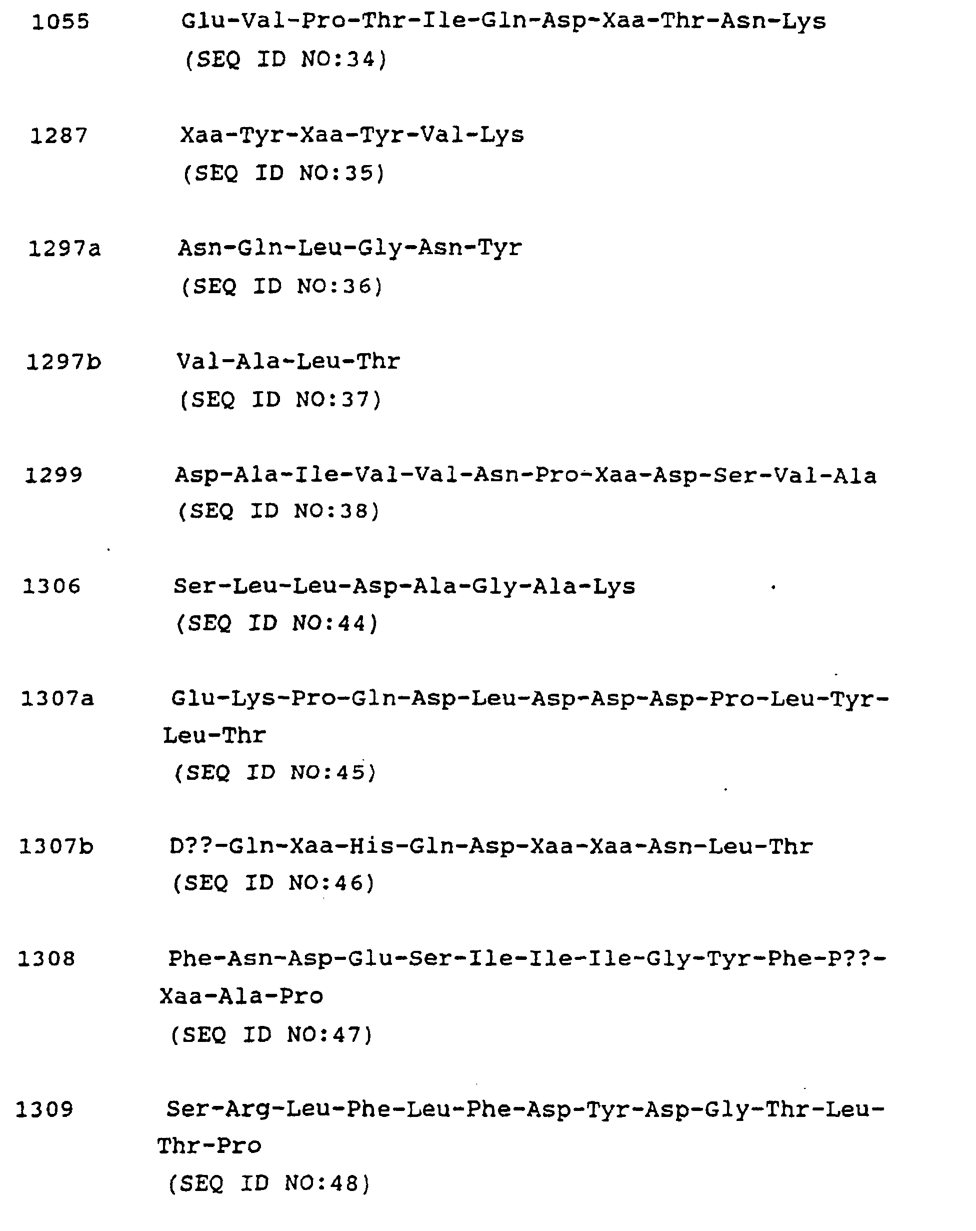

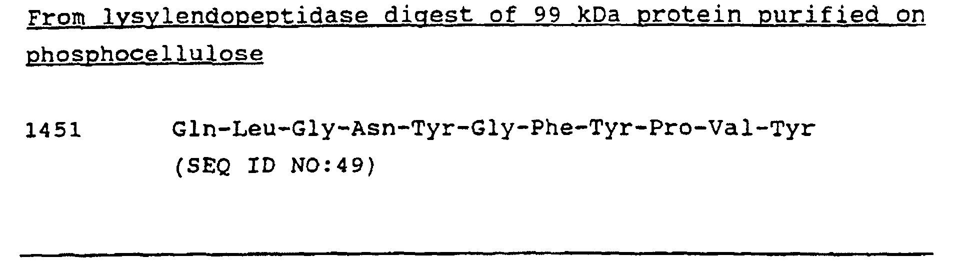

Apart from peptide 966b, all the amino acid sequences determined from the short chain samples have been located in the protein sequence deduced from the TSS1 gene (see Figure 3b). Apart from peptides 892a, 1047 and 1063, all the amino acid sequences determined from the 86 and 93 kDa fragments of the long chain and from the intact 123 kDa long chain itself have been located in the protein sequence deduced from TSL1. The HPLC profiles obtained from digests of the 86 kDa fragment were essentially identical with those from digests of the 93 kDa fragment when either trypsin or lysylendopeptidase C was used (not shown). Also, corresponding HPLC peaks from 86 and 93 kDa digests yielded the same sequences or double sequences (peptide pairs 890 & 891; 1479 & 1480; 1483a,b & 1484a,b; 1481 & 1485). These results disclose that both the 86 and 93 kDa polypeptides in truncated enzyme are derived from the 123 kDa long chain encoded by TSL1. In particular, it is not the case that one or other of these fragments is derived from the 99 kDa polypeptide, although contamination with minor amounts of (degradation products of) that polypeptide is probable (see below).

None of the 16 amino acid sequences obtained from the 99 kDa polypeptide is encoded by TSL1. The first 5 residues of peptide 1451 from the 99 kDa polypeptide purified on phosphocellulose are identical with the last 5 residues of peptide 1297a from immunoprecipitated 99 kDa polypeptide. This confirms that the 99 kDa polypeptide immunoprecipitated by anti-TPS/P serum from yeast extracts is the same as the 99 kDa polypeptide in purified intact enzyme. These results disclose that the 99 kDa polypeptide is not encoded by TSL1 (or TSS1) but by another gene, which the inventors call TSL2.

The origin of peptides 1047 and 1063 found in the digest of the intact (123 kDa) long chain is not known. The only peptide from the long chain fragments of truncated enzyme not encoded by TSL1 is 892a from the 93 kDa fragment. This is identical with the last 11 residues of peptide 1299 from the 99 kDa polypeptide. This suggests that the 93 kDa band was contaminated with some material derived from the 99 kDa polypeptide, although this polypeptide itself was not visible in SDS-PAGE analyses of the truncated enzyme. The identical HPLC profiles of digests of the 86 and 93 kDa fragments and the fact that only one peptide derived from the 99 kDa polypeptide was identified in these digests shows that the contamination was at a low level. This discloses that a functional truncated trehalose synthase with both TPS and TPP activities probably requires only polypeptides encoded by TSS1 and TSL1.

### Example 4 cloning and sequencing of TSS1

### (a) Preparation and screening of a yeast genomic DNA library

A genomic library was constructed in the bacteriophage lambda vector, Lambda Zap II, using a partial HaeIII digest of S. cerevisiae strain S288C chromosomal DNA, according to Stratagene's Instruction Manual for the Zap-cDNA synthesis kit. The DNA from the ligation reaction was packaged into Giga II Gold packaging extract (Stratagene) according to the manufacturer's instructions (1990). The titer of the recombinants was determined on Luria broth plates containing X-β-galactoside (5-bromo-4-chloro-3-indoyl-β-D-galactopyranoside as a chromogenic substrate for β-galactosidase and IPTG (isopropyl β-D-thiogalactopyranoside) as an inducer. About 50,000 recombinants were amplified on large (150 mm) NZY-plates according to Stratagene's instructions. The titre of the resulting library was 5 x 10⁹ pfu/ml with a total of 150 ml.

Several positive clones were found by screening with anti-TPS/P serum. After three rounds of purification, all clones were positive. They were screened again, now with anti-57K serum.

For further manipulations of DNA, the plasmid part, pBluescript, of the Lambda Zap vector was excised as described in the manual for Predigested Lambda ZapII/EcoR1 Cloning Kit (1989).

### (b) Sequencing of TSS1

A strongly positive clone from the Lambda ZapII library was selected and sequenced manually. The sequence obtained included an open reading frame that encoded a 58 kDa protein, but none of the short chain peptide sequences disclosed in Example 3 was found in the amino acid sequence encoded by this ORF.

Therefore, a second clone was selected, from a group of clones that gave distinct restriction maps compared with the group including the first clone. It also responded less strongly to anti-57K serum, which is why it was not chosen in the first place. It was sequenced using the Exonuclease III/Mung Bean nuclease system for producing series of unidirectional deletions. The deletions were prepared according to Stratagene's manual for the pBluescript Exo/Mung DNA sequencing system. The plasmid was first digested with the restriction enzymes SacI, which leaves a 3' overhang, and BamHI, which leaves a 5' overhang. For filling in possible recessed 3'termini created by Mung Bean nuclease, 2.5 µl of 10X nick-translation buffer, 1 µl of dNTP (a mixture of all four dNTPs, each at 2 mM) and 1 µl (2U) of Klenow fragment were added. The reaction proceeded for 30 min at room temperature and was then stopped with 1 µl of 0,5 M EDTA [Sambrook et al. (1989) loc. cit.]. The deletion time points were run on a 0.8 % low melting agarose gel. The bands were cut out, melted and ligated according to Stratagene's instructions. Portions (5 µl) of each ligation mixture were used to transform XL-1 Blue cells.

The clone proved to encode all the short chain peptide sequences disclosed in Example 3, except the poorly defined pentapeptide, 966b. It is notable that the anti-57K serum alone was an inadequate tool for cloning this gene: the amino acid sequence data disclosed in Example 3 were also essential. Comparison of sequences with the Microgenie Data Bank showed that the gene sequence of the clone was available as an unknown reading frame in the post-translational region of the gene for yeast (S. cerevisiae) vacuolar H⁺-ATPase. The data in the bank contain sequence errors, and have thus been erroneously interpreted as two short unidentified ORFs instead of one long ORF. The complete sequence of the TSS1 gene with 800 bp of promoter and 200 bp of terminator regions is disclosed as SEQ ID NO:1 and the amino acid sequence deduced from its ORF (starting at nucleotide 796) as SEQ ID NO:2. SEQ ID NO:1 now incorporates the following minor corrections to the promoter region, made since February 14th 1992: the original nucleotides 60 and 61 (CA) become AC, original nucleotides 646 to 653 (CGCGTGGT) become GCCGGG and the original nucleotide 711 (C) is deleted. Fig 3A shows the promoter and terminator regions, and Fig 3b shows the deduced amino acid sequence.

### Example 5 Cloning and sequencing of TSL1 and TSL2

### (a) Preparation and screening of genomic DNA libraries

The gene TSL1 was first found in the same library as described in Example 4. Screening was done using first anti-TPS/P serum and then anti-93K serum. Later, another library was constructed from a partial EcoRl digest of chromosomal DNA from S. cerevisiae, strain S288C, using the methods described in Example 4. The anti-93K positive clones were classified by restriction mapping into groups, not all of which can represent TSL1.

### (b) Sequencing of TSL1

Clones from one group of anti-93K positive clones from the HaeIII library were partially sequenced manually and then automatically from pBluescript exonuclease deletion series as described in Example 4.

The HaeIII clones did not contain the whole of this long gene, and the N-terminus was not found in any clone. Therefore, the new EcoRl library was constructed and screened, first with anti-93K serum and then with nucleotide probes derived from the sequenced parts of TSL1.

Several anti-93K positive clones, which also hybridized with the nucleotide probes, were obtained. These contained a plasmid with an 8.2 kb insert. From this plasmid a 2 kb fragment was cut with restriction enzymes StuI and ScaI, religated into the pBluescript SmaI site and sequenced using exonuclease deletions. The deletions were started using the enzymes SacI and SpeI. Sequencing was done with the automatic sequencer. The sequence of TSL1 was thus completed.

The complete sequence is contained in the 8.2 kb insert of the EcoRI clones, and has been deposited as plasmid pALK751 on February 18, 1992 with the Deutsche Sammlung von Microorganismen (DSM), Gesellschaft fur Biotechnologische Forschung GmbH, Grisebachstr. 8, 3400 Göttingen, Germany and given the accession number DSM 6928.

The sequence is shown as SEQ ID NO:83. Nucleotides 2282 to 5575 comprise an ORF that encodes the amino acid sequence SEQ ID NO: 82. The promoter and terminator regions and amino acid sequence are also shown in Fig 4. The amino acid sequence includes the amino acid sequences obtained from (fragments of) the long (123 kDa) chain of trehalose synthase disclosed and discussed in Example 3.

### (c) Isolation and sequencing of TSL2

The information disclosed about the 99 kDa polypeptide (especially in Examples 1 & 3) provides obvious procedures for the isolation and characterization of the TSL2 gene by one ordinarily skilled in the art. Because the anti-93K serum recognizes the 99 kDa polypeptide, anti-93K positive clones isolated as described above can include clones representing TSL2. Several positive clones not representing TSL1 were identified by restriction mapping. One of these was deposited on January 28th 1993 as the plasmid pALK756 (see Table 1) with the Deutsche Sammlung von Mikroorganismen und Zellkulturen GmbH, Mascheroder Weg 1 B, D-3300 Braunschweig, Germany (Accession number DSM 7425). This plasmid comprises a 3.5 kb insert in pBluescript. The insert was not cut by the restriction enzymes, NotI SacI, SpeI or XhoI. The sequences of these and similar clones can be examined to identify an ORF that encodes the amino acid sequences of peptides isolated from the 99 kDa polypeptide (viz., SEQ ID NO:S 29 to 38 and 44 to 49). Another well established procedure is to use these amino acid sequences to design nucleotide primers that can be used to amplify parts of the TSL2 gene by the polymerase chain reaction. When a part of the TSL2 gene has been isolated and sequenced by either procedure, the rest of the gene can be easily isolated as described for TSL1.

### Example 6. Characterization of TSS1 and TSL1

The nucleotide sequence of TSS1 encodes a polypeptide of 495 amino acid residues with a calculated molecular mass of 56 kDa. This open reading frame starts with an ATG codon and ends with two TGA codons. The promoter region contains a TATA box at -178 (see Fig 3) and the sequence CCCCGC at -270, which has been implicated in catabolite repression [Nehlin & Ronne, (1990) European Molecular Biology organization Journal 9, 2891-2898]. This may account for the low expression of trehalose synthase in the presence of glucose disclosed in Example 2.

The open reading frame of TSL1 encodes a polypeptide of 1098 amino acids, corresponding to a calculated molecular mass of 123 kDa. This ORF starts with an ATG codon and ends with a TAA codon. Sixty base pairs downstream from the TAA codon is a possible TATATA transcription termination element [Russo et al. (1991) European Molecular Biology Organization Journal 10, 563-571].

The promoter sequence of TSL1 contains two putative TATA boxes at -100 and -117. The promoter was searched for possible heat shock elements and four AAGGGG elements were found (-166, -180, - 232 and -378). Of these, the one furthest upstream, at -378, was part of the sequence GGTAAAAGGGGCGAA, which corresponds well to the UAS₋₃₆₀ heat shock stress control element GGTAAGGGGCCAA [Marchler, G. et al (1992) Yeast 8, S154]. Two copies of the canonical heat shock element GAANNTTC were found, one at -353 and the other at -425; thus, one on either side of the UAS₋₃₆₀ element.

The sequence GCCCCTGCATTTT at -327 could be a MIG1 protein binding site (the consensus sequence is TCCCCRGATTNT). MIG1 appears to act as a repressor of transcription in the presence of glucose [Nehlin, J.O. & Ronne, H. (1990) European Molecular Biology Organization Journal 9, 2891-2898; Nehlin, J.O. et al (1991) ibid 10, 3373-3378]. These features of the TSL1 sequence are shown in Fig 4.

The amino acid sequence encoded by TSL1 contains two polyglutamine tracts, four Qs starting at amino acid 42 and five Qs starting at 164. Such glutamine-rich sequences have been associated with heteromeric protein-protein interaction [Gancedo, J.-M. (1992) European Journal of Biochemistry 206, 297-313].

Fig. 5 discloses that the entire TSS1 gene exhibits 37 % identity at the amino acid level to a 502 amino acid stretch from the middle of the TSL1 product. The genes are obviously closely related.

Most surprisingly, the TSS1 gene is identical to the CIF1 gene that has been recently cloned and sequenced by Gancedo's group [Gonzales et al (1992) Yeast 8 183-192]. This disclosure reveals that special methodology is required to handle mutants containing modified forms of the TSS1 gene, because cif1 mutants have severe defects in sugar metabolism, as discussed in the Detailed Description. It also explains, of course, why no recognisable short chain is present in the Klg 102 mutants, which carry the cif1 mutation (see Example 7). Previously, it has been (tacitly) assumed that failure of cif1 and fdp1 mutants to express TPS activity is the consequence of a lengthy cascade of regulatory effects. The findings disclosed here and in Example 7 show that absence of the short chain of trehalose synthase is the primary defect, from which, in an as yet completely obscure way, the other regulatory defects of these mutants result.

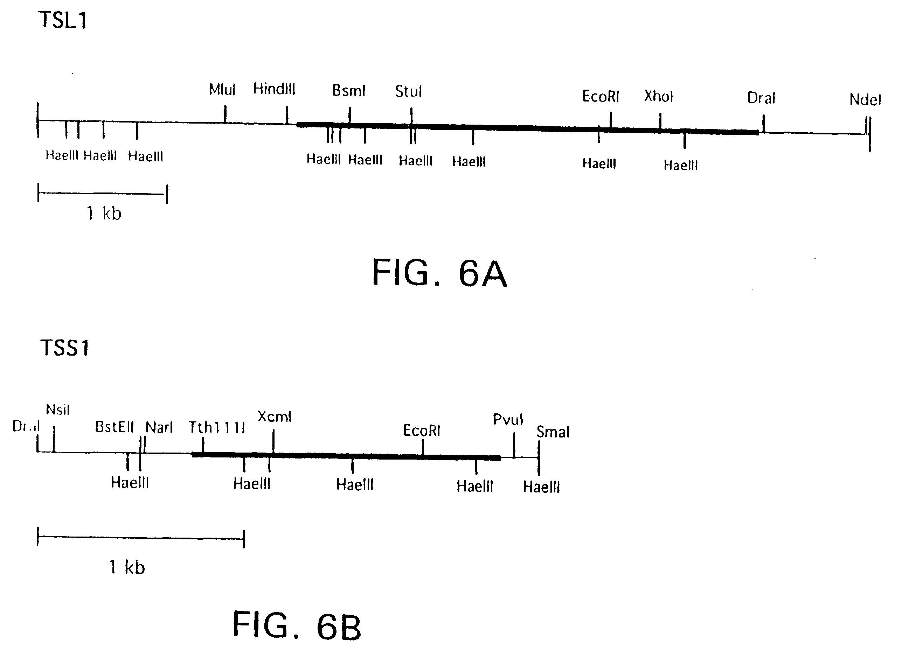

S.cerevisiae chromosomes were separated by pulsed field electrophoresis, with pulse times of 60 sec for 15 h and 90 sec for 9 h at 200 volts, as recommended by the instruction manual for the CHEF-DR II BioRad Laboratories, Richmond, California]. Genes were located using digoxigenin-labelled non-radioactive probes, following the instructions in the manual by Boehringer Mannheim. The following probes were used: a 2.1 kb DraI restriction fragment from TSL1 and a 1.9 kb NarI-SmaI restriction fragment of TSS1 (the SmaI site is in the linker between the insert and the vector; important restriction sites in TSS1 and TSL1 are shown in Fig 6). TSS1 was located exclusively on Chromosome 2, which is where both FDP1 [Van de Poll and Schambert (1977) loc. cit.] and CIF1 [Gonzales et al. (1992) loc. cit.] have been located. This disclosure further strenqthens the evidence for the identity of TSS1 with CIF1 and FDP1. By using the GalH gene as a marker for chromosome 16 TSL1 was located exclusively on the adjacent Chromosome 13. Immediately downstream of TSL1 lies, in opposite orientation, the ARGRII gene, sequenced by Messenguy et. al. [(1986) European Journal of Biochemistry 157, 77-81]. The start of the overlapping sequence is shown in Fig 4.

### Example 7 A functional TSS1 gene is required for expression of both TPS and TPP activities

The S. cerevisiae mutant Klg 102, was obtained from Dan Fraenkel (Harvard Medical School) and has the genotype MATα, ura1, leu1, trp5, cif1-102. It was routinely grown on YP/2% galactose or YP/2% glucose, and long term storage was under liquid nitrogen. As reported by others [Navon, G., et al. (1979) Biochemistry 18, 4487-4499; Ba 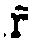 uelos, M. & Fraenkel, D.G. (1982) Molecular and Cellular Biology 2, 921-929], this mutant would not grow on YP/2% fructose, though revertants were frequent.

Six individual colonies from each of two substrains of Klg 102, ALKO 2669 and ALKO 2670, that differed in reversion frequency and colony size, were streaked onto YP/2% fructose and YP/2% glucose at 30 °C. After 45 h, all 12 streaks were growing on glucose, although slower than the control yeast, X2180, but none showed any growth on fructose. After 4 days, five of the ALKO 2669 streaks showed several large, but isolated colonies on fructose and one ALKO 2670 streak showed several small colonies on fructose. From the glucose plates, three streaks from each substrain were chosen for the smallest number of revertants on the corresponding fructose plate, and used to inoculate 100 ml portions of YPD in 250 ml shake flasks, and grown at 200 r.p.m. and 30 °C. Three parallel flasks were inoculated with X2180. A600 and residual glucose in the media were monitored and samples were plated out quantitatively onto YP/2% glucose and YP/2% fructose. The ALKO 2669 cultures grew faster than the ALKO 2670 cultures, and both grew much slower then X2180 (not shown).

At appropriate times the cells were harvested, broken and analyzed as described in the General Materials and Methods. Results are shown in Table 5.

**Table 5.**

| Growth of Klg 102 and X2180 strains on YPD | | | | | | | | |
|---|---|---|---|---|---|---|---|---|
| The cultures were performed as described in the text. Residual glucose and cell mass are given as, respectively, g/100 ml and mg/ml of growth medium. Phosphoglucoisomerase (PGI) was determined as described in Example 11. PGI, TPS and TPP are given as U/g of wet cells (TPS was determined in the presence of 5 mM F6P). Trehalose is given as mg/g of wet cells. Viability Fru/Glu shows the number of cells able to grow on fructose as a percentage of the number of cells able to grow on glucose at the time of harvesting. Cells from the cultures 2670/1 and 2670/2 were combined for breakage and subsequent analysis. ND, not determined. | | | | | | | | |

| Strain | Age (h) | Residual Glucose (g %) | Cell Mass (mg/ml) | PGI (U/G) | TPS (U/G) | TPP (U/G) | Trehalose (mg/g) | Viability Fru/Glu (%) |
|---|---|---|---|---|---|---|---|---|
| Klg 102 cultures | | | | | | | | |
| 2669/1 | 24 | ND | 4.3 | 88 | ≤0.02 | ND | ND | 2.4 |
| | | | | | | | | |
| 2669/2 | 48 | ≤0.02 | 11.6 | 81 | ≤0.03 | 0.034 | ND | ≤1.7 |
| | | | | | | | | |
| 2669/3 | 114 | none | 10.3 | ND | ND | ≤0.02 | ≤0.22 | ≤1.8 |
| | | | | | | | | |
| 2670/1 2670/2 | 110 110 | none | 9.7 | 89 | ≤0.03 | 0.081 | ND | 1.4 4.0 |
| | | | | | | | | |
| 2670/3 | 114 | none | 11.2 | ND | ND | ≤0.02 | ≤0.19 | ≤0.3 |

| X2180 cultures | | | | | | | | |
|---|---|---|---|---|---|---|---|---|
| 1 | 24 | ND | 19.1 | 93 | 6.3 | 1.7 | ND | ND |
| | | | | | | | | |
| 2 | 110 | none | 31.7 | 126 | 6.3 | 2.3 | ND | ND |
| | | | | | | | | |
| 3 | 114 | none | 34.4 | ND | ND | 2.9 | 29.3 | ND |

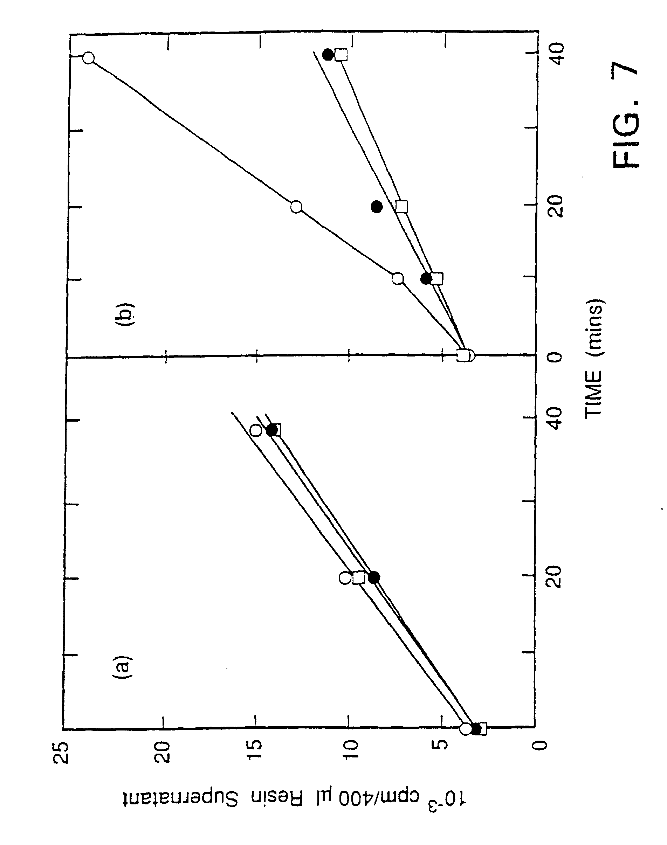

These results show that TPS activity was below the detection level in the Klg 102 samples and less than 0.5 % of the value in X2180, which is typical of wild type S. cerevisiae. This agrees with previously reported results [Paschoalin, V.M.F., et al. (1989) Current Genetics 16, 81-87]. Surprisingly, however, TPP activities were also very low, between ≤ 1 % and 5 % of the X2180 values. Even this residual ability to hydrolyse trehalose-6-phosphate is likely to be due to non-speciflc phosphatases. Paschoalin et al. [(1989) loc. cit.] claim that Klg 102 specifically lacks UDPG-linked TPS activity, but that, like the wild-type yeast S288C (which is the haploid form of X2180), it contains an ADPG-linked activity. If this were true, and accepting the conventional view that trehalose synthesis in yeast proceeds via free trehalose-6-phosphate, Klg 102 should contain significant TPP activity. our results disclose that this is not the case. Furthermore, when we tested whether wild type yeast (X2180) was able to synthesise [¹⁴C]-trehalose from [¹⁴C]-G6P in the presence of UDPG or ADPG, we found significant activity only in the presence of UDPG. The assay systems used by Paschoalin et al. [(1989) loc. cit.] have been criticised by Vandercammen et al. [(1989) loc. cit.], so we tested the overall reaction directly. Yeast extracts were incubated in 40 mM HEPES pH 6.8 containing 1 mg BSA/ml, 10 mM MgCl₂ and 10 mM [U ¹⁴C]-G6P (736 c.p.m./nmol) in the presence or absence of 5 mM UDPG or 2.5 mM ADPG and presence or absence of 5 mM K phosphate. Reactions were stopped by boiling for 2 min and addition of AG1-X8 (formate) anion exchange resin, as in the TPP assay system described by Londesborough & Vuorio [(1991) loc. cit.]. Results are shown in Fig 7. Without UDPG or ADPG, radioactivity appeared in the resin supernatants, presumably due to phosphatases active on G6P. UDPG caused a clear increase in this rate in the absence of phosphate and a marked increase in the presence of 5 mM phosphate, which stimulates the TPP activity and inhibits the TPS activity of trehalose synthase. With UDPG and 5 mM phosphate, the increase in rate corresponded, after a lag phase, to 0.94 µmol/min/g of fresh yeast, which is about 50 % of the TPP activity of this yeast at 20 mM phosphate. ADPG, however, did not cause any significant increase in the rate of appearance of radioactivity in the resin supernatant, indicating that no ADPG-linked TPS activity was present.

Western blots of the homogenates of Klg 102 and X2180 yeast are shown in Fig. 8. The origin of the bands marked D is not clear: they may be degraded short chain. X2180 shows a strong 57 kDa band, due to the short chain of trehalose synthase and several weak bands at 100 to 130 kDa due to intact and truncated versions of the long chain. In contrast, although the Klg 102 samples showed stronger long chain bands, because more yeast sample was applied to the gel, they showed no trace of a short chain band. Thus, Klg 102 does not contain a recognisable form of the product of the TSS1 gene (it might contain a truncated version lacking the epitopes recognised by our polyclonal antibodies), but contains normal amounts of the TSL1 product. Furthermore, the TSL1 product appears to increase as Klg 102 traverses the diauxic lag (compare e.g. lanes 3 and 2 of Fig. 8), suggestinq that expression of the long chain of trehalose synthase in this yeast increases when all glucose is consumed. In wild type yeast, increases in both short and long chains occur concomitant with the increases in TPS and TPP activities when glucose is consumed (Example 2).

These results disclose that the failure of Klg 102 to express immunologically recognisable short chain of trehalose synthase is correlated with the absence of both TPS and TPP activities. This unexpected behaviour, in contradiction of the views of Paschoalin et al. [(1989) loc. cit.], indicates that a functional short chain is required to assemble a trehalose synthase with either partial activity.

Similar experiments were done with S. cerevisiae, strain MV6807 (obtained from Johan Thevelein, Laboratorium voor Moleculaire Celbiologie, Instituut voor Plantkunde, Heverlee, Belgium), which carries the fdp1 mutation, which is allelic to CIF1 and TSS1. This strain grew poorly on glucose (fructose was not tested) and so was grown on galactose. Stationary phase cells contained 6 ± 6 % of normal TPS but about 20 % of normal TPP. Western analyses showed the presence of a band at 57 kDa recognised by anti-57K serum as well as normal long chain bands, so the mutation in MV6807 must be an aminoacid substitution. Apparently, this substitution causes a greater decrease in TPS activity than TPP activity.

### Example 8. Biochemical evidence that a long chain of trehalose synthase is required for TPP activity

Truncated trehalose synthase containing the short (57 kDa) chain and the 86 and 93 kDa long chain fragments was prepared according to the method of Londesborough & Vuorio (1991) loc. cit.] for proteolytically activated TPS/P complex. TPS and TPP activities were assayed as described by Londesborough & Vuorio [(1991) loc. cit.]. [N-ethyl-1-¹⁴C]- maleimide (ethyl-labelled NEM; 40 mCi/mmol) was NEC-454 from New England Nuclear. N-ethyl-[2,3-¹⁴C]-maleimide (ring-labelled NEM; 6 mCi/mmol) was CFA 293 from Amersham International. Both were obtained as solutions in n-pentane and the manufacturer's stated specific activities were assumed to be correct. Unlabelled N-ethyl-maleimide (NEM) was E-3876 from Sigma. It was dissolved in 25 mM HEPES pH 7.0 immediately before use and standardized by absorption measurements at 305 nm, assuming an E^{mM} of 0.62.

Treatment of truncated trehalose synthase with 1.9 mM NEM at 24 °C in the presence of about 0.17 mM dithiothreitol (which presumably rapidly consumes about 0.34 mM NEM) caused a rapid and essentially complete (≥ 98 %) loss of TPP activity, but little (≤ 24 %) loss of TPS activity (Fig. 9). This suggested that NEM modified one or more amino acid (presumably cysteine) side chains that are required intact for TPP but not for TPS.

To permit quantitative experiments with low concentrations of labelled NEM, the dithiothreitol in the enzyme preparation was removed by gel-filtration through Pharmacia NAP5 columns equilibrated with 1 mg BSA/ml of 25 mM HEPES pH 7.0 containing 2 mM MgCl₂, 1 mM EDTA and 0.2 M NaCl. Recoveries of TPS and TPP activities through this gel-filtration were above 85 %.

In one experiment, 2.0 µl of 2.4 mM ethyl-labelled NEM was mixed with 150 µl of gel-filtered enzyme and incubated at 23 °C. Samples (10 µl) taken at various times up to 190 min were mixed with 60 µl of Laemmli sample buffer (the mercapto-ethano in this buffer should destroy residual NEM), boiled for 5 min and subjected to SDS-PAGE. At closely similar times (and also at 23 h) other samples (10 µl) were mixed with 100 µl (for TPS) or 700 µl (for TPP) of 5 mg BSA/ml 25 mM HEPES pH 7.0 containing 2 mM MgCl₂, 1 mM EDTA, 0.2 M NaCl and 1 mM dithiothreitol (the dithiothreitol should destroy residual NEM) and assayed for TPS and TPP. The enzyme dilution used for the TPP assay was sufficient that radioactivity from the NEM (about 1/3 of which remains in the resin supernatant) did not interfere with the TPP determinations.

After electrophoresis, the upper (cathode) buffer, containing most of the added radioactivity, was completely removed before disassembling the apparatus. The gel was then fixed, stained and destained as described by Laemmli [(1970) Nature, London 227, 680-685] and dried. An autoradiogram of this gel (Fig. 10) showed that the 93 kDa band (and also BSA) became labelled during the experiment, while the 86 and 57 kDa bands were much more weakly labelled. The Coomassie blue stained bands and adjacent, empty areas (as blanks) were cut out of the dried gel (in later experiments, they were cut from undried gels), broken up and extracted overnight with 1 ml of 5 % SDS in pre-blanked scintillation vials. Then 10 ml of a toluene/Triton X100-based scintillant was added, and the tubes were repeatedly counted using a wide energy window to minimise quench effects. After 10 h constant counting levels were reached. Excess radioactivity was calculated by subtracting a blank value obtained from empty regions of the gel. Results are shown in Fig. 11. In control experiments, in which enzyme was omitted, it was shown that the excess radioactivity found in the 93 and 86 kDa bands did not originate from potential labelling of impurities in the BSA.

Fig. 11 shows that label from NEM enters mainly the 93 kDa fragment of the long chain, with relatively small amounts entering the 86 kDa fragment and the 57 kDa short chain. Also, the amount of label entering the long chain fragments (93 + 86 kDa) is roughly proportional to the loss of TPP activity, but lags increasingly behind this loss: at 10.5 min 30 % of the initial TPP was lost and 0.20 moles of NEM had entered the long chain fragments per mole (150 Kg) of enzyme, whereas at 190 min, 56 % of TPP was lost and 0.32 moles of NEM had entered the long chain fragments. Possibly, since trehalose synthase may be an octamer (its native molecular mass is about 800 kDa), reaction of one long chain with NEM can eventually lead to loss of activity associated with the other long chains in the octamer. Fig. 12 collates data from several experiments, using both ring- and ethyl-labelled NEM. Parallel experiments with identical concentrations of ring- and ethyl-labelled NEM suggested that about 25 % of the radioactivity from ethyl-labelled NEM originally fixed in the protein was lost during SDS-PAGE processing (some loss is expected in acidic condition), and the results with ethyl-labelled NEM have been corrected accordingly. Within the limits of accuracy (a specific activity of 30 TPS units/mg was used to calculate the mass of protein and a dimer molecular mass of 150 kDa was assumed for the truncated enzyme) complete loss of TPP reflected incorporation of rather less than 1 mole of NEM into, specifically, the long chain fragments.

Another reagent with high specificity for cysteine, dithiodinitro-benzoate (DTNB), also caused a specific loss of TPP activity: after 10 min treatment with 0,9 mM DTNB over 95 % of the TPP was lost and less than 28 % of the TPS.

These findings disclose that TPP activity requires a long chain with a proper structure, because modification of a single amino acid (presumable cysteine) residue in the 93 kDa fragment eliminates TPP but not TPS activity. sequencing data given in Example 3 disclosed that the 93 kDa band contained material from both the 99 kDa and 123 kDa long chains. Thus, the present results disclose that either the 99 kDa or the 123 kDa or both long chains are involved in TPP activity.

### Example 9. An isolated 99 kDa polypeptide from trehalose synthase contains TPP activity

Because the long and short chains of trehalose synthase were difficult to separate by usual chromatographic procedures, fractionations were attempted in the presence of a non-ionic detergent. During fractionation with a NaCl gradient on DEAE-cellulose (Whatman DE52) in 1 % Triton X100 at pH 8.0, the enzyme was recovered in about 90 % yield at 140 mM NaCl. Some minor polypeptides (e.g. the weak 68 kDa polypeptides visible in Fig 1) were removed, but the main 57, 99 and 123 kDa polypeptides were not resolved. However, the ratio of the 99 and 123 kDa bands changed from about 1.5 to 0.3 across the enzyme peak, while concomitantly the TPP/TPS ratio decreased steadily from 0.54 to 0.42 (data not shown). This suggested that the procedure was partially resolving trehalose synthase molecules enriched in the 99 kDa polypeptide from those enriched in the 123 kDa polypeptide and that the former had a relatively higher TPP activity. By extrapolation it can be calculated that the TPP/TPS ratio of (hypothetical) enzyme containing only 57 and 99 kDa chains would be 0.65 ± 0.10, whereas that of enzyme with only 57 and 123 kDa chains would be 0.32 ± 0.10.

Because the long chain appears to contain an avid phosphate binding site (see Examples 10 and 12), chromatography on phosphocellulose was attempted. Native trehalose synthase (4.2 TPS units) was transferred above a PM10 membrane in an Amicon cell to 25 mM HEPES pH 7.0 containing 2 mM MgCl₂, 1 mM EDTA, 1 mM dithiothreitol and 0.3 % Triton X100 (HMED/0.3 %T) and applied to a 0.7 x 4.2 cm column of phosphocellulose (Whatman P11-cellulose) equilibrated with the same buffer. The column was washed with 4 ml of HMED/0.3 %T and developed with a linear gradient from zero to 0.6 M NaCl in 60 ml of HMED/0.3 %T at 5 ml/h. By 0.35 M NaCl only traces of TPS had been eluted (≤ 3 % in the first 9 ml and ≤ 9 % spread between 0.15 and 0.35 M NaCl). The gradient was interrupted and the column was washed sequentially with (a) 8 ml of 10 mM fructose-6-phosphate in HMED/0.3 %T/0.35 M NaCl, (b) 6 ml of HMED/0.3 %T/0.6 M NaCl and (c) 0.2 M K phosphate pH 7.0/2 mM MgCl₂/1 mM EDTA/1 mM dithiothreitol. No TPS or TPP activity was recovered except in a single 1.5 ml fraction in which the 0.6 M NaCl began to elute. This contained 12 % of the applied TPP, but ≤0.1 % of the applied TPS.

Fractions were examined by SDS-PAGE (Fig. 13), which showed:
(1) almost pure short chain eluted at and just before the start of the NaCl gradient in fractions devoid of enzyme activity;
(2) traces of short and long chain eluted diffusely at about 0.2 to 0.35 M NaCl in fractions containing altogether ≤ 7 % of the applied TPS activity; (3) at least 50 % and possibly all of the applied 99 kDa polypeptide eluted at 0.6 M NaCl in the fraction containing 12 % of the applied TPP activity; and (4) most of the 123 kDa polypeptide remained bound to the column.

Intact trehalose synthase has also been fractionated on phosphocellulose in the absence of Triton, and with elution by a simple linear gradient from 0 to 0.6 M NaCl. Pure or nearly pure 99 kDa polypeptide eluted at about 0.45 M NaCl and contained specific TPP activity (¹⁴C-G6P was not hydrolyzed). This activity differed from the TPP activity of intact trehalose synthase in that the ratio of activities at 25 mM phosphate and 50 mM Hepes was between 1.5 and 3 in different experiments (cf, this ratio is 5 to 6 for intact trehalose synthase). Furthermore, during storage of the isolated 99 kDa polypeptide at 0 °C, the TPP activity at 25 mM phosphate decreased and that at 50 mM Hepes increased, until the ratio was about 0.7 after 7 weeks.

These findings disclose that the 99 kDa polypeptide isolated from intact trehalose synthase is a specific trehalose-6-phosphatase, but that its catalytic properties are unstable and differ from the TPP activity of intact trehalose synthase. Together with the disclosure in Example 7 that yeast requires a properly functional TSS1 gene to exhibit TPP activity, the results suggest that proper folding of the 99 kDa polypeptide requires the presence of the 57 kDa chain.

These findings also disclose that when the short chain is separated from the long chain by chromatography in a buffer containing 0.3 % Triton, in which intact trehalose synthase is stable, it rapidly looses any TPP or TPS activity it possessed when correctly folded in the trehalose synthase.

The findings also indicate that the full-length long chain has extraordinarily high affinity for phosphocellulose, which is consistent with the location of a high affinity phosphate binding site in a terminal portion of this chain as suggested by Examples 10 and 12.

### Example 10 Truncation of the 123 kDa long chain of trehalose synthase by trypsin in vitro dramatically increases TPS activity

Removal of the N-terminal 325 or so amino acids from the 123 kDa long chain of intact trehalose synthase by treatment with trypsin in vitro produces an enzyme with catalytic properties like those of the truncated enzyme purified by Londesborough & Vuorio [(1991) loc. cit.]. In one experiment intact trehalose synthase (0.28 TPS units, ≈ 9.4 µg) was incubated with or without 0.5 µg of trypsin at 30 °C in 250 µl of 13 mM HEPES pH 7.0 containing 1 mM MgCl₂, 0.5 mM EDTA, 0.5 mM dithiothreitol, 0.2 M NaCl and 0.5 mM benzamidine. Its TPS activity was determined at intervals using standard assay mixtures (containing 5 mM F6P) containing no or 4 mM K phosphate pH 6.8, and samples were prepared for SDS-PAGE analysis immediately before and 48 min after addition of the trypsin.

During the first 48 min the TPS activity measured in the absence of phosphate decreased faster in the presence of trypsin than in its absence. However, in the first 10 min, trypsin caused a 4-fold increase in the activity measured at 4 mM phosphate, and by 48 min the activities with and without phosphate were essentially equal (Fig. 14). By 48 min, the 123 kDa full length long chain had disappeared and been replaced by a doublet of polypeptides at 85 kDa (Fig. 15). In contrast, the short chain (57 kDa) was unchanged and the 99 kDa band was only slightly decreased in strength. The changes in TPS activity were accompanied by loss of about 50 % of the TPP activity.

Which part of the 123 kDa chain was removed by trypsin was determined as follows. Intact trehalose synthase (180 µg) was transferred to 0.5 ml of 25 mM HEPES pH 7.0 containing 2 mM MgCl₂, 1 mM EDTA, 1 mM dithiothreitol and 0.2 M NaCl using a Centricon 30 tube, and then treated with 11 µg trypsin at 25°C. The standard TPS activity did not decrease during the trypsin treatment, whereas TPS activity measured in the absence of F6P and presence of 10 mM phosphate increased from 26 % to 73 % of the standard activity during the first 30 min of treatment. After 68 min treatment, when SDS-PAGE analysis showed the complete disappearance of the 123 and 99 kDa bands and appearance of a doublet with apparent molecular mass about 85 kDa (the components differing by about 1.5 kDa), the mixture was centrifuged through a Centricon 30 tube to separate the tryptic peptides from the core enzyme. The retentate was then boiled in 0.5 % SDS and again centrifuged through the Centricon 30 tube. The combined filtrates were diluted to 0.1 % SDS and incubated for 18 h at 25 °C with 4 % by weight of endoproteinase Glu-C (Boehringer). The peptides were then separated by HPLC using a DEAE pre-column and sequenced as described in Example 3.

Twenty sequences were obtained (Seq ID NOs 58 to 77 in Table 6). Fifteen of these were found in the N-terminal 325 amino acids coded by TSL1. One (peptide 1407, recovered at less than half the yield of the others) was amino acids 1089 - 1093, i.e., 5 amino acids from the C-terminus of the protein coded by TSL1. This peptide is presumably derived by endoproteinase Glu-C cleavage of the tryptic peptide starting after Lys 1079. Both the 86 and 93 kDa long chain fragments in the truncated trehalose synthase purified by Londesborough & Vuorio [(1991) loc. cit.] are disclosed in Example 3 to contain a peptide (1483b & 1484b) derived from Ala1064 to Lys1079, confirming that the truncated polypeptides extend at least this close to the C-terminus of the full length 123 kDa chain. The N-terminal peptide furthest from the N-terminus was peptide 1443, obtained by cleavage after Arg 335. Thus, the truncated long chain extends from Ser 336 to Lys 1079 or Asp 1098, and is predicted to have a molecular mass of 87.3 or 86.2 kDa. The SDS-PAGE analysis of trypsin-treated enzyme suggests both of these truncated chains are formed, and because the TPS activity in the presence of F6P changes little during the trypsin treatment, the two truncated chains probably have similar activities.

Of the remaining four peptides in Table 5, two (1419b and 1437b) are still unidentified, but may originate from the 99 kDa polypeptide, whereas two (1442 and 1451) clearly originate from that polypeptide. Thus, peptide 1442 is identical to peptide 1307a of Table 4, and the first 5 amino acids of peptide 1451 are identical to peptide 1297a (Table 4). These results disclose that removal of the N-terminal 325 amino acids of the long chain, with or without removal of the C-terminal 19 amino acids, results in a trehalose synthase that is relatively insensitive to inhibition by phosphate, and does not require F6P for full activity. Analysis of the secondary structure of the long chain according to Garnier et al [(1978) Journal of Molecular Biology, 120, 97-120] suggests that whereas the C-terminal 700 amino acids are likely to be in alpha-helices or beta-sheets, the N-terminal 360 amino acid portion of the protein is relatively devoid of such structures. Taken together, these data suggest that the N-terminal 330 or so amino acids comprise a distinct domain, that confers regulatory properties upon the TPS activity of trehalose synthase, including sensitivity to inhibition by phosphate and a requirement for F6P to express full catalytic activity. Thus, the TSL1 gene product must also be involved in TPS activity.

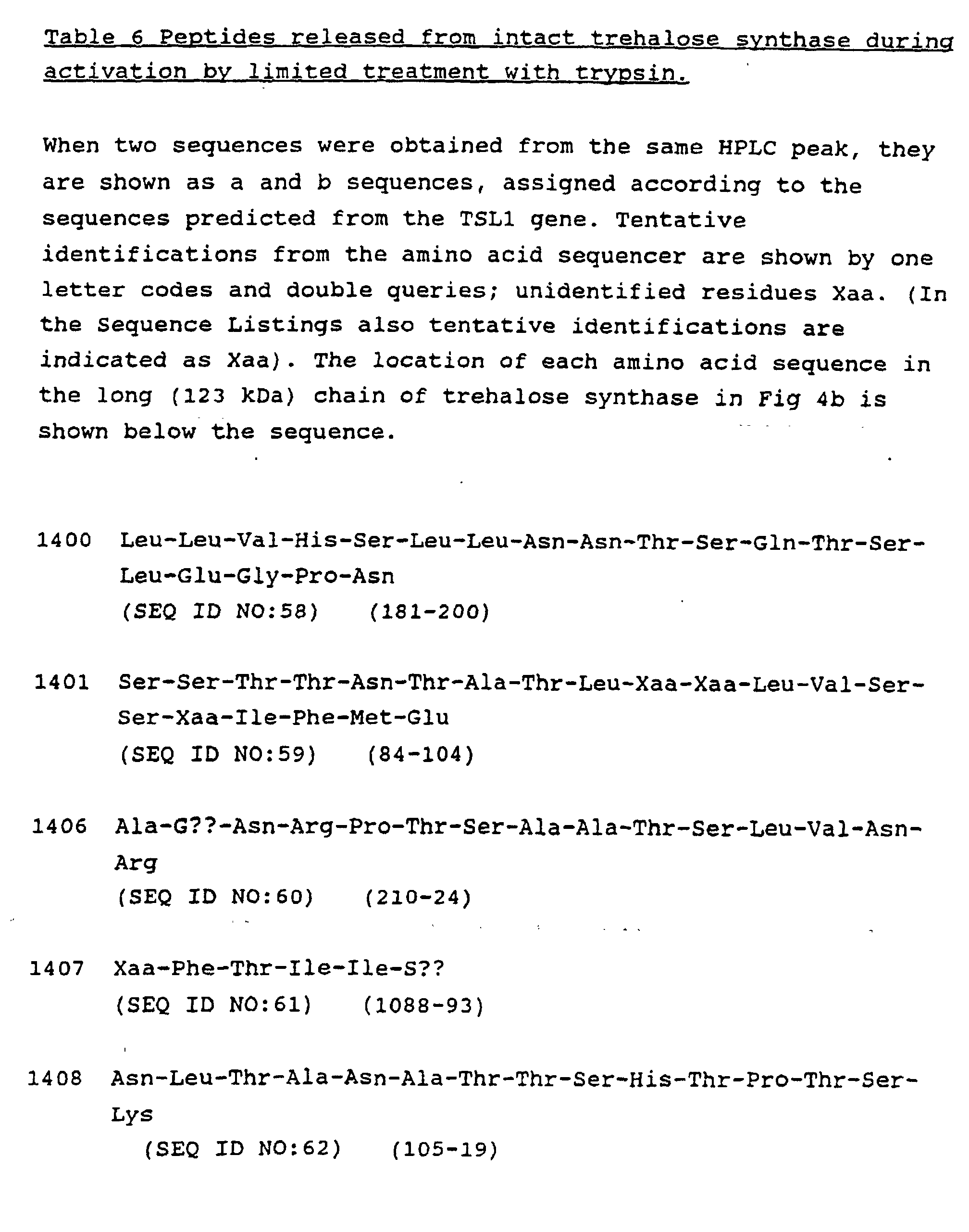

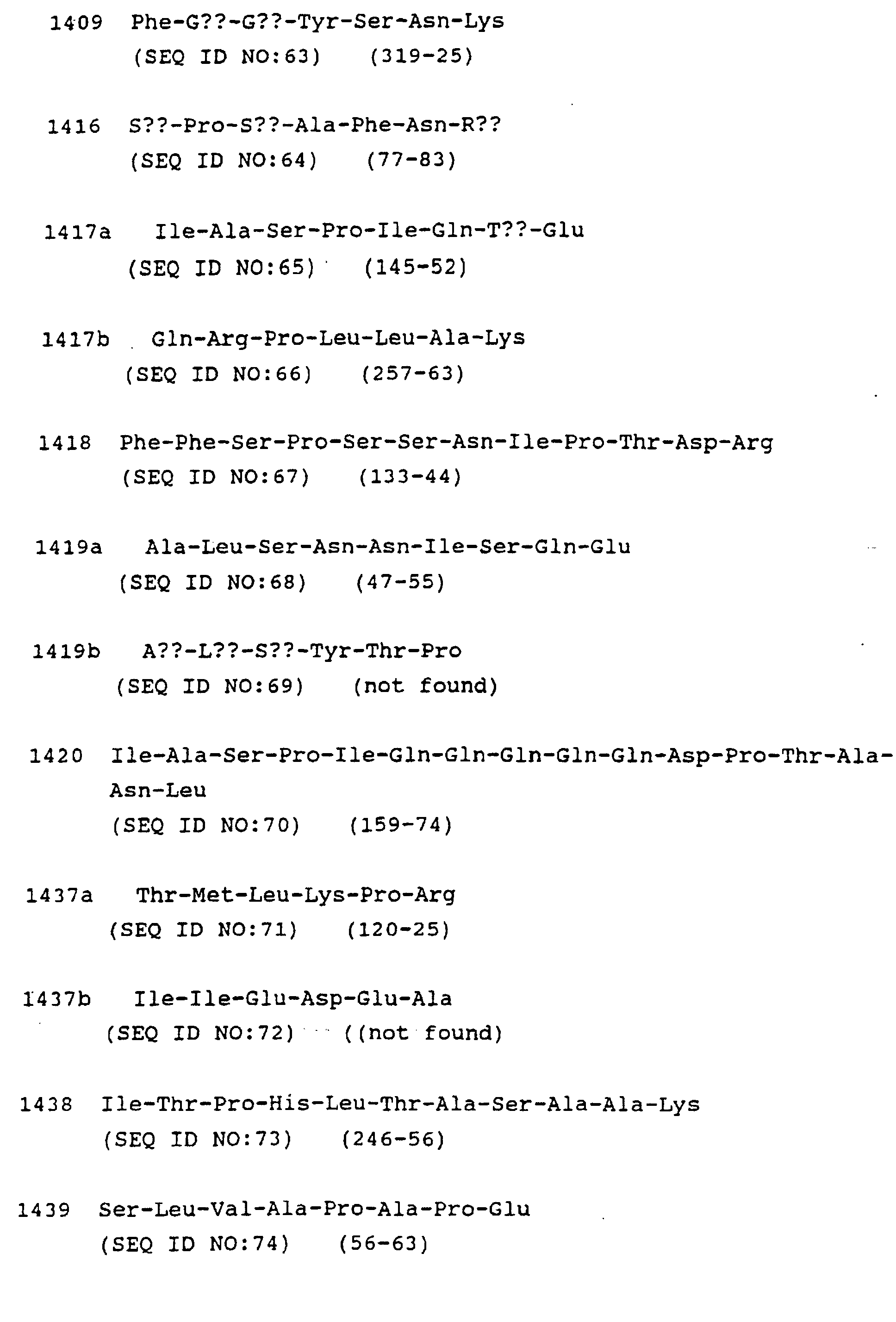

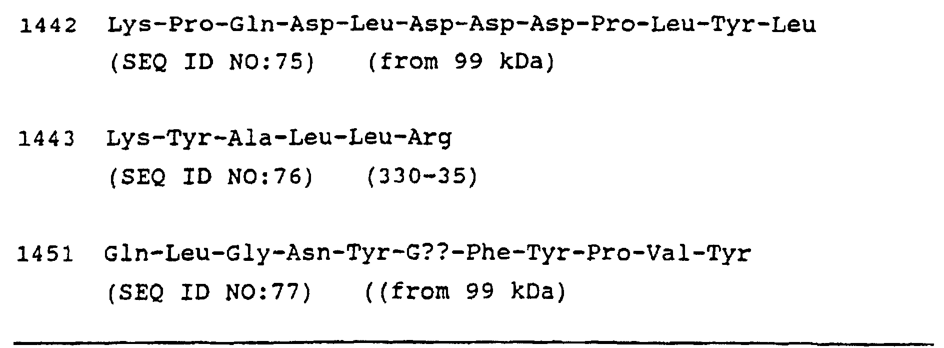

### Example 11 Identification of the TPS activator as phosphoglucoisomerase

TPS activator was transferred to 0.1 M Tris/HCl pH 9.0 above a PM10 membrane in an Amicon cell. A 300 µl sample (34 µg) was digested for 20 h at 37 °C by 0.8 µg of lysylendo-peptidase C (Wako). Peptides were separated by HPLC and sequenced as described in Example 3. All five sequences obtained and disclosed in Table 7 are identical to sequences found in yeast phosphoglucoisomerase (PGI).

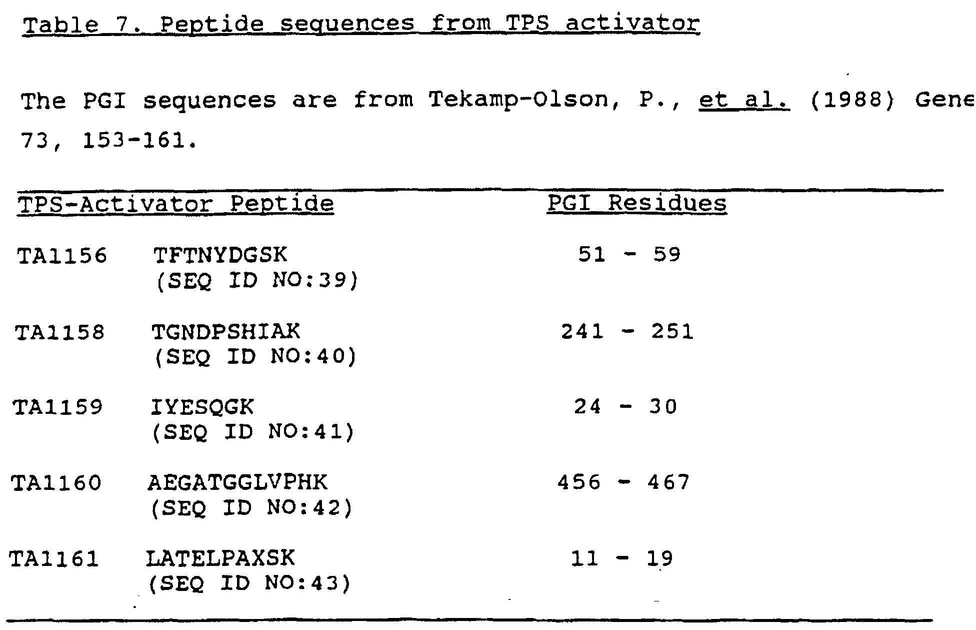

The PGI activity of a sample of TPS activator that had been stored for several months at 0 °C was measured in 50 mM HEPES/KOH pH 7.0, 5 mM MgCl₂, 5 mM F6P and 0.4 mg/ml NADP. A specific activity of 190 U/mg was found.

These findings disclose that TPS activator from S. cerevisiae is identical to PGI. Example 12 discloses that F6P is a powerful activator of the TPS activity of intact, but not of truncated, trehalose synthase. Because the assay mixtures for TPS contain G6P, it is clear that TPS activator can activate TPS by producing F6P from the substrate G6P. This is a complete explanation for the activation. Thus, at initial concentrations of 6.7 mM G6P and 1.9 mM F6P (i.e. G6P/F6P = 3.5, the experimental equilibrium ratio) the rate was independent of TPS activator and equal to that at 9 mM G6P with TPS activator. Previous investigations [Londesborough & Vuorio (1991) loc. cit.] had to use crude preparations of intact trehalose synthase because pure intact trehalose synthase was not available. Although the effectiveness of TPS activator preparations was reported to vary between different enzyme preparations, under certain circumstances data were obtained that suggested TPS activator might interact stoichiometrically with native trehalose synthase [Londesborough & Vuorio (1991) loc. cit.]. The present findings show that this suggestion was completely incorrect. The findings also imply that kinetic data in the literature are confused, because some preparations of so-called "trehalose-6-phosphate synthase" will have contained PGI whereas some may not. With the former preparations, the activator F6P will have been generated from the substrate G6P, but the amount so generated will have depended upon the details of the experimental procedure used.

### Example 12. The different kinetic behaviours of intact and truncated trehalose synthase

Truncated trehalose synthase was prepared as described by Londesborough & Vuorio [(1991) loc. cit.] and contained the 57 kDa short chain and 86 and 93 kDa fragments of the long chain. Intact trehalose synthase was prepared as in Example 1. Kinetic assays were done at 30 °C as described in General Methods and Materials.

### (a) The TPS Partial Activity

**Table 8.**

| Inhibition of the TPS activities of intact and truncated enzyme bv phosphate at 5 mM F6P | | |
|---|---|---|
| The effect of adding K phosphate pH 6.8 to standard assay mixtures (10 mM G6P, 5 mM UDPG and 5 mM F6P) is shown. For each enzyme, the activity without phosphate is set at 100 %. | | |

| Added Phosphate | Intact Enzyme | Truncated Enzyme |
|---|---|---|
| None | 100 % | 100 % |
| 1.3 mM | 69 % | 94 % |
| 4.0 mM | 14 % | 83 % |

The TPS activity of intact enzyme was much more sensitive to inhibition by phosphate than was that of the truncated enzyme (Table 8). The results in Table 8 underestimate the difference between the phosphate responses of intact and truncated enzyme, because F6P partially reverses the phosphate inhibition of intact enzyme (see below) but has virtually no effect on truncated enzyme. Table 9 shows the effect of shifting from the salt conditions of the standard assay (40 mM HEPES/KOH pH 6.8, 10 mM MgCl₂) to conditions closer to those of yeast cytosol. In the absence of F6P, the shift caused 67 % inhibition of intact enzyme (from 43 % to 14 % of the standard activity) but only 10 % inhibition of truncated enzyme (from 96 % to 86 %).

**Table 9.**

| Effect on the TPS activity of intact and truncated enzvme of shifting to more physiological salt conditions | | | | |
|---|---|---|---|---|
| For measurements at "physiological conditions", 1.3 mM K phosphate and 0.1 M KCl were added to the standard assay mixtures and the MgCl₂ was decreased from 10 to 2.5 mM. | | | | |

| | Standard Cond. | | Physiological Cond. | |
|---|---|---|---|---|
| | (5 mM F6P) | No F6P | 5 mM F6P | No F6P |
| Intact | 100 % | 43 % | 72 % | 14 % |
| Truncated | 100 % | 96 % | 90 % | 86 % |

These results disclose the insensitivity of the TPS activity of truncated trehalose synthase to physiological phosphate concentrations and the presence or absence of F6P at a concentration well above the normal value in yeast cytosol (between 0.1 and 1 mM; Lagunas, R. & Gancedo, C. (1983) European Journal of Biochemistry 137, 479-483).

Fig. 16 illustrates the F6P-dependence of the TPS activity of intact enzyme at different phosphate concentrations. Double-reciprocal plots of these data (not shown) indicate that at 1.3 mM phosphate, and perhaps at 4 mM phosphate, sufficiently high concentrations of F6P completely overcome the inhibition by phosphate. With no added phosphate, F6P caused a maximum activation of 2.5-fold, with a K_{½} of 60 µM. At 1.3 mM phosphate, the maximum activation was at least 20-fold, and the K_{½} was 1.4 mM F6P. The slopes of these double-reciprocal plots varied linearly with the square of the phosphate concentration, suggesting that two phosphate binding sites are involved. At 4 mM phosphate, which is still within the probable range of phosphate concentrations in yeast cytosol [Lagunas & Gancedo (1983) loc. cit], inhibition was so severe that even 10 mM F6P permitted only 40 % of the activity observed under standard conditions. Thus, expression of a truncated trehalose synthase in yeast would be expected to cause a large increase in the intracellular specific activity of the enzyme.

Fructose-1-phosphate, fructose-1,6-bisphosphate, fructose-2,6-bisphosphate and glucose-1-phosphate were tested at sub-optimal F6P concentrations (1 mM F6P at 1.3 mM phosphate). None caused activation at 5 or 2.5 mM concentrations; instead inhibitions of about 25 % occurred, probably due to competition with G6P and F6P.

### (b) The TPP Partial Activity.

At phosphate concentrations equal to or less than 1 mM, the progress curves of TPP reactions catalysed by truncated trehalose synthase accelerated markedly over at least the first 10 min of reaction. This did not happen with intact enzyme. For the initial rates of reaction, intact enzyme was activated by smaller phosphate concentrations than was truncated enzyme (Fig. 17). For truncated enzyme, double-reciprocal plots of the activation (vΔ = the rate with phosphate, vₚᵢ, minus the rate without phosphate, vₒ) were linear when 1/vΔ was plotted against 1/[phosphate], with a K_{½} of 3 mM phosphate. For intact enzyme these plots were non-linear, and linear plots resulted when 1/[phosphate]² was used (Fig. 18). This, again, suggests that intact enzyme has two strong phosphate binding sites, one of which is lost in the truncated enzyme. For intact enzyme, half maximal activation was obtained at 0.6 mM phosphate.

In the absence of phosphate, F6P did not affect the TPP activity of intact enzyme. At sub-optimal phosphate concentrations, 5 mM F6P caused modest (20 to 30 %) inhibitions of the TPP activity of both intact and truncated enzymes, and at saturating phosphate concentrations, smaller inhibitions (10 to 15 %) were observed (data not shown).

These findings disclose a profound sensitivity of the TPS activity of intact trehalose synthase to physiological phosphate and F6P concentrations that is lost by truncation of the 123 kDa long chain to about 85 kDa. The effects of truncation are less marked on the TPP activities, both enzymes being activated by physiological phosphate concentrations, and neither showing a strong response to F6P. The data suggest that intact enzyme has two strong phosphate binding sites, one of which is located in the region of the 123 kDa long chain removed by truncation. The finding that the 123 kDa long chain could not be recovered from phosphocellulose, disclosed in Example 9 supports this conclusion.

### Example 13 Expression of TPS activity in Escherichia coli cells transformed with TSS1 and TSL1

E.coli, strain HB101 (ALKO 683) was transformed with the plasmids pALK752 and pALK754 consisting of pBluescript containing TSS1 and TSL1, respectively (see Example 14 and Figs 20a and 20c). Transformants ALKO3566 and ALKO3568 containing, respectively, pALK752 and pALK754 were selected and maintained by growth in the presence of 50 µg/ml of ampicillin. Shake flasks containing Luria Broth with no ampicillin (ALKO 683) or 15 µg/ml ampicillin (ALKO3566 and ALKO3568) were inoculated with 1 ml of a suspension (A600 = 1.5) of the appropriate cells and shaken at 250 rpm and 30 °C for 15 h. Cells were harvested (5 min and 3000g), washed twice with water, suspended (1.5 g cells/ 3.7 ml) in HBMED containing 1 mM PMSF and 10 µg/ml pepstatin A, and broken by two passes through a French press (Aminco) at 15 000 psi. Samples of the homogenates were centrifuged 20 min at 28 000g. Homogenates and supernatants were assayed for TPS and TPP at once and the protein contents of the supernatants were determined (Table 10).

**Table 10.**

| Expression of TPS activity in E. coli transformed with TSS1 and TSL1. | | | |
|---|---|---|---|
| Host (ALKO 683) and transformants (ALKO3566 containing TSS1 and ALKO3568 containing TSL1) were grown, harvested and broken as described in the text. Cell homogenates and supernatants were assayed at once for TPS, using the standard assay and a blank assay from which G6P and F6P were omitted, and for TPP. Activities are shown as mU/g fresh cells unless stated otherwise. | | | |

| | ALKO 683 | ALKO3566 | ALKO3568 |
|---|---|---|---|
| Cell yield (g/200 ml) | 1.57 | 1.51 | 1.56 |

| Homogenates | | | |
|---|---|---|---|
| Standard TPS | 361 ± 75 | 1065 ± 118 | 260 ± 23 |
| TPS Blank | 363 ± 57 | 227 ± 45 | 117 ± 77 |
| Net TPS | 0 ± 20 | 840 ± 70 | 140 ± 50 |
| | | | |
| Standard TPP | 1130 ± 70 | 1110 ± 100 | 1190 ± 80 |
| | | | |

| Supernatants | | | |
|---|---|---|---|
| Standard TPS | 273 ± 73 | 699 ± 47 | 233 ± 68 |
| TPS Blank | 263 ± 21 | 155 ± 42 | 135 ± 9 |
| Net TPS | 10 ± 50 | 540 ± 10 | 100 ± 60 |
| | | | |
| Net TPS (mU/mg protein) | 0.08 | 4.50 | 0.87 |
| | | | |
| Standard TPP | 1130 ± 100 | 910 ± 90 | 1020 ± 80 |

Standard and blank TPS assays both showed accelerating progress curves and results in Table 10 are mean ± range of 5 min and 10 min assays, which were handled separately to calculate the net TPS activity. Essentially all of the standard TPS activity measured in the host cells and about half of that in ALKO3568 cells was due to a blank reaction (presumably a phosphodiesterase) generating UDP from UDPG in the absence of G6P and F6P. The net TPS activity of host cells grown under these conditions was close to zero, whereas cells transformed with TSS1 or TSL1 contained 840 or 140 mU/g fresh cells, most of which (64 % and 71 %, respectively) was soluble. Compared to the host preparation, the specific activities of the net TPS in the 28 000 g supernatants were increased about 50-fold (ALKO3566) and 10-fold (ALKO3568). There are probably two reasons for the very low TPS activity of the host cells: trehalose-6-phosphate synthase of E. coli is induced by high osmotic strength, and although some strains also acquire activity in stationary phase, the enzyme activity itself is strongly activated by higher (0.25 M) cation concentrations than in our assay conditions [Giaever et al (1988) Journal of Bacteriology 170, 2841-2849].

No significant change in the TPP activities was observed. Host cells already contained 1100 mU/g of TPP measured in 25 mM phosphate (and more than 5 U/g measured in 25 mM Hepes buffer). If transformation with the plasmids would have generated TPP activity with a TPP/TPS ratio the same as in pure trehalose synthase from yeast, then the increments in TPP (about 250 and 40 mU/g for ALKO3566 and ALKO3568, repectively) would have been undetectable for ALKO3568 and close to the experimental error for ALKO3566.

Western analyses (Fig. 19) showed that ALKO3566 specifically expressed a 57 kDa band recognized by anti-57K serum and more weakly reacting bands with smaller molecular masses. ALKO3568 specifically expressed bands recognized by anti-93K serum at about 60, 36 and 35 kDa (strong), suggesting that extensive degradation of the long chain occurs in ALKO3568 or that TSL1 is not correctly transcribed and translated.

These results disclose (1) TPS activity can be transferred to heterologous cells by either TSS1 or TSL1, (2) a TSS1 gene product has TPS activity and (3) also one or more (degraded) products of TSL1 has TPS activity. This latter finding is unexpected, because yeasts containing a defective (Example 7) or disrupted (Example 14) TSS1 gene lack TPS activity. Possibly ALKO3568 accumulates fortuitously degraded proteolytic products of the 123 kDa long chain of trehalose synthase that exhibit TPS activity even in the absence of the TSS1 product.

Obviously, transformation with TSS1 (or TSL1) alone can be used to introduce a trehalose synthetic pathway to an organism, such as E. coli HB101, that already has the capacity to generate trehalose from trehalose-6-phosphate, possibly via a nonspecific phosphatase.

### Example 14. Transformation of Yeast

### (1) Assembly of complete genes and truncated versions of TSL1.

Plasmids comprising the complete ORFs of TSS1 and TSL1 and a truncated ORF of TSL1 were assembled from appropriate immunopositive clones of the HaeIII and EcoRI libraries used in Examples 4 and 5 to sequence these genes:

### (a) The TSS1 gene with its promoter (pALK 752)

A 516 bp fragment was cut from HaeIII clone 7 with restriction enzymes DraI and BstEII (see Fig 6 for restriction sites). The DraI site marks the beginning of the disclosed TSS1 sequence. This fragment was joined to HaeIII clone 20 after this had been digested with BstEII and ClaI (the ClaI site was in the polylinker) and the ClaI end filled with Klenow fragment. The sequence at the junction at the BstEII site in the religated plasmid (shown in Fig 20a) was confirmed by sequencing.

### (b) The TSS1 gene without its promoter (pALK753)

HaeIII clone 21 was cut with the restriction enzyme Tth111I. To this site the following linker (SEQ ID NO:84, synthesized with the ABI DNA Synthesizer) was added:

This includes nucleotides -13 to +33 of TSS1 (see Fig. 4) but, when correctly orientated, introduces a SmaI site at nucleotide - 16 from the ATG start site. The plasmid (shown in Fig 20b) can be used to release with SmaI the ORF of the TSS1 gene and about 200 bp of its terminator for further constructions (e.g. expression vectors containing a new promoter).

### (c) The TSL1 gene with its promoter (pALK754)

EcoRI clone 10 was cut with the restriction enzymes MluI and NdeI, and the resulting 4.4 kb fragment was religated into the pBluescript SmaI site. This procedure destroyed all these sites, so that these restriction enzymes cannot be used in further manipulations. The plasmid is shown in Fig 20c.

### (d) The TSL1 gene without its promoter (pALK757)

Primers for the polymerase chain reaction (PCR) were made against the beginning of the TSL1 gene and the sequence at +318. PCR (Techne PHC-2 Heat/Cool Dri-Block^{R}) was used to synthesize (at 55 °C) a 325 bp fragment, which had at one end a SpeI site and close to the other end a BsmI site. This fragment was digested with BsmI and can be ligated to pALK754 after cutting the latter with SpeI (at the site in the pBluescript polylinker) and BsmI and filling the SpeI site with Klenow fragment. For further manipulations, the gene can be isolated by cutting the resulting plasmid with SpeI and, for example, ClaI.

### (e) A truncated TSL1 gene

A truncated version of TSL1 can be made by cutting pALK754 with StuI and joining the following linker (SEQ ID NO:85) to this site:

The linker recreates the StuI site and creates a new ATG start codon at +627 in frame with the coding sequence. Thus, this version of the gene encodes a truncated 123 kDa long chain lacking the first 209 amino acids. It was disclosed in Example 10 that removal of the first 325 or so amino acids proceeds without loss of catalytic activity, but releases trehalose synthase from strong inhibition by phosphate and the requirement for F6P. Hence, this construction can encode a truncated 123 kDa polypeptide leading to a trehalose synthase with increased activity at physiological phosphate and F6P concentrations. A new SmaI site is included in the linker. The sequence flanking the new ATG on the 5'-side resembles the original ATG flanking sequence and the surrounding nucleotides are in accordance with the sequences known to occur most frequently at positions -7 to +4.

### (2) Disruption mutants.

The TSS1 gene was disrupted to confirm that it is an essential gene in trehalose synthesis. The one-step gene disruption method [Rothstein, R.J. (1983) Methods in Enzymology 101, 202-211)] was used as follows:
Plasmid pALK752 was cut with XcmI. A blunted SalI-XhoI fragment containing the LEU2 gene from plasmid yEp13 [Broach, J et al (1979) Gene 8, 121-133] was ligated to the blunted XcmI site. The resulting plasmid was cut with NsiI and PvuI and the reaction mixture was run through a 0.8% low-melting point agarose gel. A band of 4 kb was excised from the gel and purified. S. cerevisiae strain S150-2B was transformed (using the one-step alkali-cation method of Chen et al [(1992) Current Genetics 21, 83-84}) with the 4 kb DNA fragment containing the TSS1 gene interrupted by the LEU2 gene. Leu⁺ transformants were selected on minimal plates lacking leucine and containing glucose or galactose, and the clones obtained were then grown on YPD or YP/2% galactose, respectively.

As expected the phenotype of the disruptants resembled the fdp1 and cif1 phenotypes (see Example 2). Only one transformant (ALKO3569) was isolated on glucose and the several transformants isolated on galactose were unable to grow on glucose. The glucose transformant and the tested galactose transformant (ALKO3570) did not accumulate trehalose in stationary phase (≤0.2 % of dry wt.), lacked TPS and had low TPP activity (≤ 10 % of wild type). The 57 kDa band could not be seen on Western blots. Southern analysis (Fig 21) showed that the TSS1 gene had been disrupted by a LEU2 gene, but the TSL1 gene was intact.

Another mutant, WDC-3A (see Table 1) with a disrupted TSS1 gene was obtained from the laboratory of Dr. C. Gancedo (Instituto de Investigaciones Biomédicas, CSIC, Madrid, Spain) as a cif1::HIS3 disruptant. This mutant was easier to transform than were the tss1::LEU2 disruptants, and so it was used to confirm that the TSS1 gene on a plasmid can confer TPS and TPP activities, trehalose accumulation and improved stress resistance. WDC-3A was transformed with the plasmid pMB4 (see Table 1; the plasmid contains an intact CIF1 *≡* TSS1 gene and a selectable URA3 marker) and transformants selected in the absence of uracil. Western analyses (not shown) indicated that the transformants has acquired the 57 kDa band absent from WDC-3A. The parent and a transformant were grown in parallel in minimal medium containing 2 % galactose and (transformant) no uracil or (parent) uracil. Duplicate cultures of each strain were harvested in early stationary phase after 28 h growth samples taken for studies of stress resistance, and the rest used for trehalose and enzyme assays (Table 11).

**Table 11.**

| Analysis of WDC-3A and its pMB14 transformant. | | |
|---|---|---|
| Duplicate cultures were analyzed separately for trehalose and combined for enzyme assays. TPS activities were corrected for UDPGase activity in the absence of G6P and F6P. | | |

| | WDC-3A (tss1::HIS3) | pMB14 (TSS1) Transformant |
|---|---|---|
| Cell mass (g/100 ml medium) | 2.6 | 2.8 |
| Trehalose (% of dry wt.) | 0.84, 0.81 | 2.9, 3.0 |

| Whole homogenates | | |
|---|---|---|
| TPP (U/g fresh yeast) | 0.02 | 0.84 |
| TPS (U/g fresh yeast) | 0.84 ± 0.37 | 17.9 ± 3.5 |

| 28,000 g supernatants | | |
|---|---|---|
| TPP (U/g fresh yeast) | 0.01 | 0.67 |
| TPS (U/G fresh yeast) | 0.22 ± 0.20 | 14.6 ± 3.3 |
| TPS (mU/mg protein) | 4.0 | 223 |

These results disclose that introduction of TSS1 on a plasmid can restore both TPS and TPP activities and increase the trehalose content of an organism. The TPP/TPS ratio (5 %) is much lower than that (about 35 %) of purified trehalose synthase whereas the baker' yeast used in Example 1 and the X2180 used in Example 2 both have TPP/TPS ratios in their homogenates close to that of pure enzyme. This suggests that transformation with TSS1 in pMB14 increases TPP only up to a limit set by the genetic background of the host (probably the amounts of 99 and 123 kDa polypeptides present) but causes a larger increase in TPS due to activity associated also with 57 kDa chains not incorporated into the trehalose synthase complex.

Samples of the transformant and host were frozen in water at 1 µg yeast/ml and kept for 5 days at -20 °C. The viability was then tested on plates containing YP/2 % galactose. After freezing stress, 1.0 ± 0.1 % of the transformants and ≤ 0.05 % of the host cells were viable. These results disclose that transformation of an organism with TSS1 can increase its resistance to freezing-stress.

### (3) Strategies for transformation.

Laboratory strains of S. cerevisiae bearing auxotrophic markers such as his3, leu2, lys 2, trp1 and ura3 can be easily transformed with the trehalose synthase genes by essentially the same methods described for transformation of tss1 disruptants with TSS1. Versions of the genes in which the natural promoters and terminators are intact or have been replaced by (stronger and regulatable) promoters and terminators from other yeast genes can be used. For example, PGKI [pMA91; Mellor et al (1983) Gene 24, 1-14], ADC1 [pAAH5; Ammerer (1983) Methods in Enzymology 101, 192-201] and MEL1 [pALK3537, pALK41, etc., Suominen, P.L. (1988) Doctoral dissertation, University of Helsinki] systems have been used to increase the expression levels of genes in s. cerevisiae and other yeast. The MEL1 system has the advantage that the expression can be regulated, being repressed by glucose and induced by galactose. Standard vectors are available [episomal and integrating and centromere yeast plasmids are reviewed by Rose & Broach (1990) Methods in Enzymology 185, 234-279 and Stearns, T., Ma, H & Botstein, D. (1990) Methods in Enzymology 185, 280-291] that incorporate auxotrophic markers such as HIS3, LEU2, TRP1 and URA3, which can be used to select the transformants. Vectors based on these principles, but suited to a particular task can be constructed by a person familiar with the art.

The basic strategy is to leave the yeast with an intact version of its natural genes for trehalose synthase and introduce, either on episomes or integrated into a yeast chromosome, extra copies of the genes. These may be under control of their own promoters, or of stronger promoters and promoters that can be regulated, for example by adding substances such as galactose to the growth medium or by changing the temperature. The use of such promoters has been described [see, e.g., Mylin et al. (1990) Methods in Enzymology 185, 297-308; Sledziewski et al. (1990) Methods in Enzymology 185, 351-366]. This strategy avoids problems that can be foreseen if all copies of the genes genes are put under tight control (such as the defects in sugar catabolism expected if TSS1 is not properly expressed; see Example 7.) Transformed yeast bearing additional copies of the genes with their natural promoters may accumulate enough trehalose to exhibit the desired improvement in stability. They may also cycle enough glucose units through trehalose during fermentative conditions to generate an ATPase that accelerates fermentation and increases the yield of ethanol on glucose. Alternatively, the promoters of one or more genes can be changed to promoters that are more active under fermentative conditions. In another aspect of the invention, copies of the ORFs of the genes can be inserted into expression vectors equipped with powerful promoters (that may be regulatable) to cause still larger increases in trehalose. This can be particularly useful for the production of trehalose.

Transforming yeast with two or all three genes can be achieved in several ways. The most obvious procedure is to use different auxotrophic markers and introduce the genes sequentially. Another method is to construct a YIp containing URA3 and a modified version of, say, TSL1 with a stronger promoter but still containing a region of homology upstream of this promoter. After directed integration of this plasmid to the chromosomal ura3 site and selection of URA+ transformants, mutants in which the URA3 has again been excised (with a frequency of about 1 x 10⁻⁴) can be selected by growth on media containing 5-fluoroorotic acid [see Stearns et al. (1990) loc. cit.]. Some of the selected cells would contain a new version of the gene, with the stronger promoter and can again be transformed, this time with, say, a modified TSS1 gene. The resultant transformants will contain one copy of TSL1 driven by the new promoter, and two copies of TSS1, one of which is still under the control of its natural promoter. Thirdly, a YIp containing two or all three genes can be used to introduce the genes in a single step. various methods to transform industrial, polyploid yeast, which lack auxotrophic markers have been described in the literature. Earlier methods have been reviewed by Knowles, J.K.C. & Tubb, R.S. [(1987) E.B.C. symposium on brewer's yeast, Helsinki, 1986. Monograph XII 169-185] and include the use of marker genes that confer resistance to antibiotics, methylglyoxal, copper, cinnamic acid and other compounds. These markers facilitate selection of transformants. Some of the marker genes are themselves of yeast origin, and so are preferred for acceptability reasons. When suitable modifications and combinations of the genes have been identified by using laboratory yeast, they may be transferred to industrial yeast using these procedures or others described in the literature, such as co-transformation with pALK2 and pALK7 [Suominen, P. I. (1988) loc. cit.]. These plasmids contain a readily selectable MEL1 marker gene on a 2 µ-based plasmid that can readily be cured, thus facilitating sequential transformation with more than one gene if it is not practicable to introduce the modified genes in one step using this co-transformation procedure.

### Example 15. Transformation of crop plants.

Methods for the transformation of higher plants, including crop plants of economic importance, have been described [Goodman et al (1987) Science 236, 48-54; Weising et al, (1988) Annual Review of Genetics 22, 421-477; Glasser & Fraley (1989) Science 244, 1293-1299; Lindsey, K. (1992) Journal of Biotechnology 26, 1 - 28] and laboratory manuals setting out standard procedures are available such as the Plant Molecular Biology Manual [ed. Gelvin & Schnilperoort (1988) Kluwer Academic Press]. Of particular utility is the use of tissue specific promoters from the genes of proteins that are expressed in a highly tissue-specific manner [see, e.g., Higgins (1984) Annual Review of Plant Physiology 35, 191 et seg.; Shotwell and Larkins (1989) in The Biochemistry of Plants 15, 297 et seg.]. The use of such promoters will allow the expression of trehalose synthase in (a) specifically the frost- and drought-sensitive tissues of plants so that they may be protected from these and equivalent stresses without diverting carbohydrate metabolism in the major storage tissues, or alternatively (b) precisely in the edible tissues. The purpose of this second alternative is to cause the accumulation of trehalose in products such as the fruit of tomatoes, in order to increase the shelf-life of these products. The expression of non-plant genes, with higher A+T contents than are commonly found in plant genes can generally be improved by changing the codons to increase the G+C content, and in particular to avoid regions of overall high A+T content Perlak et al. (1991) loc. cit.]. It is foreseen that such modifications will be beneficial in the case of the genes TSS1 and TSL1, which have A+T-rich regions. Selection systems are available for use in the transformation of higher plants, including plasmids comprising the gene (hpt) for hygromycin phosphotransferase [Dale & Ow (1991) Proceedings of the National Academy of Sciences, USA 88, 1055810562]. These and similar methods familiar to persons skilled in the art can be used, first to introduce various modifications of the yeast trehalose synthase genes into Arabidopsis thaliana, and then to transfer the most successful modifications to plants of economic importance.

One example of how one would transform a crop plant (dicots and some monocots) is via a Ti plasmid. A large fragment of the Ti plasmid encompassing both the T-DNA and vir regions is first cloned into the common bacterial plasmid pBR322. One or more of the trehalose synthase genes are then cloned into a non-essential region of the T-DNA and introduced into Agrobacterium tumefaciens carrying an intact Ti plasmid. The plants are then infected with these bacteria and the gene products of the vir region on the intact Ti plasmid mobilize the recombinant T-DNA, and the recombinant T-DNA integrates into the plant genome. One or more of the trehalose synthase genes can be introduced into the plant in this manner, by inserting the genes into either the same plasmid or separate plasmids.

### SEQUENCE LISTING

(1) GENERAL INFORMATION
   (i) APPLICANT:
      (A) NAME: ALKO LTD. (et al.)
      (B) STREET: Salmisaarenranta 7
      (C) CITY: Helsinki
      (D) COUNTRY: FINLAND
      (E) POSTAL CODE: SF-00100
   (ii) TITLE OF INVENTION: Increasing the trehalose content of organisms by transforming them with combinations of the structural genes for trehalose synthase.
   (iii) NUMBER OF SEQUENCES: 85
   (iv) PRIOR APPLICATION DATA:
      (A)APPLICATION NUMBER: US / 07/836,021
      (B)FILING DATE: 14 February 1992
      (A)APPLICATION NUMBER: US / 07/841,997
      (B)FILING DATE: 28 February 1992
(2)INFORMATION FOR SEQ ID NO:1:
   (i) SEQUENCE CHARACTERISTICS:
      (A) LENGTH: 2481 base pairs
      (B) TYPE: Nucleotide
      (C) STRANDEDNESS: Doublestranded
      (D) TOPOLOGY: Linear
   (ii) MOLECULAR TYPE: Genomic DNA
   (iii) HYPOTHETICAL: no
   (iv) ANTI-SENSE: no
   (vi) ORIGINAL SOURCE:
      (A) ORGANISM: Saccharomyces cerevisiae
      (B) STRAIN: S288C
      (E) HAPLOTYPE: Haploid
   (vii) IMMEDIATE SOURCE
      (A) LIBRARY: Genomic
      (B) CLONE: 20
   (viii) POSITION IN GENOME:
      (A) CHROMOSOME: 2R
   (xi) SEQUENCE DESCRIPTION: SEQ ID NO:1
   TTTTTAAACG TATATAGATG TCTACATGTG TGTTTTTGTT TTTTTACGTA   50
   CGTATACCCA CCTATATATG CATAATCCGT AATTGAAAAA AAAAAAAGTA  100
   AAGATCAAGG AACACATCAC CCTGGGCACA TCAAGCGTGA GGAATGCCGT  150
   CCAACTGGTG GAGACGCTTG ATTTGCTCTT TTTGTTCCTG GGTCCAACCC  200
   GGTCTCGAAG AACATCAGCA CCACGCCCGC AACGACAAAG AACATTGCAA  250
   TACACTTGCA TATGTGAGCA TAGTCGAGCG GTCCGTTCTG TGGTTGATGC  300
   TGTTGTTCTT TCTTCTGTTT GTCAGGGGTG ATAGCCATAT CTTCGTGCTC  350
   TTGTTGCGAT TGTTCTGTTC CATCTGCACC AGAACAAAGA ACAAAAGAAC  400
   AAGGAACAAA GTCCAAGCAC GTCAGCGCTG TTTATAAGGG GATTGACGAG  450
   GGATCGGGCC TAGAGTGCCA GCGCGCCAGG GAGAGGGAGC CCCCTGGGCC  500
   CTCATCCGCA GGCTGATAGG GGTCACCCCG CTGGGCAGGT CAGGGCAGGG  550
   GCTCTCAGGG GGGCGCCATG GACAAACTGC ACTGAGGTTC TAAGACACAT  600
   GTATTATTGT GAGTATGTAT ATATAGAGAG AGATTAAGGC GTACAGCCGG  650
   GTGGTAGAGA TTGATTAACT TGGTAGTCTT ATCTTGTCAA TTGAGTTTCT  700
   GTCAGTTTCT TCTTGAACAA GCACGCAGCT AAGTAAGCAA CAAAGCAGGC  750
   TAACAAACTA GGTACTCACA TACAGACTTA TTAAGACATA GAACTATGAC  800
   TACGGATAAC GCTAAGGCGC AACTGACCTC GTCTTCAGGG GGTAACATTA  850
   TTGTGGTGTC CAACAGGCTT CCCGTGACAA TCACTAAAAA CAGCAGTACG  900
   GGACAGTACG AGTACGCAAT GTCGTCCGGA GGGCTGGTCA CGGCGTTGGA  950
   AGGGTTGAAG AAGACGTACA CTTTCAAGTG GTTCGGATGG CCTGGGCTAG 1000
   AGATTCCTGA CGATGAGAAG GATCAGGTGA GGAAGGACTT GCTGGAAAAG 1050
   TTTAATGCCG TACCCATCTT CCTGAGCGAT GAAATCGCAG ACTTACACTA 1100
   CAACGGGTTC AGTAATTCTA TTCTATGGCC GTTATTCCAT TACCATCCTG 1150
   GTGAGATCAA TTTCGACGAG AATGCGTGGT TCGGATACAA CGAGGCAAAC 1200
   CAGACGTTCA CCAACGAGAT TGCTAAGACT ATGAACCATA ACGATTTAAT 1250
   CTGGGTGCAT GATTACCATT TGATGTTGGT TCCGGAAATG TTGAGAGTCA 1300
   AGATTCACGA GAAGCAACTG CAAAACGTTA AGGTCGGGTG GTTCCTGCAC 1350
   ACACCATTCC CTTCGAGTGA AATTTACAGA ATCTTACCTG TCAGACAAGA 1400
   GATTTTGAAG GGTGTTTTGA GTTGTGATTT AGTCGGGTTC CACACATACG 1450
   ATTATGCAAG ACATTTCTTG TCTTCCGTGC AAAGAGTGCT TAACGTGAAC 1500
   ACATTGCCTA ATGGGGTGGA ATACCAGGGC AGATTCGTTA ACGTAGGGGC 1550
   CTTCCCTATC GGTATCGACG TGGACAAGTT CACCGATGGG TTGAAAAAGG 1600
   AATCCGTACA AAAGAGAATC CAACAATTGA AGGAAACTTT CAAGGGCTGC 1650
   AAGATCATAG TTGGTGTCGA CAGGCTGGAT TACATCAAAG GTGTGCCTCA 1700
   GAAGTTGCAC GCCATGGAAG TGTTTCTGAA CGAGCATCCA GAATGGAGGG 1750
   GCAAGGTTGT TCTGGTACAG GTTGCAGTGC CAAGTCGTGG AGATGTGGAA 1800
   GAGTACCAAT ATTTAAGATC TGTGGTCAAT GAGTTGGTCG GTAGAATCAA 1850
   CGGTCAGTTC GGTACTGTGG AATTCGTCCC CATCCATTTC ATGCACAAGT 1900
   CTATACCATT TGAAGAGCTG ATTTCGTTAT ATGCTGTGAG CGATGTTTGT 1950
   TTGGTCTCGT CCACCCGTGA TGGTATGAAC TTGGTTTCCT ACGAATATAT 2000
   TGCTTGCCAA GAAGAAAAGA AAGGTTCCTT AATCCTGAGT GAGTTCACAG 2050
   GTGCCGCACA ATCCTTGAAT GGTGCTATTA TTGTAAATCC TTGGAACACC 2100
   GATGATCTTT CTGATGCCAT CAACGAGGCC TTGACTTTGC CCGATGTAAA 2150
   GAAAGAAGTT AACTGGGAAA AACTTTACAA ATACATCTCT AAATACACTT 2200
   CTGCCTTCTG GGGTGAAAAT TTCGTCCATG AATTATACAG TACATCATCA 2250
   AGCTCAACAA GCTCCTCTGC CACCAAAAAC TGATGAACCC GATGCAAATG 2300
   AGACGATCGT CTATTCCTGG TCCGGTTTTC TCTGCCCTCT CTTCTATTCA 2350
   CTTTTTTTAT ACTTTATATA AAATTATATA AATGACATAA CTGAAACGCC 2400
   ACACGTCCTC TCCTATTCGT TAACGCCTGT CTGTAGCGCT GTTACTGAAG 2450
   CTGCGCAAGT AGTTTTTTCA CCGTATAGGC C                      2481
(3)INFORMATION FOR SEQ ID NO:2
   (i) SEQUENCE CHARACTERISTICS
      (A) LENGTH: 495 amino acids
      (B) TYPE: amino acid
      (D) TOPOLOGY: linear
   (ii) MOLECULAR TYPE: Polypeptide
   (iii) HYPOTHETICAL: Yes
   (xi) SEQUENCE DESCRIPTION:SEQ ID NO:2
   Met Thr Thr Asp Asn Ala Lys Ala Gln Leu Thr Ser Ser Ser Gly
                   5                   10                  15
   Gly Asn Ile Ile Val Val Ser Asn Arg Leu Pro Val Thr Ile Thr
                   20                  25                  30
   Lys Asn Ser Ser Thr Gly Gln Tyr Glu Tyr Ala Met Ser Ser Gly
                   35                  40                  45
   Gly Leu Val Thr Ala Leu Glu Gly Leu Lys Lys Thr Tyr Thr Phe
                   50                  55                  60
   Lys Trp Phe Gly Trp Pro Gly Leu Glu Ile Pro Asp Asp Glu Lys
                   65                  70                  75
   Asp Gln Val Arg Lys Asp Leu Leu Glu Lys Phe Asn Ala Val Pro
                   80                  85                  90
   Ile Phe Leu Ser Asp Glu Ile Ala Asp Leu His Tyr Asn Gly Phe
                   95                  100                 105
   Ser Asn Ser Ile Leu Trp Pro Leu Phe His Tyr His Pro Gly Glu
                   110                 115                 120
   Ile Asn Phe Asp Glu Asn Ala Trp Phe Gly Tyr Asn Glu Ala Asn
                   125                 130                 135
   Gln Thr Phe Thr Asn Glu Ile Ala Lys Thr Met Asn His Asn Asp
                   140                 145                 150
   Leu Ile Trp Val His Asp Tyr His Leu Met Leu Val Pro Glu Met
                   155                 160                 165
   Leu Arg Val Lys Ile His Glu Lys Gln Leu Gln Asn Val Lys Val
                   170                 175                 180
   Gly Trp Phe Leu His Thr Pro Phe Pro Ser Ser Glu Ile Tyr Arg
                   185                 190                 195
   Ile Leu Pro Val Arg Gln Glu Ile Leu Lys Gly Val Leu Ser Cys
                   200                 205                 210
   Asp Leu Val Gly Phe His Thr Tyr Asp Tyr Ala Arg His Phe Leu
                   215                 220                 225
   Ser Ser Val Gln Arg Val Leu Asn Val Asn Thr Leu Pro Asn Gly
                   230                 235                 240
   Val Glu Tyr Gln Gly Arg Phe Val Asn Val Gly Ala Phe Pro Ile
                   245                 250                 255
   Gly Ile Asp Val Asp Lys Phe Thr Asp Gly Leu Lys Lys Glu Ser
                   260                 265                 270
   Val Gln Lys Arg Ile Gln Gln Leu Lys Glu Thr Phe Lys Gly Cys
                   275                 280                 285
   Lys Ile Ile Val Gly Val Asp Arg Leu Asp Tyr Ile Lys Gly Val
                   290                 295                 300
   Pro Gln Lys Leu His Ala Met Glu Val Phe Leu Asn Glu His Pro
                   305                 310                 315
   Glu Trp Arg Gly Lys Val Val Leu Val Gln Val Ala Val Pro Ser
                   320                 325                 330
   Arg Gly Asp Val Glu Glu Tyr Gln Tyr Leu Arg Ser Val Val Asn
                   335                 340                 345
   Glu Leu Val Gly Arg Ile Asn Gly Gln Phe Gly Thr Val Glu Phe
                   350                 355                 360
   Val Pro Ile His Phe Met His Lys Ser Ile Pro Phe Glu Glu Leu
                   365                 370                 375
   Ile Ser Leu Tyr Ala Val Ser Asp Val Cys Leu Val Ser Ser Thr
                   380                 385                 390
   Arg Asp Gly Met Asn Leu Val Ser Tyr Glu Tyr Ile Ala Cys Gln
                   395                 400                 405
   Glu Glu Lys Lys Gly Ser Leu Ile Leu Ser Glu Phe Thr Gly Ala
                   410                 415                 420
   Ala Gln Ser Leu Asn Gly Ala Ile Ile Val Asn Pro Trp Asn Thr
                   425                 430                 435
   Asp Asp Leu Ser Asp Ala Ile Asn Glu Ala Leu Thr Leu Pro Asp
                   440                 445                 450
   Val Lys Lys Glu Val Asn Trp Glu Lys Leu Tyr Lys Tyr Ile Ser
                   455                 460                 465
   Lys Tyr Thr Ser Ala Phe Trp Gly Glu Asn Phe Val His Glu Leu
                   470                 475                 480
   Tyr Ser Thr Ser Ser Ser Ser Thr Ser Ser Ser Ala Thr Lys Asn
                   485                 490                 495
(4)INFORMATION FOR SEQ ID NO:3
   (i) SEQUENCE CHARACTERISTICS
      (A) LENGTH: 3000 base pairs
      (B) TYPE: Nucleotide
      (C) STRANDEDNESS: Doublestranded
      (D) TOPOLOGY: Linear
   (ii) MOLECULAR TYPE: Genomic DNA
   (iii) HYPOTHETICAL: No
   (iv) ANTI-SENSE: No
   (vi) ORIGINAL SOURCE:
      (A) ORGANISM: Saccharomyces cerevisiae
      (B) STRAIN: S288C
      (E) HAPLOTYPE: Haploid
   (vii) IMMEDIATE SOURCE:
      (A) LIBRARY: Genomic
      (B) CLONE: 6
   (vii) POSITION IN GENOME
      (A) CHROMOSOME: 13
   (xi) SEQUENCE DESCRIPTION: SEQ ID NO:3
   CCTCCTCTGG ATCTTCTGGG TCTTCTGCGC CACCTTCCAT TAAAAGGATT   50
   ACGCCCCACT TGACTGCGTC TGCTGCAAAA CAGCGTCCCT TATTGGCTAA  100
   ACAGCCTTCT AATCTGAAAT ATTCGGAGTT AGCAGATATT TCGTCGAGTG  150
   AGACGTCTTC GCAGCATAAT GAGTCGGACC CGGATGATCT AACTACTGCC  200
   CTGACGAGGA TATGTTTCTG ATTAGGAATT GATGACGCGA GAGGACTACA  250
   AGGTTCAAAG TTCGGCGCTA TTCATAAATC AACTAAGAAA TATGCGCTGT  300
   TAAGGTCATC TCAGGAGCTG TTTAGCCGTC TTCCATGGTC GATCGTTCCC  350
   TCTATCAAAG GTAATGGCGC CATGAAGAAC GCCATAAACA CTGCAGTCTT  400
   GGAGAATATC ATTCCGCACC GTCATGTTAA GTGGGTCGGT ACCGTCGGAA  450
   TCCCAACGGA TGAGATTCCG GAAAATATCC TTGCGAACAT CTCTGACTCT  500
   TTAAAAGACA AGTACGACTC CTATCCTGTC CTTACGGACG ACGACACCTT  550
   CAAAGCCGCA TACAAAAACT ACTGTAAACA AATCTTGTGG CCTACGCTGC  600
   ATTACCAGAT TCCAGACAAT CCGAACTCGA AGGCTTTTGA AGATCACTCT  650
   TGGAAGTTCT ATAGAAACTT AAACCAAAGG TTTGCGGACG CGATCGTTAA  700
   AATCTATAAG AAAGGTGACA CCATCTGGAT TCATGATTAC CATTTAATGC  750
   TGGTTCCGCA GATGGTGAGA GACGTCTTGC CTTTTGCCAA AATAGGATTT  800
   ACCTTACATG TCTCGTTCCC CAGTAGTGAA GTGTTTAGGT GTCTGGCTCA  850
   GCGTGAGAAG ATCTTAGAAG GCTTGACCGG TGCAGACTTT GTCGGCTTCC  900
   AGACGAGGGA GTATGCAAGA CATTTCTTAC AGACGTCTAA CCGTCTGCTA  950
   ATGGCGGACG TGGTACATGA TGAAGAGCTA AAGTATAACG GCAGAGTCGT 1000
   TTCTGTGAGG TTCACCCCAG TTGGTATCGA CGCCTTTGAT TTGCAATCGC 1050
   AATTGAAGGA TGGAAGTGTC ATGCAATGGC GTCAATTGAT TCGTGAAAGA 1100
   TGGCAAGGGA AAAAACTAAT TGTGTGTCGT GATCAATTCG ATAGAATTAG 1150
   AGGTATTCAC AAGAAATTGT TGGCTTATGA AAAATTCTTG GTCGAAAATC 1200
   CGGAATACGT GGAAAAATCG ACTTTAATTC AAATCTGTAT TGGAAGCAGT 1250
   AAGGATGTAG AACTGGAGCG CCAGATCATG ATTGTCGTGG ATAGAATCAA 1300
   CTCGCTATCC ACCAATATTA GTATTTCTCA ACCTGTGGTG TTTTTGCATC 1350
   AAGATCTAGA TTTTTCTCAG TATTTAGCTT TGAGTTCAGA GGCAGATTTG 1400
   TTCGTAGTCA GCTCTCTAAG GGAAGGTATG AACTTGACAT GTCACGAATT 1450
   TATCGTTTGT TCTGAGGACA AAAATGCTCC CCTACTGTTG TCAGAATTTA 1500
   CTGGTAGTGC ATCTTTATTG AATGATGGCG CTATAATAAT TAACCCATGG 1550
   GATACCAAGA ACTTCTCACA AGCCATTCTC AAGGGGTTGG AGATGCCATT 1600
   CGATAAGAGA AGGCCACAGT GGAAGAAATT GATGAAAGAC ATTATCAACA 1650
   ACGACTCTAC AAACTGGATC AAGACTTCTT TACAAGATAT TCATATTTCG 1700
   TGGCAATTCA ATCAAGAAGG TTCCAAGATC TTCAAATTGA ATACAAAAAC 1750
   ACTGATGGAA GATTACCAGT CATCTAAAAA GCGTATGTTT GTTTTCAACA 1800
   TTGCTGAACC ACCTTCATCG AGAATGATTT CCATACTGAA TGACATGACT 1850
   TCTAAGGGCA ATATCGTTTA CATCATGAAC TCATTTCCAA AGCCCATTCT 1900
   GGAAAATCTT TACAGTCGTG TGCAAAACAT TGGGTTGATT GCCGAGAATG 1950
   GTGCATACGT TAGTCTGAAC GGTGTATGGT ACAACATTGT TGATCAAGTC 2000
   GATTGGCGTA ACGATGTAGC CAAAATTCTC GAGGACAAAG TGGAGAGATT 2050
   ACCTGGCTCG TACTACAAGA TAAATGAGTC CATGATCAAG TTCCACACTG 2100
   AAAATGCGGA AGATCAAGAT CGTGTAGCTA GTGTTATCGG TGATGCCATC 2150
   ACACATATCA ATACTGTTTT TGACCACAGA GGTATTCATG CCTACGTTTA 2200
   CAAAAACGTT GTTTCCGTAC AACAAGTGGG ACTTTCCTTA TCGGCAGCTC 2250
   AATTTCTTTT CAGATTCTAT AATTCTGCTT CGGATCCACT GGATACGAGT 2300
   TCCGGCCAAA TCACAAATAT TCAGACACCA TCTCAACAAA ATCCTTCAGA 2350
   TCAAGAACAA CAACCTCCAG CCTCTCCCAC TGTGTCGATG AACCATATTG 2400
   ATTTCGCATG TGTCTCTGGT TCATCGTCTC CTGTGCTTGA ACCATTGTTC 2450
   AAATTGGTCA ATGATGAAGC AAGTGAAGGG CAAGTAAAAG CCGGACACGC 2500
   CATTGTTTAT GGTGATGCTA CTTCTACTTA TGCCAAAGAA CATGTAAATG 2550
   GGTTAAACGA ACTTTTCACG ATCATTTCAA GAATCATTGA AGATTAAATT 2600
   TTACCATTTT AAAATTTTAA TGTTCTTGGG TATGAACTTT TATTTTCAAC 2650
   TGCTTATTAT ATATCAATTC TATAAATTTT TTTCTTCTCT CTAACGACCA 2700
   ATTATAAAAT TCATCCTCTT ATTTATTACA GCATCTTATA CATTATGTAT 2750
   ATGGGTAGCT ATTATTCATT TTTGCTTCGT AAGGACTTTT TTTGTCAACT 2800
   TTTTCATCCT AAGCGGCTAA AAGTGATTGG AGAGGAATGT CCAGGCGACC  2850
   AATGATAAAA ACGCTTTCTC TTGGAACAAG AAATAGGAGC AATTGACAGT  2900
   TGTCGATGAA CAGCGAAAAT AGTAAGATAA CCTTCAAGCC CAATATTCTA  2950
   ATTAAAGGCG TTTATATATT TGTACTTTAT GGTATGTGCA TATGTATTGT  3000
(5) INFORMATION FOR SEQ ID NO:4
   (i) SEQUENCE CHARACTERISTICS:
      (A) LENGTH: 785 amino acids
      (B) TYPE: Amino acid
      (D) TOPOLOGY: Linear
   (ii) MOLECULAR TYPE: Polypeptide
   (iii) HYPOTHETICAL: Yes
   (v) FRAGMENT TYPE: C-terminal
   (xi) SEQUENCE DESCRIPTION: SEQ ID NO:4
   Arg Gly Leu Gln Gly Ser Lys Phe Gly Ala Ile His Lys Ser Thr
                   5                   10                  15
   Lys Lys Tyr Ala Leu Leu Arg Ser Ser Gln Glu Leu Phe Ser Arg
                   20                  25                  30
   Leu Pro Trp Ser Ile Val Pro Ser Ile Lys Gly Asn Gly Ala Met
                   35                  40                  45
   Lys Asn Ala Ile Asn Thr Ala Val Leu Glu Asn Ile Ile Pro His
                   50                  55                  60
   Arg His Val Lys Trp Val Gly Thr Val Gly Ile Pro Thr Asp Glu
                   65                  70                  75
   Ile Pro Glu Asn Ile Leu Ala Asn Ile Ser Asp Ser Leu Lys Asp
                   80                  85                  90
   Lys Tyr Asp Ser Tyr Pro Val Leu Thr Asp Asp Asp Thr Phe Lys
                   95                  100                 105
   Ala Ala Tyr Lys Asn Tyr Cys Lys Gln Ile Leu Trp Pro Thr Leu
                   110                 115                 120
   His Tyr Gln Ile Pro Asp Asn Pro Asn Ser Lys Ala Phe Glu Asp
                   125                 130                 135
   His Ser Trp Lys Phe Tyr Arg Asn Leu Asn Gln Arg Phe Ala Asp
                   140                 145                 150
   Ala Ile Val Lys Ile Tyr Lys Lys Gly Asp Thr Ile Trp Ile His
                   155                 160                 165
   Asp Tyr His Leu Met Leu Val Pro Gln Met Val Arg Asp Val Leu
                   170                 175                 180
   Pro Phe Ala Lys Ile Gly Phe Thr Leu His Val Ser Phe Pro Ser
                   185                 190                 195
   Ser Glu Val Phe Arg Cys Leu Ala Gln Arg Glu Lys Ile Leu Glu
                   200                 205                 210
   Gly Leu Thr Gly Ala Asp Phe Val Gly Phe Gln Thr Arg Glu Tyr
                   215                 220                 225
   Ala Arg His Phe Leu Gln Thr Ser Asn Arg Leu Leu Met Ala Asp
                   230                 235                 240
   Val Val His Asp Glu Glu Leu Lys Tyr Asn Gly Arg Val Val Ser
   245 250 255
   Val Arg Phe Thr Pro Val Gly Ile Asp Ala Phe Asp Leu Gln Ser
   260 265 270
   Gln Leu Lys Asp Gly Ser Val Met Gln Trp Arg Gln Leu Ile Arg
   275 280 285
   Glu Arg Trp Gln Gly Lys Lys Leu Ile Val Cys Arg Asp Gln Phe
   290 295 300
   Asp Arg Ile Arg Gly Ile His Lys Lys Leu Leu Ala Tyr Glu Lys
   305 310 315
   Phe Leu Val Glu Asn Pro Glu Tyr Val Glu Lys Ser Thr Leu Ile
   320 325 330
   Gln Ile Cys Ile Gly Ser Ser Lys Asp Val Glu Leu Glu Arg Gln
   335 340 345
   Ile Met Ile Val Val Asp Arg Ile Asn Ser Leu Ser Thr Asn Ile
   350 355 360
   Ser Ile Ser Gln Pro Val Val Phe Leu His Gln Asp Leu Asp Phe
   365 370 375
   Ser Gln Tyr Leu Ala Leu Ser Ser Glu Ala Asp Leu Phe Val Val
   380 385 390
   Ser Ser Leu Arg Glu Gly Met Asn Leu Thr Cys His Glu Phe Ile
   395 400 405
   Val Cys Ser Glu Asp Lys Asn Ala Pro Leu Leu Leu Ser Glu Phe
   410 415 420
   Thr Gly Ser Ala Ser Leu Leu Asn Asp Gly Ala Ile Ile Ile Asn
   425 430 435
   Pro Trp Asp Thr Lys Asn Phe Ser Gln Ala Ile Leu Lys Gly Leu
   440 445 450
   Glu Met Pro Phe Asp Lys Arg Arg Pro Gln Trp Lys Lys Leu Met
   455 460 465
   Lys Asp Ile Ile Asn Asn Asp Ser Thr Asn Trp Ile Lys Thr Ser
   470 475 480
   Leu Gln Asp Ile His Ile Ser Trp Gln Phe Asn Gln Glu Gly Ser
   485 490 495
   Lys Ile Phe Lys Leu Asn Thr Lys Thr Leu Met Glu Asp Tyr Gln
   500 505 510
   Ser Ser Lys Lys Arg Met Phe Val Phe Asn Ile Ala Glu Pro Pro
   515 520 525
   Ser Ser Arg Met Ile Ser Ile Leu Asn Asp Met Thr Ser Lys Gly
   530 535 540
   Asn Ile Val Tyr Ile Met Asn Ser Phe Pro Lys Pro Ile Leu Glu
   545 550 555
   Asn Leu Tyr Ser Arg Val Gln Asn Ile Gly Leu Ile Ala Glu Asn
   560 565 570
   Gly Ala Tyr Val Ser Leu Asn Gly Val Trp Tyr Asn Ile Val Asp
   575 580 585
   Gln Val Asp Trp Arg Asn Asp Val Ala Lys Ile Leu Glu Asp Lys
   590 595 600
   Val Glu Arg Leu Pro Gly Ser Tyr Tyr Lys Ile Asn Glu Ser Met
   605 610 615
   Ile Lys Phe His Thr Glu Asn Ala Glu Asp Gln Asp Arg Val Ala
   620 625 630
   Ser Val Ile Gly Asp Ala Ile Thr His Ile Asn Thr Val Phe Asp
   635 640 645
   His Arg Gly Ile His Ala Tyr Val Tyr Lys Asn Val Val Ser Val
   650 655 660
   Gln Gln Val Gly Leu Ser Leu Ser Ala Ala Gln Phe Leu Phe Arg
                           665                     670                     675
   Phe Tyr Asn Ser Ala Ser Asp Pro Leu Asp Thr Ser Ser Gly Gln
                   680                     685                     690
   Ile Thr Asn Ile Gln Thr Pro Ser Gln Gln Asn Pro Ser Asp Gln
                   695                     700                     705
   Glu Gln Gln Pro Pro Ala Ser Pro Thr Val Ser Met Asn His Ile
                   710                     715                     720
   Asp Phe Ala Cys Val Ser Gly Ser Ser Ser Pro Val Leu Glu Pro
                   725                     730                     735
   Leu Phe Lys Leu Val Asn Asp Glu Ala Ser Glu Gly Gln Val Lys
                   740                     745                     750
   Ala Gly His Ala Ile Val Tyr Gly Asp Ala Thr Ser Thr Tyr Ala
                   755                     760                     765
   Lys Glu His Val Asn Gly Leu Asn Glu Leu Phe Thr Ile Ile Ser
                   770                     775                     780
   Arg Ile Ile Glu Asp
                   785
(6) INFORMATION FOR SEQ ID NO:5
   (i) SEQUENCE CHARACTERISTICS:
      (A) LENGTH: 4 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:5
   Tyr Ile Ser Lys
(7)INFORMATION FOR SEQ ID NO:6
   (i) SEQUENCE CHARACTERISTICS:
      (A) LENGTH: 9 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:6
   Asp Val Glu Glu Tyr Gln Tyr Leu Arg
                   5
(8)INFORMATION FOR SEQ ID NO:7
   (i) SEQUENCE CHARACTERISTICS:
      (A) LENGTH: 8 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:7
   His Phe Leu Ser Ser Val Gln Arg
                   5
(9) INFORMATION FOR SEQ ID NO:8
   (i) SEQUENCE CHARACTERISTICS:
      (A) LENGTH: 14 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:8
   Val Leu Asn Val Asn Thr Leu Pro Asn Gly Val Glu Tyr Gln
                   5                   10
(10) INFORMATION FOR SEQ ID NO:9
   (i) SEQUENCE CHARACTERISTICS:
      (A) LENGTH: 9 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:9
   Ser Val Val Asn Glu Leu Val Gly Arg
   5
(11) INFORMATION FOR SEQ ID NO:10
   (i) SEQUENCE CHARACTERISTICS:
      (A) LENGTH: 4
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:10
   Glu Thr Phe Lys
(12) INFORMATION FOR SEQ ID NO:11
   (i) SEQUENCE CHARACTERISTICS:
      (A) LENGTH: 5 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:11
   Leu Asp Tyr Ile Lys
   5
(13) INFORMATION FOR SEQ ID NO:12
   (i) SEQUENCE CHARACTERISTICS:
      (A) LENGTH: 5 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:12
   Ile Leu Pro Val Arg
                     5
(14) INFORMATION FOR SEQ ID NO:13
   (i) SEQUENCE CHARACTERISTICS:
      (A) LENGTH: 6 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:13
   Glu Val Asn Xaa Glu Lys
                     5
(15) INFORMATION FOR SEQ ID NO:14
   (i) SEQUENCE CHARACTERISTICS:
      (A) LENGTH: 5 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:14
   Phe Tyr Asp Xaa Xaa
                     5
(16) INFORMATION FOR SEQ ID NO:15
   (i) SEQUENCE CHARACTERISTICS:
      (A) LENGTH: 13 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:15
   Leu Xaa Ala Met Glu Val Phe Leu Asn Glu Xaa Pro Glu
                   5                   10
(17) INFORMATION FOR SEQ ID NO:16
   (i) SEQUENCE CHARACTERISTICS:
      (A) LENGTH: 14 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:16
   Tyr Thr Ser Ala Phe Trp Gly Glu Asn Phe Val Xaa Glu Leu
                   5                   10
(18) INFORMATION FOR SEQ ID NO:17
   (i) SEQUENCE CHARACTERISTICS:
      (A) LENGTH 9 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:17
(19) INFORMATION FOR SEQ ID NO:18
   (i) SEQUENCE CHARACTERISTICS:
      (A) LENGTH 6 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:18
(20) INFORMATION FOR SEQ ID NO:19
   (i) SEQUENCE CHARACTERISTICS:
      (A) LENGTH 7 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:19
(21) INFORMATION FOR SEQ ID NO:20
   (i) SEQUENCE CHARACTERISTICS:
      (A) LENGTH 12 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:20
(22) INFORMATION FOR SEQ ID NO:21
   (i) SEQUENCE CHARACTERISTICS:
      (A) LENGTH 5 amino acids
      (B)TYPE: Amino acid
      (D)TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:21
(23) INFORMATION FOR SEQ ID NO:22
   (i) SEQUENCE CHARACTERISTICS:
      (A) LENGTH 6 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:22
(24) INFORMATION FOR SEQ ID NO:23
   (i) SEQUENCE CHARACTERISTICS:
      (A) LENGTH 5 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:23
(25) INFORMATION FOR SEQ ID NO:24
   (i) SEQUENCE CHARACTERISTICS:
      (A) LENGTH 15 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:24
(26) INFORMATION FOR SEQ ID NO:25
   (i) SEQUENCE CHARACTERISTICS:
      (A) LENGTH 11 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:25
(27) INFORMATION FOR SEQ ID NO:26
   (i) SEQUENCE CHARACTERISTICS:
      (A) LENGTH 18 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:26
(28) INFORMATION FOR SEQ ID NO:27
   (i) SEQUENCE CHARACTERISTICS:
      (A) LENGTH 12 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:27
(29) INFORMATION FOR SEQ ID NO:28
   (i) SEQUENCE CHARACTERISTICS:
      (A) LENGTH 11 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:28
(30) INFORMATION FOR SEQ ID NO:29
   (i) SEQUENCE CHARACTERISTICS:
      (A) LENGTH 14 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:29
(31) INFORMATION FOR SEQ ID NO:30
   (i) SEQUENCE CHARACTERISTICS:
      (A) LENGTH 11 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:30
(32) INFORMATION FOR SEQ ID NO:31
   (i) SEQUENCE CHARACTERISTICS:
      (A) LENGTH 10 amino acids
      (B) TYPE: Amino acid
      (D) TOPOLOGY: Linear
   (ii) MOLECULAR TYPE: Peptide
   (iii) HYPOTHETICAL: Yes
   (iv) FRAGMENT TYPE: N-terminal
   (v) SEQUENCE DESCRIPTION: SEQ ID NO:31
(33) INFORMATION FOR SEQ ID NO:32
   (i) SEQUENCE CHARACTERISTICS:
      (A) LENGTH 4 amino acids
      (B) TYPE: Amino acid
      (D) TOPOLOGY: Linear
   (ii) MOLECULAR TYPE: Peptide
   (iii) HYPOTHETICAL: Yes
   (iv) FRAGMENT TYPE: N-terminal
   (v) SEQUENCE DESCRIPTION: SEQ ID NO:32
(34) INFORMATION FOR SEQ ID NO:33
   (i) SEQUENCE CHARACTERISTICS:
      (A) LENGTH 11 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:33
(35) INFORMATION FOR SEQ ID NO:34
   (i) SEQUENCE CHARACTERISTICS:
      (A) LENGTH 11 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:34
(36) INFORMATION FOR SEQ ID NO:35
   (i) SEQUENCE CHARACTERISTICS:
      (A) LENGTH 6 amino acids .
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:35
(37) INFORMATION FOR SEQ ID NO:36
   (i) SEQUENCE CHARACTERISTICS:
      (A) LENGTH 6 amino acids
      (B) TYPE: Amino acid
      (D) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION: SEQ ID NO:36
(38) INFORMATION FOR SEQ ID NO:37
   (i) SEQUENCE CHARACTERISTICS:
      (A) LENGTH 4 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:37
(39) INFORMATION FOR SEQ ID NO:38
   (i) SEQUENCE CHARACTERISTICS:
      (A) LENGTH 12 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:38
(40) INFORMATION FOR SEQ ID NO:39
   (i) SEQUENCE CHARACTERISTICS:
      (A) LENGTH 9 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:39
(41) INFORMATION FOR SEQ ID NO:40
   (i) SEQUENCE CHARACTERISTICS:
      (A) LENGTH 10 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:40
(42) INFORMATION FOR SEQ ID NO:41
   (i) SEQUENCE CHARACTERISTICS:
      (A) LENGTH 7 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:41
(43) INFORMATION FOR SEQ ID NO:42
   (i) SEQUENCE CHARACTERISTICS:
      (A) LENGTH 12 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:42
(44) INFORMATION FOR SEQ ID NO:43
   (i) SEQUENCE CHARACTERISTICS:
      (A) LENGTH 10 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:43
(45) INFORMATION FOR SEQ ID NO:44
   (i) SEQUENCE CHARACTERISTICS:
      (A) LENGTH 8 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:44
(46) INFORMATION FOR SEQ ID NO:45
   (i) SEQUENCE CHARACTERISTICS:
      (A) LENGTH 14 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:45
(47) INFORMATION FOR SEQ ID NO:46
   (i) SEQUENCE CHARACTERISTICS:
      (A) LENGTH 11 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:46
(48) INFORMATION FOR SEQ ID NO:47
   (i) SEQUENCE CHARACTERISTICS:
      (A) LENGTH 15 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:47
(49) INFORMATION FOR SEQ ID NO:48
   (i) SEQUENCE CHARACTERISTICS:
      (A) LENGTH 14 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:48
(50) INFORMATION FOR SEQ ID NO:49
   (i) SEQUENCE CHARACTERISTICS:
      (A) LENGTH 11 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:49
(51) INFORMATION FOR SEQ ID NO:50
   (i) SEQUENCE CHARACTERISTICS:
      (A) LENGTH 11 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:50
(52) INFORMATION FOR SEQ ID NO:51
   (i) SEQUENCE CHARACTERISTICS:
      (A) LENGTH 11 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:51
(53) INFORMATION FOR SEQ ID NO:52
   (i) SEQUENCE CHARACTERISTICS:
      (A) LENGTH 10 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:52
(54) INFORMATION FOR SEQ ID NO:53
   (i) SEQUENCE CHARACTERISTICS:
      (A) LENGTH 15 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:53
(55) INFORMATION FOR SEQ ID NO:54
   (i) SEQUENCE CHARACTERISTICS:
      (A) LENGTH 14 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:54
(56) INFORMATION FOR SEQ ID NO:55
   (i) SEQUENCE CHARACTERISTICS:
      (A) LENGTH 7 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:55
(57) INFORMATION FOR SEQ ID NO:56
   (i) SEQUENCE CHARACTERISTICS:
      (A) LENGTH 23 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:56
(58) INFORMATION FOR SEQ ID NO:57
   (i) SEQUENCE CHARACTERISTICS:
      (A) LENGTH 18 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:57
(59) INFORMATION FOR SEQ ID NO:58
   (i) SEQUENCE CHARACTERISTICS:
      (A) LENGTH 19 amino acids
      (B) TYPE: Amino acid
      (C) TOPOLOGY: Linear
   (ii) MOLECULAR TYPE: Peptides
   (iii) HYPOTHETICAL: No
   (iv) FRAGMENT TYPE: N-terminal
   (v) SEQUENCE DESCRIPTION FOR SEQ ID NO:58
(60) INFORMATION FOR SEQ ID NO:59
   (i) SEQUENCE CHARACTERISTICS:
      (A) LENGTH 20 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:59
(61) INFORMATION FOR SEQ ID NO:60
   (i) SEQUENCE CHARACTERISTICS:
      (A) LENGTH 15 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:60
(62) INFORMATION FOR SEQ ID NO:61
   (i) SEQUENCE CHARACTERISTICS:
      (A) LENGTH 6 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:61
(63) INFORMATION FOR SEQ ID NO:62
   (i) SEQUENCE CHARACTERISTICS:
      (A) LENGTH 15 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:62
(64) INFORMATION FOR SEQ ID NO:63
   (i) SEQUENCE CHARACTERISTICS:
      (A) LENGTH 7 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:63
(65) INFORMATION FOR SEQ ID NO:64
   (i) SEQUENCE CHARACTERISTICS:
      (A) LENGTH 7 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:64
(66) INFORMATION FOR SEQ ID NO:65
   (i) SEQUENCE CHARACTERISTICS:
      (A) LENGTH 8 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:65
(67) INFORMATION FOR SEQ ID NO:66
   (i) SEQUENCE CHARACTERISTICS:
      (A) LENGTH 7 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:66
(68) INFORMATION FOR SEQ ID NO:67
   (i) SEQUENCE CHARACTERISTICS:
      (A) LENGTH 12 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:67
(69) INFORMATION FOR SEQ ID NO:68
   (i) SEQUENCE CHARACTERISTICS:
      (A) LENGTH 9 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:68
(70) INFORMATION FOR SEQ ID NO:69
   (i) SEQUENCE CHARACTERISTICS:
      (A) LENGTH 6 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:69
(71) INFORMATION FOR SEQ ID NO:70
   (i) SEQUENCE CHARACTERISTICS:
      (A) LENGTH 16 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:70
(72) INFORMATION FOR SEQ ID NO:71
   (i) SEQUENCE CHARACTERISTICS:
      (A) LENGTH 6 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:71
(73) INFORMATION FOR SEQ ID NO:72
   (i) SEQUENCE CHARACTERISTICS:
      (A) LENGTH 6 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:72
(74) INFORMATION FOR SEQ ID NO:73
   (i) SEQUENCE CHARACTERISTICS:
      (A) LENGTH 11 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:73
(75) INFORMATION FOR SEQ ID NO:74
   (i) SEQUENCE CHARACTERISTICS:
      (A) LENGTH 8 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:74
(76) INFORMATION FOR SEQ ID NO:75
   (i) SEQUENCE CHARACTERISTICS:
      (A) LENGTH 12 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:75
(77) INFORMATION FOR SEQ ID NO:76
   (i) SEQUENCE CHARACTERISTICS:
      (A) LENGTH 6
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:76
(78) INFORMATION FOR SEQ ID NO:77
   (i) SEQUENCE CHARACTERISTICS:
      (A) LENGTH 11 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:77
(79) INFORMATION FOR SEQ ID NO:78
   (i) SEQUENCE CHARACTERISTICS:
      (A) LENGTH 8 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:78
(80) INFORMATION FOR SEQ ID NO:79
   (i) SEQUENCE CHARACTERISTICS:
      (A) LENGTH 16 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:79
(81) INFORMATION FOR SEQ ID NO:80
   (i) SEQUENCE CHARACTERISTICS:
      (A) LENGTH 9 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:80
(82) INFORMATION FOR SEQ ID NO:81
   (i) SEQUENCE CHARACTERISTICS:
      (A) LENGTH 7 amino acids
      (B) TYPE: Amino acid
      (C) TOPOLOGY: Linear
   (ii) MOLECULAR TYPE: Peptide
   (iii) HYPOTHETICAL: No
   (iv) FRAGMENT TYPE: N-terminal
   (v) SEQUENCE DESCRIPTION FOR SEQ ID NO:81
(83) INFORMATION FOR SEQ D NO:82
   (i) SEQUENCE CHARACTERISTICS:
      (A) LENGTH 1098 amino acids
      (B) TYPE: Amino acid
      (C) TOPOLOGY: Linear
   (ii) MOLECULAR TYPE: Polypeptide
   (iii) HYPOTHETICAL: Yes
   (v) SEQUENCE DESCRIPTION FOR SEQ ID NO:82
(84) INFORMATION FOR SEQ ID NO:83
   (i) SEQUENCE CHARACTERISTICS
      (A) LENGTH 5981 basepairs
      (B) TYPE: Nucleotide
      (C) STRANDEDNESS: Doublestranded
      (D) TOPOLOGY: Linear
   (ii) MOLECULAR TYPE: Genomic DNA
   (iii) HYPOTHETICAL: No
   (iv) ANTI-SENSE: No
   (vi) ORIGINAL SOURCE:
      (A) ORGANISM: Saccharomyces cerevisiae
      (B) STRAIN: S288C
      (C) HAPLOTYPE: Haploid
   (vii) IMMEDIATE SOURCE:
      (A) LIBRARY: Genomic
      (B)CLONES: 6 and 10
   (viii) POSITION IN GENOME
      (A)CHROMOSOME: 13
   (xi) SEQUENCE DESCRIPTION: SEQ ID NO:83
(85) INFORMATION FOR SEQ ID NO:84
   (i) SEQUENCE CHARACTERISTICS
      (A) LENGTH50 base pairs
      (B) TYPE: Nucleotide
      (C)STRANDEDNESS: Doublestranded
      (D)TOPOLOGY: Linear
   (ii) MOLECULAR TYPE: Synthetic DNA
   (iii) HYPOTHETICAL: No
   (iv) ANTISENSE: No
   (xi) SEQUENCE DESCRIPTION: SEQ ID NO:84
(86) INFORMATION FOR SEQ ID NO:85
   (i) SEQUENCE CHARACTERISTICS
      (A) LENGTH48 basepairs
      (B) TYPE: Nucleotide
      (C) STRANDEDNESS: Doublestranded
      (D) TOPOLOGY: Linear
   (ii) MOLECULAR TYPE: Synthetic DNA
   (iii) HYPOTHETICAL: No
   (iv) ANTISENSE: No
   (xi) SEQUENCE DESCRIPTION: SEQ ID NO:85

## Claims

1. A cell or non-human organism having a capacity to produce trehalose provided by heterologous DNA encoding for expression of a yeast trehalose synthase complex sub-unit polypeptide or polypeptides
**characterised in that** the heterologous DNA encodes for a functional sub-unit polypeptide involved in trehalose-6-phosphate synthase catalytic activity, that being the ability to convert uridine diphosphoglucose (UDPG) and glucose-6-phosphate (G6P) to trehalose-6-phosphate, and is selected from
(i) the about 57kDa sub-unit polypeptide exhibiting trehalose-6-phosphate synthase activity, and
(ii) the about 123kDa trehalose synthase regulatory sub-unit polypeptide or an N-terminally truncated form of the 123kDa sub-unit lacking up to 600 amino acids from its N-terminal, which is capable of altering the sensitivity of the yeast trehalose synthase trehalose-6-phosphate synthase activity to physiological phosphate and fructose-6-phosphate (F6P).

2. A cell or non-human organism as claimed in Claim 1 **characterised in that** the 123kDa trehalose synthase regulatory sub-unit includes an amino acid sequence having 37% amino acid identity to the 57kDa sub-unit and is degradable to products having trehalose-6-phosphate synthase activity.

3. A cell or non-human organism as claimed in Claim 1 or Claim 2 **characterised in that** the sub-unit polypeptide or polypeptides consist of one or both of the trehalose synthase complex sub-unit polypeptides
(i) an about 57kDa sub-unit comprising SEQ ID No 2 exhibiting trehalose-6-phosphate synthase activity and functionally equivalent mutations thereof and
(ii) an about 123kDa trehalose synthase regulatory polypeptide or or an N-terminally truncated form thereof lacking up to 600 amino acids from its N-terminal, the polypeptide or truncate comprising SEQ ID No 82, or a functionally equivalent mutation thereof, and being capable of altering the sensitivity of the yeast trehalose synthase trehalose-6-phosphate synthase activity to physiological phosphate and F6P.

4. A cell or non-human organism as claimed in any one of Claims 1 to 3 **characterised in that** the heterologous DNA, together with native cell trehalase activity, provides it with a trehalose futile cycle capability whereby trehalose formed is converted to glucose by trehalase with consumption of ATP.

5. A cell or non-human organism as claimed in any one of Claims 1 to 4 **characterised in that** the heterologous DNA, together with native cell trehalase activity, provides it with an increased trehalose futile cycle capability whereby trehalose formed is converted to glucose by trehalase with increased consumption of ATP.

6. A cell or non-human organism as claimed in any one of Claims 1 to 5 **characterised in that** it further comprises a second heterologous DNA encoding for expression of an about 99kDa polypeptide sub-unit of yeast trehalose synthase complex comprising SEQ ID No 29 to 38 and 44 to 49 which exhibits trehalose-6-phosphate phosphatase activity, and functionally equivalent mutations thereof.

7. A cell or non-human organism as claimed in any one of Claims 1 to 6 **characterised in that** it comprises DNA of the open reading frame of SEQ ID No 1 or functionally equivalent mutations thereof.

8. A cell or non-human organism as claimed in any one of Claims 1 to 7 **characterised in that** it comprises DNA of the open reading frame of SEQ ID No 3 or functionally equivalent mutations thereof.

9. A cell or non-human organism as claimed in Claim 8 **characterised in that** the DNA comprises the open reading frame of SEQ ID No 83 or functionally equivalent mutations thereof.

10. A cell or non-human organism as claimed in any one of Claims 1 to 9 **characterised in that** the truncated form of the 123 kDa long chain lacks up to 330 amino acids from the N-terminal end.

11. A cell or non-human organism as claimed in Claim 10 **characterised in that** it has a trehalose-6-phosphate synthase activity that is less inhibited by phosphate than is native yeast trehalose synthase.

12. A cell or non-human organism as claimed in any one of Claims 1 to 11 **characterised in that** it is more resistant to water deprivation, heat and cold than are the corresponding cells or non-human organisms lacking the selected heterologous DNA having the trehalose-6-phosphate synthase activity, grown under the same conditions.

13. A cell or non-human organism as claimed in any one of the preceding claims **characterised in that** it is a yeast, fungi, bacteria or plant cell or non-human organism.

14. A cell or non-human organism as claimed in Claim 13 **characterised in that** it is a higher plant cell or non-human organism.

15. An edible tissue of a plant **characterised in that** it includes a cell as claimed in Claim 13 or 14.

16. A process for producing ethanol **characterised in that** it comprises fermentation of carbohydrate using a yeast cell as claimed in Claim 13.

17. A process of Claim 16 **characterised in that** the heterologous DNA is functionally combined with a promoter active under fermentative conditions.

18. A process for producing trehalose by cultivating a cell or plant as claimed in any one of Claims 1 to 15.

19. A process for producing trehalose-enriched food products from plants comprising cultivating non-human organism as claimed in Claim 13 or 14 **characterised in that** the organism is a plant and wherein trehalose is synthesized in the edible tissues of the plant.

20. A process for producing a transformed cell or transformed non-human organism having increased capacity to produce trehalose compared to the nontransformed cell or non-human organism, comprising transforming the cell by introducing into it DNA encoding for expression of a yeast trehalose synthase complex sub-unit
**characterised in that** the DNA encodes for a functional sub-unit involved in trehalose-6-phosphate synthase catalytic activity, that being the ability to convert uridine diphosphoglucose (UDPG) and glucose-6-phosphate (G6P) to trehalose-6-phosphate, selected from
(i) the about 57kDa yeast sub-unit exhibiting trehalose-6-phosphate synthase activity, and
(ii) the about 123kDa yeast sub-unit trehalose synthase regulatory polypeptide or an N-terminally truncated form thereof, the polypeptide or truncated form being capable of altering the sensitivity of yeast trehalose synthase trehalose-6-phosphate synthase activity to physiological phosphate and fructose-6-phosphate (F6P).

21. A process as claimed in Claim 20 **characterised in that**
(i) the about 57KDa sub-unit comprises SEQ ID No 2 or a functionally equivalent mutation thereof exhibiting trehalose-6-phosphate synthase catalytic activity and
(ii) the about 123kDa sub-unit comprises SEQ ID No 82, or a functionally equivalent mutation thereof or an N-terminally truncated form thereof, which is capable of altering the sensitivity of the 57kDa sub-unit trehalose-6-phosphate synthase activity to physiological phosphate and F6P.

22. A process as claimed in Claim 21 **characterised in that** it further introduces a DNA encoding for an about 99kDa sub-unit comprising SEQ ID No 29 to 38 and 44 to 49 exhibiting trehalose-6-phosphate phosphatase activity or a functionally equivalent mutation thereof

23. A process as claimed in any one of Claims 20 to 22 **characterised in that** the DNA is introduced into the cell directly into a chromosome or in an expression vector.

24. A process as claimed in Claim 23 **characterised in that** the DNA is introduced in to a chromosome of the cell in the form of isolated or purified DNA comprising a nucleotide sequence encoding for the polypeptide sub-unit.

25. A process as claimed in Claim 23 **characterised in that** the expression vector is produced using isolated or purified DNA comprising a nucleotide sequence encoding for the polypeptide sub-unit.

26. A polynucleotide suitable for use in a process as claimed in any one of Claims 20 to 25 **characterised in that** it is isolated or purified DNA encoding the functional yeast trehalose synthase 57kDa sub-unit exhibiting trehalose-6-phosphate synthase activity.

27. A polynucleotide as claimed in Claim 26 **characterised in that** it comprises SEQ ID No 1 or functionally equivalent mutations thereof.

28. A polynucleotide suitable for use in a process as claimed in any one of Claims 20 to 25 **characterised in that** it is isolated or purified DNA encoding the long chain of about 123 kDa of trehalose synthase regulatory sub-unit including an amino acid sequence having 37% amino acid identity to the 57kDa sub-unit, and which is capable of altering the sensitivity of the yeast trehalose synthase trehalose-6-phosphate synthase activity to physiological phosphate and F6P.

29. A polynucleotide as claimed in Claim 28 **characterised in that** it comprises the polynucleotide sequence of SEQ ID No 83 or a functionally equivalent mutation thereof.

30. A polynucleotide suitable for use in a process as claimed in any one of Claims 20 to 25 **characterised in that** it is an isolated or purified DNA encoding a truncated long chain of about 123 kDa of trehalose synthase, which comprises the amino acid sequence of SEQ ID No 4 or a functionally equivalent mutation thereof.

31. A polynucleotide as claimed in Claim 30 **characterised in that** it comprises a polynucleotide sequence of SEQ ID No 3 or functionally equivalent mutations thereof.

32. A polynucleotide suitable for use in a process as claimed in any one of Claims 20 to 25 **characterised in that** it encodes a truncated form of the 123 kDa long chain of trehalose synthase of SEQ ID No 82 lacking up to 600 amino acids from the N-terminal end **characterised in that** the polynucleotide has the ability to alter the sensitivity of the yeast trehalose synthase trehalose-6-phosphate synthase activity to physiological phosphate and fructose-6-phosphate (F6P) such that when associated with the 57kDA unit the trehalose-6-phosphate synthase activity is less inhibited by phosphate than is the association of the 57kDa and 123kDa units.

33. An isolated or purified DNA as claimed in Claim 32 encoding a truncated form of the 123 kDa long chain of trehalose synthase of SEQ ID No 82 lacking up to 330 amino acids from the N-terminal end.

34. A polynucleotide suitable for use in a process as claimed in any one of Claims 20 to 25 **characterised in that** it is an isolated or purified DNA encoding the long chain of about 99 kDa of trehalose synthase, which comprises the amino acid sequences of SEQ ID Nos: 29 to 38 and 44 to 49 or a functionally equivalent mutation thereof.

35. A polynucleotide as claimed in any one of Claims 26 to 34, **characterised in that** the functionally equivalent mutation comprises codons changed degeneratively such as to increase G+C content

36. An expression vector comprising at least one or two of the DNA sequences of Claims 26 to 34

37. A vector as claimed in Claim 36 **characterised in that** it further includes a promoter that is capable of promoting expression of the DNA in a higher plant.

38. A transformed yeast cell having an increased capacity to produce trehalose compared to an untransformed yeast cell, wherein said increased capacity is provided by transformation with a DNA encoding for expression of a yeast trehalose synthase complex sub-unit polypeptide or polypeptides
**characterised in that** the DNA encodes for a functional sub-unit polypeptide involved in trehalose-6-phosphate synthase catalytic activity, that being the ability to convert uridine diphosphoglucose (UDPG) and glucose-6-phosphate (G6P) to trehalose-6-phosphate, and is selected from
(i) the about 57kDa sub-unit polypeptide exhibiting trehalose-6-phosphate synthase activity, and
(ii) the about 123kDa trehalose synthase regulatory sub-unit polypeptide or an N-terminally truncated form of the 123kDa sub-unit lacking up to 600 amino acids from its N-terminal, which is capable of altering the sensitivity of the yeast trehalose synthase trehalose-6-phosphate synthase activity to physiological phosphate and fructose-6-phosphate (F6P).

39. A process for producing a transformed yeast cell having increased capacity to produce trehalose compared to a nontransformed yeast cell; comprising transforming the yeast cell by introducing into it DNA encoding for expression of a yeast trehalose synthase complex sub-unit
**characterised in that** the DNA encodes for a functional sub-unit involved in trehalose-6-phosphate synthase catalytic activity, that being the ability to convert uridine diphosphoglucose (UDPG) and glucose-6-phosphate (G6P) to trehalose-6-phosphate, selected from
(i) the about 57kDa yeast sub-unit exhibiting trehalose-6-phosphate synthase activity, and
(ii) the about 123kDa yeast sub-unit trehalose synthase regulatory polypeptide or an N-terminally truncated form thereof, the polypeptide or truncated form being capable of altering the sensitivity of yeast trehalose synthase trehalpse-6-phosphate synthase activity to physiological phosphate and fructose-6-phosphate (F6P).

## Patentansprüche

1. Zelle oder nicht menschlicher Organismus mit der Fähigkeit, Trehalose zu produzieren, bereitgestellt durch heterologe DNA, die die Expression von (einem) Hefe-Trehalosesynthasekomplex-Unterheitspolypeptid oder - polypeptiden kodiert,
**dadurch gekennzeichnet, dass** die heterologe DNA ein funktionelles Untereinheitspolypeptid kodiert, das in Trehalose-6-Phosphat-Synthasekatalyseaktivität involviert ist, wobei dies das Vermögen ist, Uridin-Diphosphoglucose (UDPG) und Glucose-6-Phosphat (G6P) in Trehalose-6-Phosphat umzuwandeln, und ausgewählt ist aus
(i) dem etwa 57 kDa Untereinheitspolypeptid mit Trehalose-6-Phosphat-Synthaseaktivität, und
(ii) dem etwa 123 kDa Trehalosesynthase-Regulationsuntereinheitspolypeptid oder einer N-terminal verkürzten Form der 123 kDa Untereinheit, der bis zu 600 Aminosäuren von ihrem N-Terminal fehlen, das/die die Empfindlichkeit der Hefe-Trehalosesynthase-Trehalose-6-Phosphat-Synthaseaktivität gegenüber physiologischem Phosphat und Fructose-6-Phosphat (F6P) verändern kann.

2. Zelle oder nicht menschlicher Organismus nach Anspruch 1, **dadurch gekennzeichnet, dass** die 123 kDa Trehalosesynthase-Regulationsuntereinheit eine Aminosäuresequenz mit 37 % Aminosäureidentität zur 57 kDa Unterheit einschließt und zu Produkten mit Trehalose-6-Phosphat-Synthaseaktivität abbaubar ist.

3. Zelle oder nicht menschlicher Organismus nach Anspruch 1 oder Anspruch 2,
**dadurch gekennzeichnet, dass** das/die Untereinheitspolypeptid oder -polypeptide aus einem oder beiden Trehalosesynthasekomplex-Untereinheitspolypeptiden besteht, wobei
(i) eine etwa 57 kDa Untereinheit SEQ ID Nr. 2 mit Trehalose-6-Phosphat-Synthaseaktivität und funktionell äquivalente Mutationen davon umfasst, und
(ii) einem etwa 123 kDa Trehalosesynthase-Regulationspolypeptid oder einer N-terminal verkürzten Form davon bis zu 600 Aminosäuren von ihrem N-Terminal fehlen, wobei das Polypeptid oder die Verkürzung SEQ ID Nr. 82 oder eine funktionell äquivalente Mutation davon umfasst und die Empfindlichkeit der Hefe-Trehalosesynthase-Trehalose-6-Phosphat-Synthaseaktivität gegenüber physiologischem Phosphat und F6P verändern kann.

4. Zelle oder nicht menschlicher Organismus nach einem der Ansprüche 1 bis 3,
**dadurch gekennzeichnet, dass** die heterologe DNA ihr/ihm, zusammen mit nativer Zelltrehalaseaktivität, eine Trehalose-Leerlaufzyklusfähigkeit gibt, wobei ausgebildete Trehalose durch Trehalase mit dem Verbrauch von ATP in Glucose umgewandelt wird.

5. Zelle oder nicht menschlicher Organismus nach einem der Ansprüche 1 bis 4,
**dadurch gekennzeichnet, dass** die heterologe DNA ihr/ihm, zusammen mit nativer Zelltrehalaseaktivität, eine erhöhte Trehalose-Leerlaufzyklusfähigkeit gibt, wobei ausgebildete Trehalose durch Trehalase mit erhöhtem Verbrauch von ATP in Glucose umgewandelt wird.

6. Zelle oder nicht menschlicher Organismus nach einem der Ansprüche 1 bis 5,
**dadurch gekennzeichnet, dass** sie/er ferner eine zweite heterologe DNA umfasst, die die Expression einer etwa 99 kDa Polypeptiduntereinheit eines Hefe-Trehalosesynthasekomplexes kodiert, umfassend SEQ ID Nr. 29 bis 38 und 44 bis 49 mit Trehalose-6-Phosphatphosphataseaktivität und funktionell äquivalente Mutationen davon.

7. Zelle oder nicht menschlicher Organismus nach einem der Ansprüche 1 bis 6,
**dadurch gekennzeichnet, dass** sie/er DNA des offenen Leserasters der SEQ ID Nr. 1 oder funktionell äquivalenter Mutationen davon umfasst.

8. Zelle oder nicht menschlicher Organismus nach einem der Ansprüche 1 bis 7,
**dadurch gekennzeichnet, dass** sie/er DNA des offenen Leserasters der SEQ ID Nr. 3 oder funktionell äquivalenter Mutationen davon umfasst.

9. Zelle oder nicht menschlicher Organismus nach Anspruch 8, **dadurch gekennzeichnet, dass** die DNA das offene Leseraster der SEQ ID Nr. 83 oder funktionell äquivalenter Mutationen davon umfasst.

10. Zelle oder nicht menschlicher Organismus nach einem der Ansprüche 1 bis 9,
**dadurch gekennzeichnet, dass** der verkürzten Form der 123 kDA langen Kette bis zu 330 Aminosäuren vom N-terminalen Ende fehlen.

11. Zelle oder nicht menschlicher Organismus nach Anspruch 10, **dadurch gekennzeichnet, dass** sie/er eine Trehalose-6-Phosphat-Synthaseaktivität hat, die weniger durch Phosphat inhibiert wird als native Hefe-Trehalosesynthase.

12. Zelle oder nicht menschlicher Organismus nach einem der Ansprüche 1 bis 11,
**dadurch gekennzeichnet, dass** sie/er resistenter gegenüber Wasserentzug, Wärme und Kälte ist als die entsprechenden Zellen oder nicht menschlichen Organismen, denen die ausgewählte heterologe DNA mit Trehalose-6-Phosphat-Synthaseaktivität fehlt, die unter den gleichen Bedingungen wachsen.

13. Zelle oder nicht menschlicher Organismus nach einem der vorherigen Ansprüche, **dadurch gekennzeichnet, dass** sie/er Hefe, ein Pilz, Bakterium oder eine Pflanzenzelle oder ein nicht menschlicher Organismus ist.

14. Zelle oder nicht menschlicher Organismus nach Anspruch 13, **dadurch gekennzeichnet, dass** sie/er eine höhere Pflanzenzelle oder ein nicht menschlicher Organismus ist.

15. Essbares Gewebe einer Pflanze, **dadurch gekennzeichnet, dass** es eine Zelle nach Anspruch 13 oder 14 beinhaltet.

16. Verfahren zur Herstellung von Ethanol, **dadurch gekennzeichnet, dass** es die Fermentation von Kohlenhydrat unter Verwendung einer Hefezelle nach Anspruch 13 umfasst.

17. Verfahren nach Anspruch 16, **dadurch gekennzeichnet, dass** die heterologe DNA mit einem Promotor funktionell verbunden ist, der unter fermentativen Bedingungen aktiv ist.

18. Verfahren zur Herstellung von Trehalose durch Kultivieren einer Zelle oder Pflanze nach einem der Ansprüche 1 bis 15.

19. Verfahren zur Herstellung von mit Trehalose angereicherten Lebensmittelprodukten von Pflanzen, umfassend das Kultivieren eines nicht menschlichen Organismus nach Anspruch 13 oder 14, **dadurch gekennzeichnet, dass** der Organismus eine Pflanze ist, wobei Trehalose in den essbaren Pflanzengeweben synthetisiert wird.

20. Verfahren zur Herstellung einer transformierten Zelle oder eines transformierten nicht menschlichen Organismus mit erhöhter Fähigkeit zur Erzeugung von Trehalose im Vergleich zur nicht transformierten Zelle oder zum nicht transformierten nicht menschlichen Organismus, umfassend das Transformieren der Zelle, indem DNA in sie eingeführt wird, die die Expression einer Hefe-Trehalosesynthasekomplexuntereinheit kodiert,
**dadurch gekennzeichnet, dass** die DNA eine funktionelle Untereinheit kodiert, die in Trehalose-6-Phosphat-Synthasekatalyseaktivität involviert ist, wobei dies das Vermögen zur Umwandlung von Uridin-Diphosphoglucose (UDPG) und Glucose-6-Phosphat (G6F) in Trehalose-6-Phosphat ist, ausgewählt aus
(i) der etwa 57 kDa Hefeuntereinheit mit Trehalose-6-Phosphat-Synthaseaktivität und
(ii) dem etwa 123 kDa Hefe-Untereinheits-Trehalosesynthase-Regulationspolypeptid oder einer N-terminal verkürzten Form davon, wobei das Polypeptid oder die verkürzte Form die Empfindlichkeit von Hefe-Trehalosesynthase-Trehalose-6-Phosphat-Synthaseaktivität gegenüber physiologischem Phosphat und Fructose-6-Phosphat (F6P) verändern kann.

21. Verfahren nach Anspruch 20, **dadurch gekennzeichnet, dass**
(i) die etwa 57 kDa Untereinheit SEQ ID Nr. 2 oder eine funktionell äquivalente Mutation davon mit Trehalose-6-Phosphat-Synthasekatalyseaktivität umfasst und
(ii) die etwa 123 kDa Untereinheit SEQ ID Nr. 82 oder eine funktionell äquivalente Mutation davon, oder eine N-terminal verkürzte Form davon umfasst, die die Empfindlichkeit der 57 kDa Untereinheit-Trehalose-6-Phosphat-Synthaseaktivität gegenüber physiologischem Phosphat und F6P verändern kann.

22. Verfahren nach Anspruch 21, **dadurch gekennzeichnet, dass** es ferner eine DNA einführt, die eine etwa 99 kDa Untereinheit kodiert, umfassend SEQ ID Nr. 29 bis 38 und 44 bis 49 mit Trehalose-6-Phosphat-Phosphataseaktivität oder eine funktionell äquivalente Mutation davon.

23. Verfahren nach einem der Ansprüche 20 bis 22, **dadurch gekennzeichnet, dass** die DNA in der Zelle direkt in ein Chromosom oder in einen Expressionsvektor eingeführt wird.

24. Verfahren nach Anspruch 23, **dadurch gekennzeichnet, dass** die DNA in ein Chromosom der Zelle in der Form isolierter oder gereinigter DNA eingeführt wird, die eine Nucleotidsequenz umfasst, die die Polypeptiduntereinheit kodiert.

25. Verfahren nach Anspruch 23, **dadurch gekennzeichnet, dass** der Expressionsvektor mit isolierter oder gereinigter DNA erzeugt wird, die eine Nucleotidsequenz umfasst, die die Polypeptiduntereinheit kodiert.

26. Polynucleotid, das zur Verwendung in einem Verfahren nach einem der Ansprüche 20 bis 25 geeignet ist, **dadurch gekennzeichnet, dass** es isolierte oder gereinigte DNA ist, die die funktionelle Hefe-Trehalosesynthase-57kDa-Untereinheit mit Trehalose-6-Phosphat-Synthaseaktivität kodiert.

27. Polynucleotid nach Anspruch 26, **dadurch gekennzeichnet, dass** es SEQ ID Nr. 1 oder funktionell äquivalente Mutationen davon umfasst.

28. Polynucleotid, das zur Verwendung in einem Verfahren nach einem der Ansprüche 20 bis 25 geeignet ist, **dadurch gekennzeichnet, dass** es isolierte oder gereinigte DNA ist, die die lange Kette von etwa 123 kDa der Trehalosesynthase-Regulationsuntereinheit kodiert, einschließlich einer Aminosäuresequenz mit 37 % Aminosäureidentität zur 57 kDa Untereinheit, und das die Empfindlichkeit der Hefe-Trehalosesynthase-Trehalose-6-Phosphat-Synthaseaktivität gegenüber physiologischem Phosphat und F6P verändern kann.

29. Polynucleotid nach Anspruch 28, **dadurch gekennzeichnet, dass** es die Polynucleotidsequenz von SEQ ID Nr. 83 oder eine funktionell äquivalente Mutation davon umfasst.

30. Polynucleotid, das zur Verwendung in einem Verfahren nach einem der Ansprüche 20 bis 25 geeignet ist, **dadurch gekennzeichnet, dass** es eine isolierte oder gereinigte DNA ist, die eine verkürzte lange Kette von etwa 123 kDa von Trehalosesynthase kodiert, umfassend die Aminosäuresequenz von SEQ ID Nr. 4 oder eine funktionell äquivalente Mutation davon.

31. Polynucleotid nach Anspruch 30, **dadurch gekennzeichnet, dass** es eine Polynucleotidsequenz der SEQ ID Nr. 3 von funktionell äquivalenten Mutationen davon umfasst.

32. Polynucleotid, das zur Verwendung in einem Verfahren nach einem der Ansprüche 20 bis 25 geeignet ist, **dadurch gekennzeichnet, dass** es eine verkürzte Form der 123 kDa langen Kette von Trehalosesynthase der SEQ ID Nr. 82 kodiert, der bis zu 600 Aminosäuren vom N-terminalen Ende fehlen,
**dadurch gekennzeichnet, dass** das Polynucleotid die Empfindlichkeit der Hefe-Trehalosesynthase-Trehalose-6-Phosphat-Synthaseaktivität gegenüber physiologischem Phosphat und Fructose-6-Phosphat (F6P) so verändern kann, dass, in Verbindung mit der 57 kDa Einheit, die Trehalose-6-Phosphat-Synthaseaktivität durch Phosphat weniger inhibiert wird als die Verbindung der 57 kDa und 123 kDa Einheit.

33. Isolierte oder gereinigte DNA nach Anspruch 32, die eine verkürzte Form der 123 kDa langen Kette der Trehalosesynthase der SEQ ID Nr. 82 kodiert, der bis zu 330 Aminosäuren vom N-terminalen Ende fehlen.

34. Polynucleotid, das für die Verwendung in einem Verfahren nach einem der Ansprüche 20 bis 25 geeignet ist, **dadurch gekennzeichnet**, das es eine isolierte oder gereinigte DNA ist, die die lange Kette von etwa 99 kDa von Trehalosesynthase kodiert, umfassend die Aminosäuresequenzen der SEQ ID Nr. 29 bis 38 und 44 bis 49 oder eine funktionell äquivalente Mutation davon.

35. Polynucleotid nach einem der Ansprüche 26 bis 34, **dadurch gekennzeichnet, dass** die funktionell äquivalente Mutation degenerativ veränderte Codone umfasst, um beispielsweise den G+C-Gehalt zu erhöhen.

36. Expressionsvektor, umfassend wenigstens eine oder zwei der DNA-Sequenzen der Ansprüche 26 bis 34.

37. Vektor nach Anspruch 36, **dadurch gekennzeichnet, dass** er ferner einen Promotor beinhaltet, der die Expression der DNA in einer höheren Pflanze fördern kann.

38. Transformierte Hefezelle mit erhöhtem Vermögen zur Erzeugung von Trehalose im Vergleich zu einer untransformierten Hefezelle, wobei das genannte erhöhte Vermögen durch Transformation mit einer DNA erzielt wird, die die Expression von (einem) Hefe-Trehalosesynthasekomplex-Untereinheitspolypeptid oder - polypeptiden kodiert,
**dadurch gekennzeichnet, dass** die DNA ein funktionelles Untereinheitspolypeptid kodiert, das an Trehalose-6-Phosphat-Synthasekatalyseaktivität beteiligt ist, wobei dies das Vermögen ist, Uridin-Diphosphoglucose (UDPG) und Glucose-6-Phosphat (G6P) in Trehalose-6-Phosphat umzuwandeln, und ausgewählt ist aus
(i) dem etwa 57 kDa Untereinheitspolypeptid mit Trehalose-6-Phosphat-Synthaseaktivität und
(ii) dem etwa 123 kDa Trehalosesynthase-Regulationsuntereinheitspolypeptid oder einer N-terminal verkürzten Form der 123 kDa Untereinheit, der bis zu 600 Aminosäuren von ihrem N-Terminal fehlen, das/die die Empfindlichkeit der Hefe-Trehalosesynthase-Trehalose-6-Phosphat-Synthaseaktivität gegenüber physiologischem Phosphat und Fructose-6-Phosphat (F6P) verändern kann.

39. Verfahren zur Herstellung einer transformierten Hefezelle mit erhöhtem Vermögen zur Erzeugung von Trehalose im Vergleich zu einer nicht transformierten Hefezelle, umfassend das Transformieren der Hefezelle, indem DNA in sie eingeführt wird, die die Expression einer Hefe-Trehalosesynthasekomplexuntereinheit kodiert,
**dadurch gekennzeichnet, dass** die DNA eine funktionelle Untereinheit kodiert, die an Trehalose-6-Phosphat-Synthasekatalyseaktivität beteiligt ist, wobei dies das Vermögen ist, Uridin-Diphosphoglucose (UDPG) und Glucose-6-Phosphat (G6P) in Trehalose-6-Phosphat umzuwandeln, ausgewählt aus
(i) der etwa 57 kDa Hefeuntereinheit mit Trehalose-6-Phosphatsynthaseaktivität, und
(ii) dem etwa 123 kDa Hefe-Untereinheits-Trehalosesynthase-Regulationspolypeptid oder einer N-terminal verkürzten Form davon, wobei das Polypeptid oder die verkürzte Form die Empfindlichkeit der Hefe-Trehalosesynthase-Trehalose-6-Phosphat-Synthaseaktivität gegenüber physiologischem Phosphat und Fructose-6-Phosphat (F6P) verändern kann.

## Revendications

1. Cellule ou organisme non humain ayant la capacité de produire du tréhalose fourni par encodage d'ADN hétérologue pour l'expression d'un polypeptide ou de polypeptides complexe(s) sous-unitaire(s) de tréhalose de levure synthase,
**caractérisés en ce que** l'encodage d'ADN hétérologue fournit un polypeptide sous-unitaire fonctionnel impliqué dans l'activité catalytique de tréhalose-6-phosphate synthase, soit l'aptitude à convertir l'uridine diphosphoglucose (UDPG) et le glucose-6-phosphate (G6P) en tréhalose-6-phosphate et est sélectionné à partir
(i) du polypeptide sous-unitaire d'environ 57kDa présentant une activité de tréhalose-6-phosphate synthase, et
(ii) du polypeptide sous-unitaire réglementaire d'environ 123kDa de tréhalose synthase ou d'une forme tronquée en terminaison N de la sous-unité 123kDa manquant de jusqu'à 600 acides aminés en sa terminaison N, capable de modifier la sensibilité de l'activité du tréhalose-6-phosphate synthase du tréhalose de levure synthase en phosphate physiologique et fructose-6-phosphate (F6P).

2. Cellule ou organisme non humain selon la revendication 1 **caractérisés en ce que** la sous-unité réglementaire de tréhalose synthase de 123kDa comporte une séquence d'acides aminés ayant une identité d'acides aminés à 37% pour la sous-unité de 57kDa et offrant une capacité dégradable en produits présentant une activité de tréhalose-6-phosphate synthase.

3. Cellule ou organisme non humain selon la revendication 1 ou la revendication 2,
**caractérisés en ce que** le polypeptide ou les polypeptides sous-unitaire(s) se composent d'un ou des deux polypeptides sous-unitaires complexes du tréhalose synthase
(i) une sous-unité d'environ 57kDa comprenant une identification de séquence SEQ ID N°2 présentant une activité de tréhalose-6-phosphate synthase et des mutations fonctionnellement équivalentes de celui-ci et
(ii) un polypeptide réglementaire de tréhalose synthase d'environ 123kDa ou une forme tronquée en terminaison N de celui-ci manquant de jusqu'à 600 acides aminés en sa terminaison N, le polypeptide ou la forme tronquée comprenant une identification de séquence SEQ ID N° 82, ou une mutation fonctionnellement équivalente de celui-ci, et capable de modifier la sensibilité de l'activité de tréhalose-6-phosphate synthase du tréhalose de levure synthase en un phosphate physiologique et en F6P.

4. Cellule ou organisme non humain selon l'une quelconque des revendications 1 à 3 **caractérisés en ce que** l'ADN hétérologue, allié à l'activité de tréhalase de la cellule d'origine, fournit une capacité de cycle futile du tréhalose où le tréhalose formé est converti en glucose par tréhalase avec consommation d'ATP.

5. Cellule ou organisme non humain selon l'une quelconque des revendications 1 à 4, **caractérisés en ce que** l'ADN hétérologue, allié à l'activité de tréhalase de la cellule d'origine, lui fournit une capacité augmentée de cycle futile de tréhalose où le tréhalose formé est converti en glucose par tréhalase avec une consommation accrue d'ATP.

6. Cellule ou organisme non humain selon l'une quelconque des revendications 1 à 5, **caractérisés en ce qu'**ils contiennent de plus un second encodage d'ADN hétérologue pour l'expression d'une sous-unité polypeptidique d'environ 99kDa d'un complexe de tréhalose de levure synthase comprenant une identification SEQ ID N° 29 à 38 et 44 à 49 qui présente une activité de tréhalose-6-phosphate phosphatase, et des mutations fonctionnellement équivalentes de celui-ci.

7. Cellule ou organisme non humain selon l'une quelconque des revendications 1 à 6, **caractérisés en ce qu'**ils comportent un ADN à cadre ouvert de lecture (ORF) d'identification SEQ ID N° 1 ou de mutations fonctionnellement équivalentes de celui-ci.

8. Cellule ou organisme non humain selon l'une quelconque des revendications 1 à 7, **caractérisés en ce qu'**ils comportent un ADN à cadre ouvert de lecture (ORF) d'identification SEQ ID N° 3 ou de mutations fonctionnellement équivalentes de celui-ci.

9. Cellule ou organisme non humain selon la revendication 8, **caractérisés en ce que** l'ADN comprend le cadre ouvert de lecture (ORF) d'identification SEQ ID N° 83 ou des mutations fonctionnellement équivalentes de celui-ci.

10. Cellule ou organisme non humain selon l'une quelconque des revendications 1 à 9, **caractérisés en ce que** la forme tronquée de la longue chaîne de 123 kDa manque de jusqu'à 330 acides aminés en sa terminaison N.

11. Cellule ou organisme non humain selon la revendication 10, caractérisés en qu'ils ont une activité de tréhalose-6-phosphate synthase qui soit moins inhibée par le phosphate que ne l'est le tréhalose de levure synthase d'origine.

12. Cellule ou organisme non humain selon l'une quelconque des revendications 1 à 11 **caractérisés en ce qu'**ils sont plus résistants à la privation d'eau, à la chaleur et au froid que ne le sont les cellules or organismes non humains correspondants privés de l'ADN hétérologue sélectionné offrant l'activité de tréhalose-6-phosphate synthase, cultivés dans les mêmes conditions.

13. Cellule ou organisme non humain selon l'une quelconque des revendications précédentes **caractérisés en qu'**il s'agit d'une levure, de champignons, de bactéries ou d'une cellule végétale ou d'un organisme non humain.

14. Cellule ou organisme non humain selon la revendication 13 **caractérisés en ce qu'**il s'agit d'une cellule végétale ou d'un organisme non humain de catégorie supérieure.

15. Tissu comestible d'une plante **caractérisé en ce qu'**il comprend une cellule telle que revendiquée en revendication 13 ou 14.

16. Procédé de production d'éthanol **caractérisé en ce qu'**il comporte la fermentation de glucide utilisant une cellule de levure selon ce qui est revendiqué en revendication 13.

17. Procédé selon la revendication 16 **caractérisé en ce que** l'ADN hétérologue est fonctionnellement combiné à un activeur actif dans les conditions de fermentation.

18. Procédé de production de tréhalose par la culture d'une cellule ou d'une plante selon ce qui est revendiqué en l'une quelconque des revendications 1 à 15.

19. Procédé de production de denrées alimentaires enrichies de tréhalose à partir de plantes comprenant la culture d'un organisme non humain selon les revendications 13 ou 14 **caractérisé en ce que** l'organisme est une plante et où le tréhalose est synthétisé dans les tissus comestibles de la plante.

20. Procédé de production d'une cellule transformée ou d'un organisme non humain transformé présentant une capacité accrue de production de tréhalose par rapport à une cellule non transformée ou à un organisme non humain non transformé, englobant la transformation de la cellule par l'introduction dans celle-ci de l'ADN d'encodage pour l'expression d'une sous-unité complexe de tréhalose de levure synthase,
**caractérisé en ce que** l'ADN encode pour fournir une sous-unité fonctionnelle impliquée dans l'activité catalytique du tréhalose-6-phosphate synthase, soit l'aptitude à convertir l'uridine diphosphoglucose (UDPG) et le glucose-6-phosphate (G6P) en tréhalose-6-phosphate, sélectionné à partir
(i) De la sous-unité de levure d'environ 57kDa présentant une activité de tréhalose-6-phosphate synthase, et
(ii) Du polypeptide sous-unitaire réglementaire du tréhalose de levure synthase d'environ 123kDa ou d'une forme tronquée en terminaison N de celui-ci, le polypeptide ou la forme tronquée étant capable de modifier la sensibilité de l'activité du tréhalose-6-phosphate synthase de tréhalose de levure synthase en phosphate physiologique et fructose-6-phosphate (F6P).

21. Procédé selon la revendication 20 **caractérisé en ce que**
(i) la sous-unité d'environ 57kDa comporte une identification SEQ ID N° 2 ou une mutation fonctionnellement équivalente de celle-ci présentant une activité catalytique de tréhalose-6-phosphate synthase et
(ii) la sous-unité d'environ 123kDa comporte une identification SEQ ID N° 82, ou une mutation fonctionnellement équivalente de celle-ci, ou une forme tronquée en terminaison N de celle-ci, capable de modifier la sensibilité de l'activité de tréhalose-6-phosphate synthase exhibée par la sous-unité de 57kDa en un phosphate physiologique et F6P.

22. Procédé selon la revendication 21 **caractérisé en ce qu'**il introduit en outre un encodage d'ADN pour une sous-unité d'environ 99kDa comprenant une identification SEQ ID N° 29 à 38 et 44 à 49 présentant une activité de tréhalose-6-phosphate phosphatase ou une mutation fonctionnellement équivalente de celle-ci.

23. Procédé selon l'une quelconque des revendications 20 à 22 **caractérisé en ce que** l'ADN est introduit dans la cellule directement dans un chromosome ou dans un vecteur d'expression.

24. Procédé selon la revendication 23 **caractérisé en ce que** l'ADN est introduit dans un chromosome de la cellule sous la forme d'ADN isolé ou purifié comprenant une séquence nucléotidique encodant pour la sous-unité polypeptidique.

25. Procédé selon la revendication 23 **caractérisé en ce que** le vecteur d'expression est produit en utilisant un ADN isolé ou purifié comprenant une séquence nucléotidique encodant pour la sous-unité polypeptidique.

26. Polynucléotide convenant à une utilisation selon l'une quelconque des revendications 20 à 25 **caractérisé en ce qu'**il s'agit d'un ADN isolé ou purifié encodant la sous-unité fonctionnelle de 57 kDA de tréhalose de levure synthase présentant une activité de tréhalose-6-phosphate synthase.

27. Polynucléotide selon la revendication 26 **caractérisé en ce qu'**il comporte une identification SEQ ID N° 1 ou des mutations fonctionnellement équivalentes de celle-ci.

28. Polynucléotide convenant pour une utilisation dans un procédé selon l'une quelconque des revendications 20 à 25 **caractérisé en ce qu'**il s'agit d'un ADN isolé ou purifié encodant la longue chaîne d'environ 123 kDa de sous-unité réglementaire de tréhalose synthase englobant une séquence d'acides aminés présentant une identité de 37% d'acides aminés pour la sous-unité de 57 kDa, et capable de modifier la sensibilité de l'activité de tréhalose-6-phosphate synthase du tréhalose de levure synthase en un phosphate physiologique et F6P.

29. Polynucléotide selon la revendication 28 **caractérisé en ce qu'**il comprend la séquence polynucléotidique de SEQ ID N° 83 ou une mutation fonctionnellement équivalente de celle-ci.

30. Polynucléotide convenant à une utilisation dans un procédé selon l'une quelconque des revendications 20 à 25 **caractérisé en ce qu'**il s'agit d'un ADN isolé ou purifié d'encodage d'une longue chaîne tronquée d'environ 123 kDa de tréhalose synthase, qui comporte la séquence d'acides aminés d'identification SEQ ID N° 4 ou une mutation fonctionnellement équivalente de celle-ci.

31. Polynucléotide selon la revendication 30 **caractérisé en ce qu'**il comporte une séquence polynucléotidique d'identification SEQ ID N° 3 ou des mutations fonctionnellement équivalentes de celle-ci.

32. Polynucléotide convenant à une utilisation dans un procédé selon l'une quelconque des revendications 20 à 25 **caractérisé en ce qu'**il encode une forme tronquée de la longue chaîne de 123 kDa de tréhalose synthase d'identification SEQ ID N° 82 manquant de jusqu'à 600 acides aminés en terminaison N
**caractérisé en ce que** le polynucléotide offre la capacité de modifier la sensibilité de l'activité de tréhalose-6-phosphate synthase de tréhalose de levure synthase en phosphate physiologique et fructose-6-phosphate (F6P) de telle sorte que en association avec l'unité de 57 kDa l'activité du tréhalose-6-phosphate synthase est moins inhibée par le phosphate que ne l'est l'association des unités 57 kDA et 123 kDA.

33. ADN isolé ou purifié selon la revendication 32 encodant une forme tronquée de la longue chaîne de 123 kDa de tréhalose synthase d'identification SEQ ID N° 82 manquant de jusqu'à 330 acides aminés en terminaison N.

34. Polynucléotide convenant à une utilisation dans un procédé selon l'une quelconque des revendications 20 à 25, **caractérisé en ce qu'**il s'agit d'un ADN isolé ou purifié encodant la longue chaîne d'environ 99kDa de tréhalose synthase, qui comporte les séquences d'acides aminés d'identification SEQ ID N° 29 à 38 et 44 à 49 ou une mutation fonctionnellement équivalente de celui-ci.

35. Polynucléotide selon l'une quelconque des revendications 26 à 34, **caractérisé en ce que** la mutation fonctionnellement équivalente comporte des codons changés par dégénérescence de sorte à en augmenter le taux en G+C.

36. Vecteur d'expression comprenant au moins une ou deux des séquences d'ADN des revendications 26 à 34.

37. Vecteur selon la revendication 36 **caractérisé en ce qu'**il comporte en outre un activeur capable de promouvoir l'expression de l'ADN dans une plante de catégorie supérieure.

38. Cellule de levure transformée présentant une capacité accrue de production du tréhalose par rapport à une cellule de levure non transformée, où la dite capacité accrue est produite par transformation avec un ADN encodant pour l'expression d'un polypeptide ou de polypeptides sous-unitaire(s) complexe(s) de tréhalose de levure synthase
**caractérisée en ce que** l'ADN encode pour fournir un polypeptide sous-unitaire fonctionnel impliqué dans l'activité catalytique de tréhalose-6-phosphate synthase, soit l'aptitude à convertir l'uridine diphosphoglucose (UDPG) et le glucose-6-phosphate (G6P) en tréhalose-6-phosphate, sélectionné à partir
(i) Du polypeptide sous-unitaire d'environ 57kDa présentant une activité de tréhalose-6-phosphate synthase, et
(ii) Du polypeptide sous-unitaire réglementaire de tréhalose synthase d'environ 123kDa ou d'une forme tronquée en terminaison N de la sous-unité de 123 kDa, manquant de jusqu'à 600 acides aminés en sa terminaison N, qui est capable de modifier la sensibilité de l'activité du tréhalose-6-phosphate synthase de tréhalose de levure synthase en phosphate physiologique et fructose-6-phosphate (F6P).

39. Procédé de production d'une cellule de levure transformée présentant une capacité accrue de production du tréhalose par rapport à une cellule de levure non transformée, comprenant la transformation de la cellule de levure par introduction dans celle-ci d'un ADN encodant pour l'expression d'une sous-unité complexe du tréhalose de levure synthase
**caractérisé en ce que** l'ADN encode pour fournir une sous-unité fonctionnelle impliquée dans l'activité catalytique du tréhalose-6-phosphate synthase, soit l'aptitude à convertir l'uridine diphosphoglucose (UDPG) et le glucose-6-phosphate (G6P) en tréhalose-6-phosphate, sélectionné à partir
(i) De la sous-unité de levure d'environ 57kDa présentant une activité de tréhalose-6-phosphate synthase, et
(ii) Du polypeptide sous-unitaire réglementaire d'environ 123kDa de tréhalose synthase ou d'une forme tronquée en terminaison N de celui-ci, le polypeptide ou la forme tronquée étant capables de modifier la sensibilité de l'activité du tréhalose-6-phosphate synthase de tréhalose de levure synthase en phosphate physiologique et fructose-6-phosphate (F6P).
